# EUROPÄISCHE PATENTSCHRIFT

(11) **EP 0 912 093 B1**
(45) Veröffentlichungstag und Bekanntmachung des Hinweises auf die Patenterteilung: **06.08.2003**
(21) Anmeldenummer: 97922979.6
(22) Anmeldetag: 06.05.1997
(51) Int. Cl.: A01N 47/36, C07D 251/46, C07D 239/46, C07D 251/16, C07D 239/42, C07D 239/52

(54) **(HETERO)ARYLSULFONYLHARNSTOFFE MIT EINER IMINOFUNKTION, IHRE DARSTELLUNG UND VERWENDUNG ALS HERBIZIDE UND PFLANZENWACHSTUMSREGULATOREN**
(HETERO)ARYL SULPHONYL UREAS WITH AN IMINOFUNCTION, THE PREPARATION AND USE THEREOF AS HERBICIDES AND PLANT GROWTH REGULATORS
(HETERO)ARYLSULFONYLEUREES PRESENTANT UNE FONCTION IMINO, LEUR PREPARATION ET LEUR UTILISATION COMME HERBICIDES ET REGULATEURS DE LA CROISSANCE VEGETALE

(30) Priorität: 15.05.1996 DE 19619628
(43) Veröffentlichungstag der Anmeldung: 06.05.1999
(73) Patentinhaber: Bayer CropScience GmbH, 65929 Frankfurt/Main (DE)
(72) Erfinder: SCHNABEL, Gerhard, D-63820 Elsenfeld (DE); WILLMS, Lothar, D-65719 Hofheim (DE); BAUER, Klaus, D-63456 Hanau (DE); BIERINGER, Hermann, D-65817 Eppstein (DE); ROSINGER, Christopher, D-65719 Hofheim (DE)
(86) Internationale Anmeldenummer: EP9702304
(87) Internationale Veröffentlichungsnummer: WO97042822

(56) Entgegenhaltungen:
- EP-A- 0 001 515
- EP-A- 0 116 518
- DE-A- 1 470 316
- DE-A- 2 304 587
- DE-A- 2 308 943
- DE-A- 4 230 933
- DE-A- 4 439 675
- DE-A- 4 439 676
- US-A- 3 835 115
- US-A- 4 225 337
- US-A- 4 892 946

## Beschreibung

Die Erfindung betrifft das technische Gebiet der Herbizide und Pflanzenwachstumsregulatoren, insbesondere der Herbizide zur Bekämpfung von Unkräutern und Ungräsern in Nutzpflanzenkulturen.

Es ist bekannt, daß N-Pyrimidinyl- oder N-Triazinyl-(hetero)-arylsulfonylharnstoffe, die einen oder mehrere bestimmte Substituenten am aromatischen bzw. heteroaromatischen Molekülteil tragen, herbizide Eigenschaften besitzen. Beispielsweise stellen Phenylsulfonylharnstoffe, die eine Stickstoffunktion mit bestimmtem Substitutionsmuster tragen (wie z.B. Acylamino- oder N-Acyl-N-alkyl-amino) potente Herbizide dar; siehe z.B. DE-A-4230933, DE-A-44 39 676, EP-A-116 518, US-A-4 885 337, US-A-4 892 946, US-A-4 453 971, US-A-4 369 058.

Überraschenderweise wurde nun gefunden, daß Sulfonylharnstoffe mit spezifischen Stickstoffresten als Herbizide und Pflanzenwachstumsregulatoren besonders gut geeignet sind.

Gegenstand der Erfindung sind Verbindungen der Formel (I) oder deren Salze, 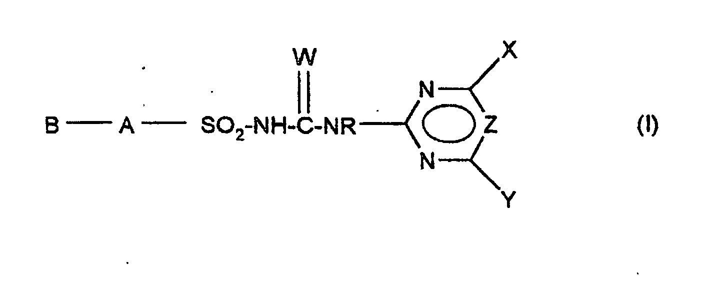 worin
- A: eine Brücke der Formeln A₁-A₉ 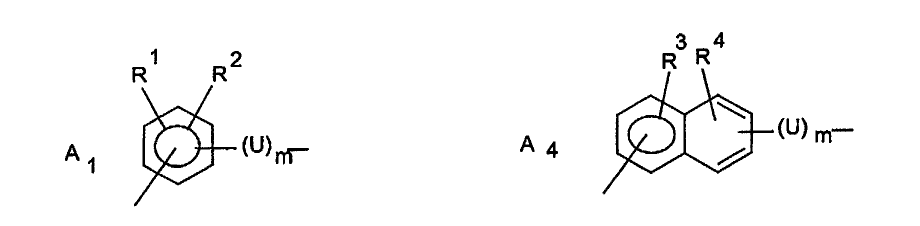 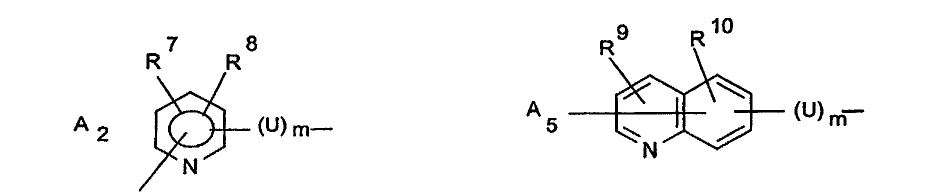 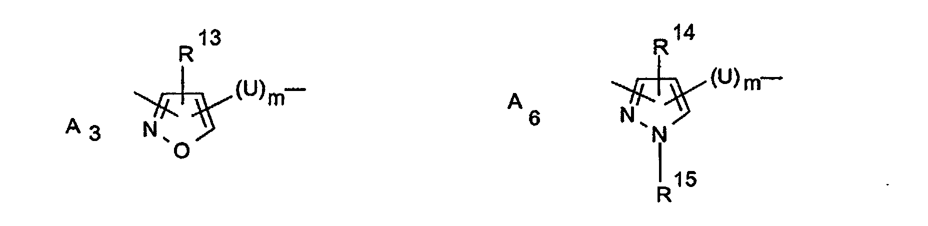 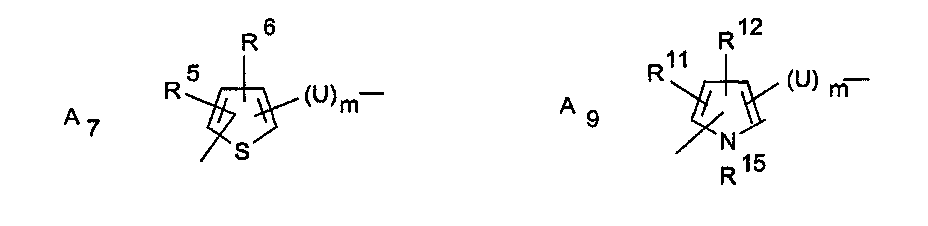 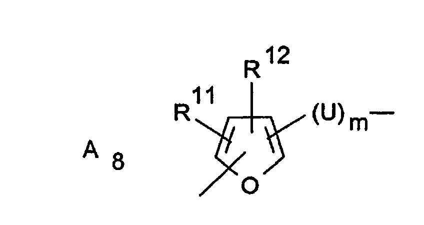
- m: 0 oder 1,
- U: CH₂, O oder NH oder (C₁-C₃-Alkyl)N,
- R¹: H, CN, Halogen, Azid, NO₂, OH, C₁-C₈-Alkyl, C₂-C₈-Alkenyl, C₂-C₈- Alkinyl C₁-C₈-Alkoxy C₂-C₈-Alkenyloxy C₂-C₈-Alkinyloxy, C₃-C₈-Cycloalkyl, C₃-C₈-Cycloalkyloxy, C₅-C₈-Cycloalkenyl, C₅-C₈-Cycloalkenyloxy,
wobei jeder der letztgenannten 10 Reste unsubstituiert oder durch einen oder mehrere Reste aus der Gruppe Halogen, C₁-C₄-Alkoxy, C₁-C₄-Haloalkoxy, C₁-C₄-Alkylthio, C₁-C₄-Haloalkylthio, OH, CN, NO₂, Oxo, C₁-C₄-Alkylsulfonyl, C₁-C₄-Haloalkylsulfonyl, (C₁-C₄-Alkoxy)-carbonyl, (C₁-C₄-Haloalkoxy)-carbonyl, NR³²R³³ und im Falle cyclischer Reste auch C₁-C₄-Alkyl und C₁-C₄-Haloalkyl substituiert ist, oder COR²⁴, CS-R²⁵, C(=NR²⁶)R²⁷, NR²⁸R²⁹, oder einen 3 bis 7-gliedrigen Heterocyclus mit bis zu 4 Heteroatomen aus der Gruppe N, O und S, der unsubstituiert ist oder durch einen oder mehrere Reste aus der Gruppe Halogen, C₁-C₄-Alkyl, OH, CN, NO₂, CHO, NH₂, Mono- und Di-(C₁-C₄-alkyl)-amino, C₁-C₄-Alkyl, C₁-C₄-Haloalkyl, C₁-C₄-Alkoxy, C₁-C₄-Haloalkoxy, C₁-C₄-Alkylthio, C₁-C₄-Haloalkylthio, C₁-C₄-Alkylcarbonyl, C₁-C₄-Alkoxycarbonyl und Oxo substituiert ist, oder Aryl, Heteroaryl, Aryl-C₁-C₃-alkyl oder Heteroaryl-C₁-C₃-alkyl, wobei jeder der letztgenannten 4 Reste im Aryl- bzw. Heteroarylteil unsubstituiert oder durch einen oder mehrere Reste aus der Gruppe Halogen, OH, CN, Nitro, C₁-C₄-Alkyl, C₁-C₄-Haloalkyl, C₁-C₄-Alkoxy, C₁-C₄-Haloalkoxy, C₁-C₄-Alkylthio, C₁-C₄-Haloalkylthio, C₁-C₄-Alkylsulfonyl, C₁-C₄-Haloalkylsulfonyl, C₁-C₄-Alkylcarbonyl, C₁-C₄-Haloalkylcarbonyl, (C₁-C₄-Alkoxy)carbonyl, (C₁-C₄-Haloalkoxy)-carbonyl, Mono- und Di(C₁-C₄-alkyl)amino, CHO, NH₂, CONH₂, Mono- und Di-(C₁-C₄-alkyl)-aminocarbonyl substituiert ist,
- R²: Wasserstoff, C₁-C₈-Alkyl, C₂-C₈-Alkenyl, C₂-C₈-Alkinyl, C₁-C₈ - Alkoxy, C₂-C₈-Alkenyloxy, C₂-C₈-Alkinyloxy, C₃-C₈-Cycloalkyl, C₅-C₈-Cycloalkenyl, C₅-C₈-Cycloalkoxy, C₅-C₈-Cycloalkenyloxy,
wobei jeder der letztgenannten 10 Reste unsubstituiert oder durch einen oder mehrere Reste aus der Gruppe Halogen, C₁-C₄-Alkoxy und C₁-C₄-Haloalkoxy und im Falle cyclischer Reste auch C₁- C₄-Alkyl und C₁-C₄-Haloalkyl substituiert ist, oder Halogen, CN, NH₂, Mono- oder Di-(C₁-C₄-alkyl)-amino,
- R³, R⁵, R⁷, R⁹, R^{11,} R¹³ und R¹⁴: jeweils einen Rest aus der Gruppe der für R¹ möglichen Reste,
- R⁴, R⁸, R⁸, R¹⁰ und R¹²: jeweils einen Rest aus der Gruppe der für R² möglichen Reste,
- R¹⁵: Wasserstoff, C₁-C₈-Alkyl, C₃-C₈-Cycloalkyl, C₁-C₈-Haloalkyl, C₃-C₈-Halocycloalkyl, Aryl, das unsubstituiert oder durch einen oder mehrere Reste aus der Gruppe Halogen, C₁-C₄-Alkyl, C₁-C₄-Haloalkyl, C₁-C₄-Alkoxy und C₁-C₄-Haloalkoxy substituiert ist,
- B: eine Gruppe, die ein iminohaltiges 3-atomiges Fragment aus der Gruppe der Fragmente der Formeln N = C-N, N = C-S, N = C-O und N = C-C enthält und die über ein Stickstoffatom des Fragments an den Rest A gebunden ist,
- R: H oder einen aliphatischen Rest,
- W: ein Sauerstoff- oder Schwefelatom,
- X,Y: unabhängig voneinander Wasserstoff, Halogen, C₁-C₆-Alkyl, C₁-C₆-Alkoxy, C₁-C₆-Alkylthio, C₃-C₆-Cycloalkyl, C₃-C₆-Cycloalkoxy, C₃-C₆-Cycloalkylthio, C₂-C₆-Alkenyl, C₅-C₆-Cycloalkenyl, C₂-C₆-Alkinyl, C₃-C₆-Alkenyloxy, C₅-C₆-Cycloalkenyloxy, C₃-C₆-Alkinyloxy, wobei jeder der elf letztgenannten Reste unsubstituiert oder durch einen oder mehrere Reste aus der Gruppe Halogen, C₁-C₄-Alkoxy, C₁-C₄-Alkylthio, C₁-C₄-Haloalkoxy und im Falle cyclischer Reste auch C₁-C₄-Alkyl und C₁-C₄-Haloalkyl substituiert ist, oder Mono- oder Di-(C₁-C₄-alkyl)amino und
- Z: CH oder N bedeuten.

In der Formel (I) und den im folgenden verwendeten Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 4 C-Atomen bzw. bei ungesättigten Gruppen mit 2 bis 4 C-Atomen, bevorzugt. Alkylreste, auch in den zusammengesetzten Bedeutungen wie Alkoxy, Haloalkyl usw., bedeuten, z.B. Methyl, Ethyl, n- oder i-Propyl, n-, i-, t-oder 2-Butyl, Pentyle, Hexyle, wie n-Hexyl, i-Hexyl und 1,3-Dimethylbutyl, Heptyle, wie n-Heptyle, 1-Methylhexyl und 1,4-Dimethylpentyl. Cycloalkyl bedeutet einen cycloaliphatischen Kohlenwasserstoffrest wie Cyclopropyl, Cyclobutyl, Cyclopentyl oder Cyclohexyl usw.; Alkenyl, Cycloalkenyl- und Alkinyl haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, Methyl-but-3-en-1-yl und 1-Methyl-but-2-en-1-yl; Cycloalkenyl ist z.B. Cyclopentenyl und Cyclohexenyl; Alkinyl bedeutet z.B. Propargyl, But-2-in-1-yl, But-3-in-1-yl, 1-Methyl-but-3-in-1-yl.
Alkenyl in der Form "(C₃-C₄)Alkenyl" oder "(C₃-C₆)Alkenyl" bedeutet vorzugsweise einen Alkenylrest mit 3 bis 4 bzw. 3 bis 6 C-Atomen, bei dem die Doppelbindung nicht an dem C-Atom liegt, das mit dem übrigen Molekülteil der Verbindung (I) verbunden ist ("yl"-Position). Entsprechendes gilt für (C₃-C₄)Alkinyl usw.

Halogen bedeutet beispielsweise Fluor, Chlor, Brom oder lod, Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl₂, CCl₃, CHCl₂, CH₂CH₂Cl; Haloalkyl ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Ein Kohlenwasserstoffrest ist ein geradkettiger, verzweiger oder cyclischer und gesättigter oder ungesättigter aliphatischer oder aromatischer Kohlenwasserstoffrest, z.B. Alkyl, Alkenyl, Alkinyl, Cycloalkyl, Cycloalkenyl oder Aryl, vorzugsweise Alkyl, Alkenyl oder Alkinyl mit bis zu 12 C-Atomen oder Cycloalkyl mit 3 bis 8 Ringatomen, vorzugsweise 5 oder 6 Ringatomen, oder Phenyl; entsprechendes gilt für einen Kohlenwasserstoffoxyrest.

Ein organischer Rest ist ein kohlenstoffhaltiger Rest mit einem oder mehreren gegebenenfalls substituierten aliphatischen und/oder (hetero)aromatischen Resten. Ein aliphatischer Rest ist ein nichtaromatischer organischer Rest, der außer C-Atomen und Wasserstoffatomen auch Heteroatome enthalten kann, vorzugsweise ein nichtaromatischer Kohlenwasserstoffrest, der unsubstituiert oder durch einen oder mehrere Reste aus der Gruppe Halogen, C₁-C₄-Alkoxy und C₁-C₄-Alkylthio substituiert ist.

Aryl bedeutet ein mono-, bi- oder polycyclisches aromatisches System, beispielsweise Phenyl, Naphthyl, Tetrahydronaphthyl, Indenyl; Indanyl, Pentalenyl, Fluorenyl und ähnliches, vorzugsweise Phenyl; Aryloxy bedeutet vorzugsweise ein dem genannten Arylrest entsprechender Oxy-Rest, insbesondere Phenoxy.

Heteroaryl oder ein heteroaromatischer Rest bedeutet ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Thienyl, Thiazolyl, Oxazolyl, Furyl, Pyrrolyl, Pyrazolyl und Imidazolyl. Im substituierten Fall werden insbesondere auch bicyclische oder polycyclische aromatische oder mit cycloaliphatischen Ringen anellierte Verbindungen, z.B. Chinolinyl, Benzoxazolyl etc. eingeschlossen. Heteroaryl schließt auch einen heteroaromatischen Ring ein, der vorzugsweise 5- oder 6-gliedrig ist und 1,2-oder 3 Heteroringatome, insbesondere aus der Gruppe N, O und S enthält. Im substituierten Fall kann der heteroaromatische Ring auch benzokondensiert sein.

Ein heterocyclischer Rest (Heterocyclyl) oder Ring (Heterocyclus) kann gesättigt, ungesättigt oder heteroaromatisch sein; er enthält ein oder mehrere Heteroringatome, vorzugsweise aus der Gruppe N, O und S; vorzugsweise ist er ein nicht aromatischer Ring mit 3 bis 8 Ringatomen und 1 bis 3 Heteroringatomen aus der Gruppe N, O und S oder er ist ein heteroaromatischer Ring mit 5 oder 6 Ringatomen und enthält 1, 2 oder 3 Heteroringatome aus der Gruppe N, O und S. Der Rest kann z.B. ein wie oben definierter heteroaromatischer Rest oder Ring sein oder ist ein partiell hydrierter Rest wie Oxiranyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte Kohlenwasserstoffreste, z.B. substitutiertes Alkyl, Alkenyl, Alkinyl, Aryl, Phenyl und Benzyl, oder substituiertes Heteroaryl, ein substituierter bicyclischer Rest oder Ring oder ein substituierter bicyclischer Rest, gegebenenfalls mit aromatischen Anteilen, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Cyano, Azido, Alkoxycarbonyl, Alkylcarbonyl, Formyl, Carbamoyl, Mono- und Dialkylaminocarbonyl, substituiertes Amino wie Acylamino, Mono- oder Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, C₁-C₄-Alkyl, vorzugsweise Methyl oder Ethyl, C₁-C₄-Haloalkyl, vorzugsweise Trifluormethyl, C₁-C₄-Alkoxy, vorzugsweise Methoxy oder Ethoxy, C₁-C₄-Haloalkoxy, Nitro und Cyano. Besonders bevorzugt sind dabei die Substituenten Methyl, Methoxy und Chlor.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, C₁-C₄-Alkyl, C₁-C₄-Alkoxy, C₁-C₄-Halogenalkyl, C₁-C₄-Halogenalkoxy und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Mono- oder disubstituiertes Amino bedeutet einen chemisch stabilen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Monoalkylamino, Dialkylamino, Acylamino, Arylamino, N-Alkyl-N-arylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechendes gilt für substituiertes Hydroxylamino oder Hydrazino.

Gegenstand der Erfindung sind auch alte Stereoisomeren, die von Formel (I) umfaßt sind, und deren Gemische. Solche Verbindungen der Formel (I) enthalten ein oder mehrere asymmetrische C-Atome oder auch Doppelbindungen, die in der allgemeinen Formel (I) nicht gesondert angegeben sind. Die durch ihre spezifische Raumform definierten möglichen Stereoisomeren, wie Enantiomere, Diastereomere, Z- und E-lsomere sind alle von der Formel (I) umfaßt und können nach üblichen Methoden aus Gemischen der Stereoisomeren erhalten oder auch durch stereoselektiven Reaktionen in Kombination mit dem Einsatz von stereochemisch reinen Ausgangsstoffen hergestellt werden.

Formel (I) umfaßt auch Tautomere der bezeichneten Verbindungen, soweit sie durch Protonenwanderung entstehen und soweit sie chemisch stabile Tautomere sind.

Die Verbindungen der Formel (I) können Salze bilden, bei denen der Wasserstoff der -SO₂-NH-Gruppe oder auch andere acide Wasserstoffatome (z.B. aus COOH u.a.) durch ein für die Landwirtschaft geeignetes Kation ersetzt wird. Diese Salze sind beispielsweise Metallsalze; vorzugsweise Alkali- oder Erdalkalisalze, insbesondere Natrium- und Kaliumsalze, oder auch Ammoniumsalze oder Salze mit organischen Aminen. Ebenso kann Salzbildung durch Anlagerung einer Säure an basische Gruppen, wie z.B. Amino und Alkylamino, erfolgen. Geeignete Säuren hierfür sind starke anorganische und organische Säuren, beispielsweise HCI, HBr, H₂SO₄ oder HNO₃.

Aus Gründen der höheren herbiziden Wirkung, besseren Selektivität und/oder besseren Herstellbarkeit sind erfindunggemäße Verbindungen der Formel (I) oder deren Salze von näherem Interesse, worin
- B: eine iminohaltige Gruppe der Formel, 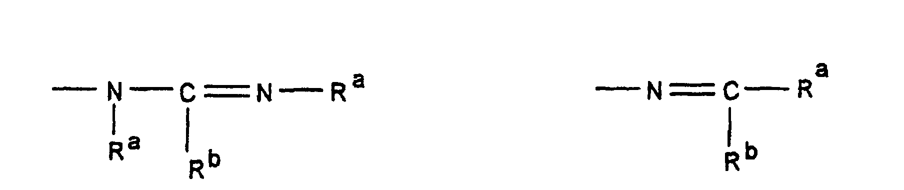 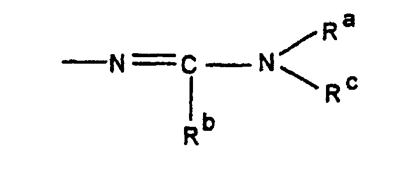 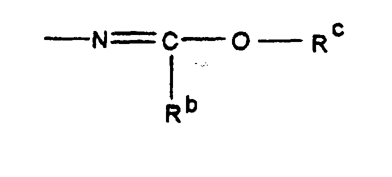
oder 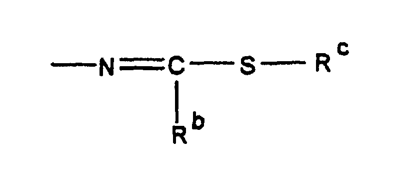 wobei die R^{a} unabhängig voneinander Wasserstoff, NH₂, OH oder einen aliphatischen, aromatischen, heterocyclischen oder anderen organischen Rest mit insgesamt 1 bis 12 C-Atomen, der auch über ein Heteroatom gebunden sein kann, bedeuten, die R^{b} unabhängig voneinander jeweils einen der für R^{a} möglichen Reste, ausgenommen OH, bedeuten, die R^{c} unabhängig voneinander H oder einen aliphatischen, aromatischen, heterocyclischen oder anderen organischen Rest mit insgesamt 1 bis 20 C-Atomen bedeuten und wobei die Reste R^{a} bis R^{c} in einer Grupppe B paarweise eine Brücke, vorzugsweise eine aliphatische Brücke, bilden können und die organische Gruppe B insgesamt 1 bis 30 C-Atome besitzt,
- R: H oder einen Kohlenwasserstoffrest mit 1 bis 8 C-Atomen, welcher unsubstituiert oder durch einen oder mehrere Reste aus der Gruppe Halogen, C₁-C₄-Alkoxy und C₁-C₄-Alkylthio substituiert ist,
- W: O oder S, vorzugsweise O,
- einer der Reste X und Y: Halogen, C₁-C₆-Alkyl, C₁-C₆-Alkoxy, C₁-C₆-Alkylthio, wobei die letztgenannten 3 Reste unsubstituiert oder durch einen oder mehrere Reste aus der Gruppe Halogen, C₁-C₄-Alkoxy und C₁-C₄-Alkylthio substituiert sind, oder C₂-C₄-Alkenyl, C₂-C₄-Alkinyl, C₂-C₄-Alkenyloxy, C₂-C₄-Alkinyloxy, wobei jeder der letztgenannten 4 Reste unsubstituiert oder durch einen oder mehrere Halogenatome substituiert ist, oder C₃-C₆-Cycloalkyl oder Mono- oder Di-(C₁-C₂-alkyl)amino und
- der andere der Reste X und Y Halogen,: C₁-C₄-Alkyl, C₁-C₄-Alkoxy oder C₁-C₄-Alkylthio, wobei jeder der letztgenannten 3 Reste unsubstituiert oder durch einen oder mehrere Reste aus der Gruppe Halogen, C₁-C₄-Alkoxy und C₁-C₄-Alkylthio substituiert ist, oder Mono- oder Di-(C₁-C₂-Alkyl)amino und
- Z: CH oder N
bedeuten.

Von besonderem Interesse sind die erfindungsgemäßen Verbindungen der Formel (I) oder deren Salze, worin
- B: eine Gruppe der Formel B1 bis B5,

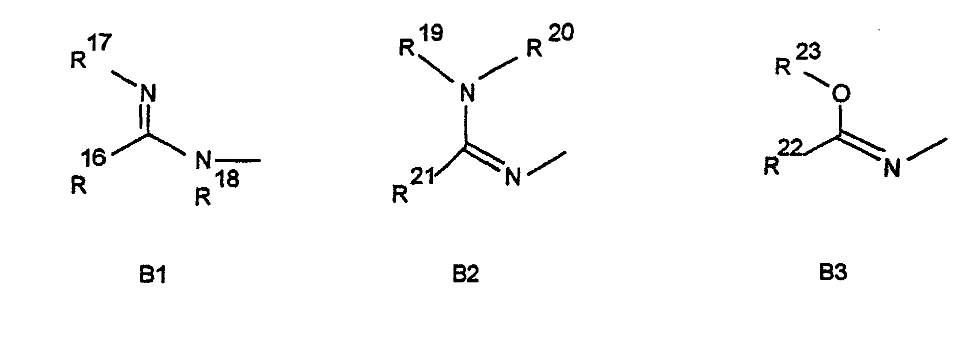

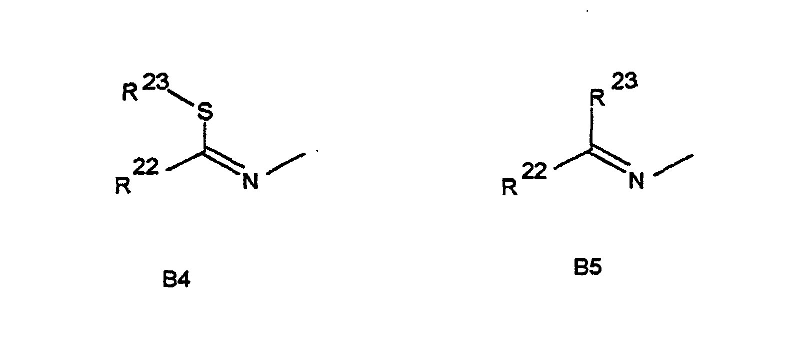

- R: Wasserstoff, C₁-C₅-Alkyl, C₁-C₅-Haloalkyl, C₂-C₅-Alkenyl, C₂-C₅-Haloalkenyl, C₂-C₅-Alkinyl oder C₂-C₅-Haloalkinyl,

- R¹⁶: NH₂, Wasserstoff, Aryl oder Heteroaryl, insbesondere Phenyl, wobei der Aryl-, Heteroaryl- bzw. Phenylrest unsubstitutiert oder substituiert ist, insbesondere unsubstituiert oder durch einen oder mehrere Reste aus der Gruppe Halogen, C₁-C₄-Alkyl, C₁-C₄-Haloalkoxy, C₁-C₄-Alkoxy, C₁-C₄-Haloalkyl, CN und Nitro substituiert ist, oder C₁-C₈-Alkyl, C₂-C₈-Alkenyl, C₂-C₈-Alkinyl, C₁-C₈-Alkoxy, C₂-C₈-Alkenyloxy, C₂-C₈-Alkinyloxy, C₁-C₈-Alkylthio, C₂-C₈-Alkenylthio, C₂-C₈-Alkinylthio, C₃-C₈-Cycloalkyl, C₅-C₈-Cycloalkenyl, C₃-C₆-Cycloalkoxy, C₅-C₈-Cycloalkenyloxy, C₃-C₈-Cycloalkylthio, C₅-C₈-Cycloalkenylthio, Mono- oder Di-(C₁-C₆)alkyl-amino, wobei jeder der letztgenannten 17 Reste unsubstituiert oder substituiert ist, insbesondere unsubstituiert oder durch einen oder mehrere Reste aus der Gruppe Halogen, C₁-C₄-Haloalkoxy, C₁-C₄-Alkoxy, CN, Oxo, (C₁-C₄-Alkoxy)carbonyl und im Falle cyclischer Reste auch C₁-C₄-Alkyl und C₁-C₄-Haloalkyl substituiert ist, oder Benzyl, CN, (C₁-C₆-Alkoxy)carbonyl, (C₁-C₆-Alkyl)-carbonyl, Benzyloxycarbonyl oder Phenyl-carbonyl,
- R¹⁷: OH, NH₂, Wasserstoff, C₁-C₈-Alkyl, C₂-C₈-Alkenyl, C₂-C₈-Alkinyl, C₁-C₈-Alkoxy, C₂-C₈-Alkenyloxy, C₂-C₈-Alkinyloxy, (C₁-C₈-Alkyl)-carbonyl, (C₁-C₈-Alkoxy)-carbonyl, Benzoyl, Benzyloxycarbonyl, (C₁-C₈)-Alkylsulfonyl, Arylsulfonyl, insbesondere Phenylsulfonyl, C₃-C₈-Cycloalkyl, C₅-C₈-Cycloalkenyl, C₃-C₈-Cycloalkoxy, C₅-C₈-Cycloalkenyloxy, C₃-C₈-Cycloalkylcarbonyl, C₃-C₈-Cycloalkoxycarbonyl, C₃-C₈-Cycloalkylsulfonyl, Mono- oder Di-(C₁-C₈-alkyl)-amino, wobei jeder der letztgenannten 22 Reste unsubstituiert oder durch einen oder mehrere Reste aus der Gruppe Halogen, C₁-C₄-Alkoxy, C₁-C₄-Haloalkoxy, CN, NO₂, OH, NH₂, Mono- und Di-(C₁-C₃)-alkylamino, C₁-C₄-Alkylthio und C₁-C₄-Haloalkylthio und im Falle cyclischer Reste auch C₁-C₄-Alkyl und C₁-C₄-Haloalkyl substituiert ist, oder Aryl, insbesondere Phenyl, oder Heteroaryl, insbesondere Furanyl oder Thienyl, wobei jeder der 5 letztgenannten Reste unsubstituiert oder durch einen oder mehrere Reste aus der Gruppe C₁-C₄-Alkyl, C₁-C₄-Haloalkyl, C₁-C₄-Alkoxy, C₁-C₄-Haloalkoxy, CN und NO₂ substituiert ist,
oder der Teil der Formel 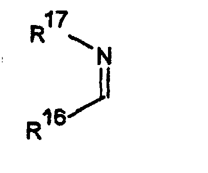 aus der Gruppe der oben genannten Formel B1 (siehe Definition von B) gemeinsam einen heterocyclischen Ring mit einer Iminofunktion im Ring, vorzugsweise ein heterocyclischer Ring mit 5 oder 6 Ringatomen und neben dem N-Atom der Iminogruppe gegebenenfalls mit 1 oder 2 Heteroringatomen aus der Gruppe N, O uns S, insbesondere ein Ring der Formeln D1 bis D15, 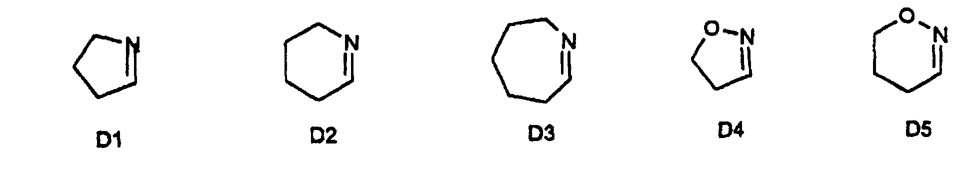 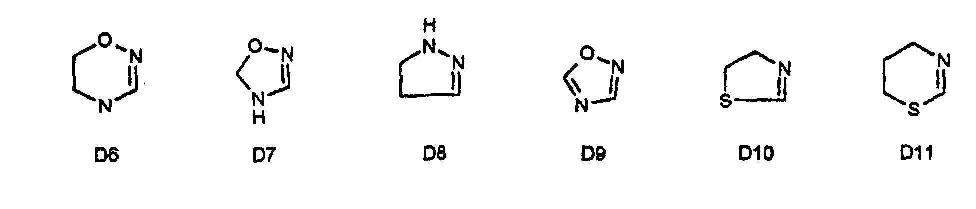 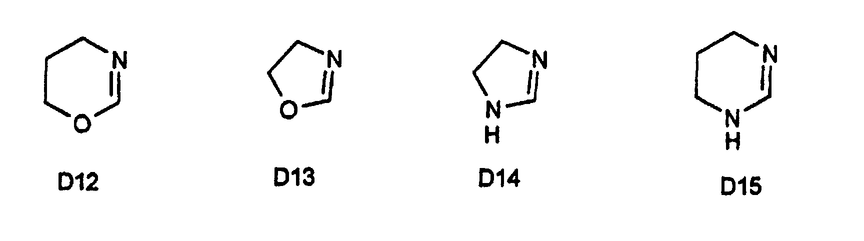 wobei der heterocyclische Ring bzw. jeder der Ringe D1 bis D15 unsubstituiert oder substituiert ist, vorzugsweise unsubstituiert oder durch einen oder mehrere Reste aus der Gruppe Halogen, C₁-C₄-Alkyl, C₁-C₄-Haloalkyl, C₁-C₄-Haloalkoxy, OH, NH₂, CN, NO₂, Mono- und Di-(C₁-C₄-alkyl)-amino substituiert ist,
- R¹⁸: Wasserstoff, C₁-C₈-Alkyl, C₂-C₈-Alkenyl, C₂-C₈-Alkinyl, C₁-C₈-Alkylsulfonyl, (C₁-C₈-Alkyl)-carbonyl, (C₁-C₈-Alkoxy)-carbonyl, C₁-C₈-Alkoxy, C₃-C₈-Cycloalkyl, C₅-C₈-Cycloalkenyl, C₃-C₈-Cycloalkylsulfonyl, C₃-C₈-Cycloalkylcarbonyl, C₃-C₈-Cycloalkoxycarbonyl, C₃-C₈-Cycloalkoxy, wobei jeder der letztgenannten 13 Reste unsubstituiert oder durch einen oder mehrere Reste aus der Gruppe Halogen, C₁-C₃-Alkoxy und C₁-C₃-Haloalkoxy und im Falle cyclischer Reste auch C₁-C₄-Alkyl und C₁-C₄-Haloalkyl substituiert ist, oder CHO, NH₂, OH, Mono- oder Di-(C₁-C₄-alkyl)-amino,
- R¹⁹: einen Rest aus der Gruppe der für R¹⁷ möglichen Reste und
- R²⁰: Wasserstoff, C₁-C₈-Alkyl, C₃-C₈-Cycloalkyl, C₅-C₈-Cycloalkenyl, C₂-C₆-Alkenyl oder C₂-C₈-Alkinyl oder
- NR¹⁹R²⁰: einen heterocyclischen Ring, vorzugsweise mit 3 bis 6 Ringatomen und neben dem 1-N-Atom gegebenenfalls mit 1 oder 2 Heteroringatomen aus der Gruppe N, O und S, insbesondere eine funktionelle Gruppe der Formeln E1 bis E9, 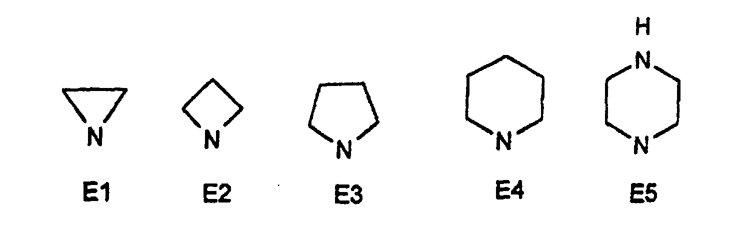 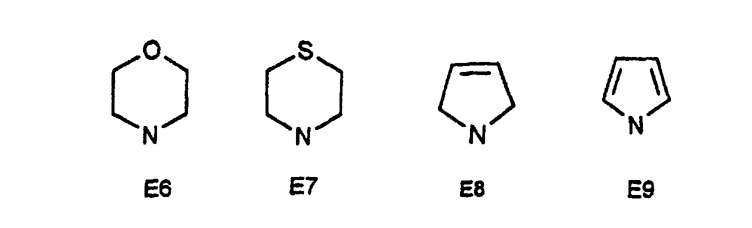 wobei jeder der heterocyclischen Reste unsubstituiert oder durch einen oder mehrere Reste aus der Gruppe Halogen, C₁-C₄-Alkyl, C₁-C₄-Haloalkyl, C₁-C₄-Alkoxyl C₁-C₄-Haloalkoxy, OH, NH₂, NO₂, CN, Mono-und Di-(C₁-C₅-alkyl)-amino substituiert ist,
- R²¹: einen Rest aus der Gruppe der für R¹⁶ möglichen Reste,
- R²²: Wasserstoff, C₁-C₈-Alkyl, C₂-C₈-Alkenyl, C₂-C₈-Alkinyl, C₁-C₈-Alkoxy, C₁-C₈-Alkylthio, C₃-C₈-Cycloalkoxy, C₃-C₈-Cycloalkylthio, C₃-C₈-Cycloalkyl, C₅-C₈-Cycloalkenyl, Mono- oder Di-(C₁-C₈)-alkylamino, wobei jeder der letztgenannten 11 Reste unsubstituiert oder durch einen oder mehrere Reste aus der Gruppe CN, Halogen, C₁-C₄-Alkoxy, C₁-C₄-Haloalkoxy und im Falle cyclischer Reste auch C₁-C₄-Alkyl und C₁-C₄-Haloalkyl substituiert ist, oder einen aromatischen oder heteroaromatischen Monocyclus oder Polycyclus, vorzugsweise Phenyl, Thienyl, Pyridyl, Furanyl, wobei jeder dieser Reste unsubstituiert oder substituiert ist, vorzugsweise unsubstituiert oder durch einen oder mehrere Reste aus der Gruppe Halogen, C₁-C₄-Alkyl, C₁-C₄-Haloalkyl, C₁-C₄-Alkoxy, C₁-C₄-Haloalkoxy, CN und Nitro substituiert ist,
- R²³: C₁-C₈-Alkyl, C₂-C₈-Alkenyl, C₂-C₈-Alkinyl, C₃-C₈-Cycloalkyl, C₅-C₈-Cycloalkenyl, Heterocyclyl, Aryl, insbesondere Phenyl, oder Benzyl, wobei jeder der 9 letztgenannten Reste unsubstituiert oder durch einen oder mehrere Reste aus der Gruppe Halogen, C₁-C₄-Alkoxy, C₁-C₄-Haloalkoxy, CN und NO₂ und im Falle cyclischer Reste auch C₁-C₄-Alkyl und C₁-C₄-Haloalkyl substituiert ist,
- R²⁴: Wasserstoff, OH, NH₂, Aryl oder einen Heterocyclus, vorzugsweise Phenyl, Furyl, Thienyl oder Pyridyl, wobei jeder der letztgenannten 6 Reste unsubstituiert oder substituiert ist, insbesondere unsubstituiert oder durch einen oder mehrere Reste aus der Gruppe Halogen, C₁-C₄-Alkyl, C₁-C₄-Haloalkyl, CN, NH₂ und Nitro substituiert ist, oder C₁-C₈-Alkyl, C₂-C₈-Alkenyl, C₂-C₈-Alkinyl, C₁-C₈-Alkoxy, C₂-C₈-Alkenyloxy, C₂-C₈-Alkinyloxy, C₁-C₈-Alkylthio, C₂-C₈-Alkenylthio, C₂-C₈-Alkinylthio, Benzyl, Aryloxy, insbesondere Phenoxy, oder Benzyloxy, C₃-C₈-Cycloalkyl, C₅-C₈-Cycloalkenyl, C₃-C₈-Cycloalkoxy, C₅-C₈-Cycloalkenyloxy, C₅-C₈-Cycloalkylthio oder C₅-C₈-Cycloalkenylthio, Mono- oder Di-(C₁-C₈-alkyl)amino, wobei jeder der letztgenannten 21 Reste unsubstituiert oder durch einen oder mehrere Reste aus der Gruppe Halogen, C₁-C₄-Alkoxy, C₁-C₄-Haloalkoxy, OH, NH₂, Mono- und Di(C₁-C₄-alkyl)amino, CN und NO₂ und im Falle cyclischer Reste auch C₁-C₄-Alkyl und C₁-C₄-Haloalkyl substituiert ist,
- R²⁵: einen Rest aus der Gruppe der für R²⁴ möglichen Reste,
- R²⁶: Wasserstoff, OH, NH₂, CN, C₁-C₈-Alkoxy, C₂-C₈-Alkenyloxy, C₂-C₈-Alkinyloxy, C₁-C₈-Alkyl, C₂-C₈-Alkenyl, C₂-C₈-Alkinyl, C₁-C₈-Alkylsulfonyl, C₂-C₈-Alkenylsulfonyl, C₂-C₈-Alkinylsulfonyl, C₃-C₈-Cycloalkoxy, C₅-C₈-Cycloalkenyloxy, C₃-C₈-Cycloalkyl, C₅-C₈-Cycloalkenyl, C₃-C₈-Cycloalkylsulfonyl, C₅-C₈-Cycloalkenylsulfonyl, Arylsulfonyl, insbesondere Phenylsulfonyl, oder Aryl oder einen Heterocyclus, insbesondere Phenyl, Furyl, Thienyl oder Pyridyl, oder Benzyl, (C₁-C₈-Alkoxy)-carbonyl, (C₁-C₈-Alkyl)-carbonyl, Phenoxycarbonyl, Benzylcarbonyl, Heterocyclylcarbonyl, insbesondere Furylcarbonyl, Pyridylcarbonyl oder Thienylcarbonyl, wobei jeder der letztgenannten 32 Reste unsubstituiert oder substitutiert ist, vorzugsweise unsubstituiert oder durch einen oder mehrere Reste aus der Gruppe Halogen, C₁-C₄-Alkoxy, C₁-C₄-Haloalkoxy, Oxo, CN, C₁-C₄-Alkylthio, C₁-C₄-Haloalkylthio, C₁-C₄-Alkylsulfinyl, C₁-C₄-Haloalkylsulfinyl, C₁-C₄-Alkylsulfonyl, C₁-C₄-Haloalkylsulfonyl und im Falle cyclischer Reste auch C₁-C₄-Alkyl und C₁-C₄-Haloalkyl substituiert ist,
- R²⁷: einen Rest aus der Gruppe der für R²⁴ möglichen Reste, ausgenommen OH,
- R²⁸: H, OH, CHO, C₁-C₈-Alkyl, C₂-C₈-Alkenyl, C₂-C₈-Alkinyl, C₁-C₈-Alkoxy, C₂-C₈-Alkenyloxy, C₂-C₈-Alkinyloxy, C₁-C₈Alkylsulfonyl, C₂-C₈-Alkenylsulfonyl, C₂-C₈-Alkinylsulfonyl, (C₁-C₈-Alkyl)-carbonyl, (C₂-C₈-Alkenyl)-carbonyl, (C₂-C₈-Alkinyl)-carbonyl, (C₁-C₈-Alkoxy)-carbonyl, (C₂-C₈-Alkenyloxy)-carbonyl, (C₂-C₈-Alkinyloxy)-carbonyl, Mono- oder Di-(C₁-C₈-alkyl)-aminocarbonyl, C₃-C₈-Cycloalkyl, C₅-C₈-Cycloalkenyl, C₃-C₈-Cycloalkoxy, C₅-C₈-Cycloalkenyloxy, C₃-C₉-Cycloalkylsulfonyl, C₅-C₈-Cycloalkenylsulfonyl, C₃-C₈-Cycloalkoxycarbonyl, C₅-C₈-Cycloalkenyloxycarbonyl, Benzoyl, Benzyl, Benzylcarbonyl, Benzyloxycarbonyl, Aryl oder einen Heterocyclus, insbesondere Phenyl, Furyl, Pyridyl oder Thienyl, wobei jeder der letztgenannten 35 Reste unsubstituiert oder substituiert ist, vorzugsweise unsubstituiert oder durch einen oder mehrere Reste aus der Gruppe Halogen, NO₂, CN, C₁-C₄-Alkoxy, C₁-C₄-Haloalkoxy, C₁-C₄-Alkylthio, C₁-C₄-Haloalkylthio, NH₂, NH-CH₃ und N(CH₃)₂ und im Falle cyclischer Reste auch C₁-C₄-Alkyl und C₁-C₄-Haloalkyl substituiert ist, oder SO₂NH₂ oder CONH₂,
- R²⁹: einen Rest aus der Gruppe der für R²⁸ möglichen Reste, ausgenommen OH,
- R³⁰: einen Rest aus der Gruppe der für R¹⁹ möglichen Reste,
- R³¹: einen Reste aus der Gruppe der für R²⁰ möglichen Reste,
- oder: NR³⁰R³¹ gemeinsam einen heterocyclischen Ring, wie er entsprechend für NR¹⁹R²⁰ definiert ist,
- R³²: Wasserstoff, C₁-C₄-Alkyl, C₁-C₄-Haloalkyl, CHO, C₁-C₄-Alkylsulfonyl, C₂-C₄-Alkenyl, C₂-C₄-Alkinyl, C₂-C₄-Haloalkenyl, C₁-C₄-Haloalkyl, C₂-C₄-Haloalkinyl, C₁-C₄-Haloalkylsulfonyl, (C₁-C₄-Alkyl)-carbonyl, (C₁-C₄-Alkoxy)-carbonyl, (C₁-C₄-Haloalkyl)-carbonyl oder (C₁-C₄-Haloalkoxy)-carbonyl,
- R³³: einen Rest aus der Gruppe der für R³² möglichen Reste oder
- NR³²R³³: gemeinsam einen heterocyclischen Ring, wie er entsprechend für NR¹⁹R²⁰ definiert ist,
bedeuten.

Von besonderem Interesse sind auch erfindungsgemäße Verbindungen der Formel (I) oder deren Salze, worin
- A: einen Rest der genannten Formel
A₁, in der m=0 oder m=1 und U=CH₂ bedeutet, oder
A₂, in der m=0 bedeutet, oder
A₆, in der m=0 bedeutet, oder
A₇, in der m=0 bedeutet,
vorzugsweise einen Rest der genannten Formel A₁ oder A₂ mit jeweils m=0 bedeutet.

Von besonderem Interesse sind auch erfindungsgemäße Verbindungen (I) (inklusive Salze), worin Beine Gruppe der genannten Formel B1, B2 oder B3, insbesondere B2, bedeutet.

Bevorzugte Bedeutungen für einzelne Reste sind folgende:
- R =: Wasserstoff, C₂-C₃-Alkyl, C₁-C₃-Alkoxy, vorzugsweise Wasserstoff oder Methyl;
- R¹ =: H, CN, NO₂, Halogen, C₁-C₆-Alkoxy, C₁-C₆-Alkyl, C₁-C₆-Haloalkoxy, C₁-C₆-Haloalkyl, (C₁-C₆-Alkoxy)-carbonyl, (C₃-C₆-Cycloalkoxy)-carbonyl oder (C₃-C₆-Cycloalkyl)-methoxycarbonyl, wie z.B. 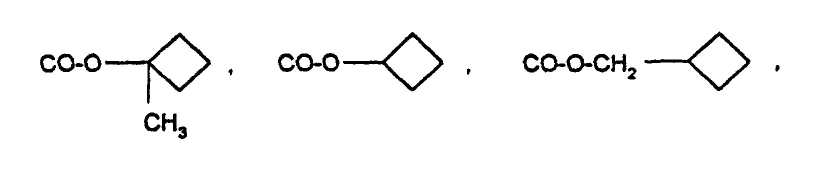 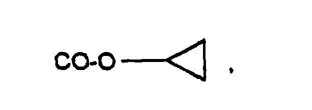 COOCH₃, COOC₂H₅ oder CO-O-CH(CH₃)₂, oder C₁-C₆-Alkylthio, C₁-C₆-Haloalkylthio, C₁-C₆-Alkylsulfonyl, C₁-C₆-Haloalkylsulfonyl, SO₂NH₂, CONH₂, Mono- oder Di-(C₁-C₄-alkyl)-aminocarbonyl oder -aminosulfonyl oder einen Rest der Formel 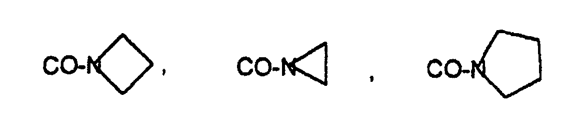 oder C₁-C₆-Alkylcarbonyl, C₁-C₆-Haloalkylcarbonyl, CHO, Cyclopropylcarbonyl, Cyclobutylcarbonyl oder NR²⁸R²⁹;
- R² =: Wasserstoff, C₁-C₆-Alkyl, C₁-C₆-Haloalkyl, C₁-C₆-Alkoxy, C₁-C₆-Haloalkoxy, Halogen oder CN;
- R³, R⁵, R⁷, R⁹, R¹¹, R¹³ und R¹⁴: ist vorzugsweise wie die bevorzugten Reste für R¹ definiert;
- R⁴, R⁶, R⁸, R¹⁰, R¹²: ist vorzugsweise wie bevorzugtes R² definiert;
- R¹⁵=: H, C₁-C₃-Alkyl oder Phenyl;
- R¹⁶=: H, NH₂, Mono- oder Di-(C₁-C₆-alkyl)amino, C₁-C₆-Alkyl, Phenyl oder C₁-C₆-Alkoxy, wobei die letztgenannten 5 Reste unsubstituiert oder durch einen oder mehrere Reste aus der Gruppe Halogen, C₁-C₃-Alkoxy, C₁-C₃-Haloalkoxy, CN, NO₂ und im Falle von Phenyl auch C₁-C₃-Alkyl und C₁-C₃-Haloalkyl substituiert sind;
- R¹⁷=: Mono- oder Di-(C₁-C₆-alkyl)amino, C₁-C₆-Alkyl, C₁-C₆-Alkoxy, Phenyl, Benzyl, wobei die 6 letztgenannten Reste unsubstituiert oder durch einen oder mehrere Reste aus der Gruppe Halogen, C₁-C₃-Alkoxy, C₁-C₃-Haloalkoxy und im Falle von Phenyl auch C₁-C₃-Alkyl und C₁-C₃-Haloalkyl substituiert sind, oder OH, NH₂ oder H;
oder der Teil der Formel R¹⁷-N=C-R¹⁶ aus der Gruppe der Formel B1 in der Definition für B gemeinsam einen Heterocyclus der Formel D1 oder D2, 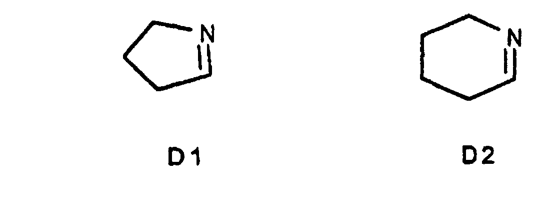
- R¹⁸=: H, C₁-C₆-Alkyl, C₁-C₆-Haloalkyl, insbesondere H, CH₃, C₂H₅, CF₃ oder CH₂CF₃,
- R¹⁹=: H, C₁-C₆-Alkyl, C₁-C₆-Haloalkyl, C₁-C₆-Haloalkoxy oder Phenyl,
- R²⁰=: H, C₁-C₆-Alkyl, C₁-C₆-Haloalkyl oder Phenyl oder
- NR¹⁹R²⁰: gemeinsam einen Heterocyclus der Formel 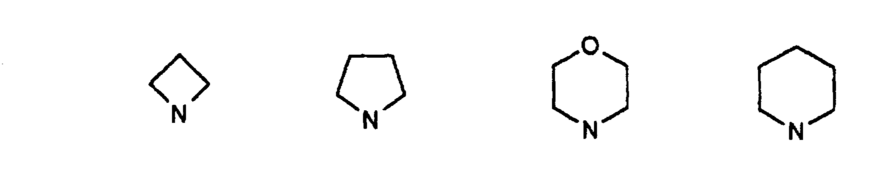 oder 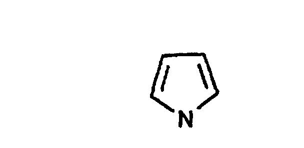
- R²¹ =: einen Rest aus der Gruppe der bevorzugten Reste für R¹⁶;
- R²² =: H, C₁-C₆-Alkyl, C₁-C₆-Haloalkyl oder Phenyl;
- R²³=: C₁-C₆-Alkyl oder C₁-C₆-Haloalkyl;
- R²⁸=: H, C₁-C₆-Alkyl, OH, C₁-C₆-Haloalkyl oder C₁-C₆-Alkoxy,
- R²⁹ =: H, C₁-C₆-Alkyl, C₁-C₆-Haloalkyl, CHO, C₁-C₃-Alkylsulfonyl, C₁-C₃-Alkoxycarbonyl oder C₁-C₃-Alkylcarbonyl;
- X, Y =: CI, F, CF₃, CH₃, CH₂CH₃, OCH₃ OC₂H₅, N(CH₃)₂, NHCH₃ oder OCH₂CF₃.

Weiterer Gegenstand der vorliegenden Erfindung sind Verfahren zur Herstellung der erfindungsgemäßen Verbindungen der Formel (I) oder deren Salze, dadurch gekennzeichnet, daß man
a) eine Verbindung der Formel (II)

   B-A-SO₂NH₂ (II)

   mit einem heterocyclischen Carbamat der Formel (III), 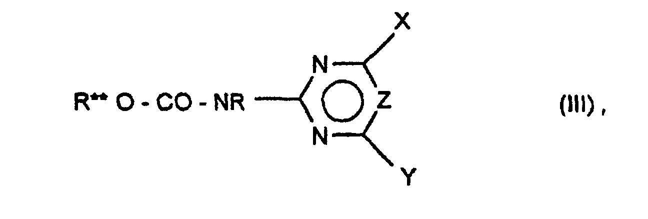 worin R** unsubstituiertes oder substituiertes Phenyl oder C₁-C₄-Alkyl bedeutet, umsetzt oder
b) ein Sulfonylisocyanat der Formel (IV)

   B-A-SO₂-N=C=O (IV)

   mit einem heterocyclischen Amin der Formel (V) 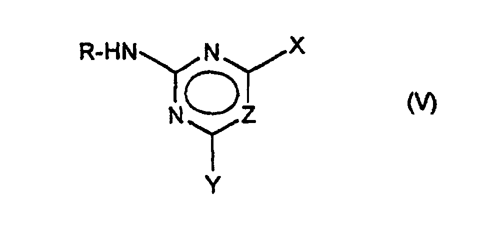 umsetzt oder
c) ein Sulfochlorid der Formel (VI)

   B-A-SO₂Cl (VI)

   mit einem heterocyclischen Amin der genannten Formel (V) in Gegenwart eines Cyanats, z.B. eines Alkalimetallcyanats wie Natrium- oder Kaliumcyanat, umsetzt oder
d) ein Sulfochlorid der genannten Formel (II) mit einem (Thio-)Isocyanat der Formel (VII) 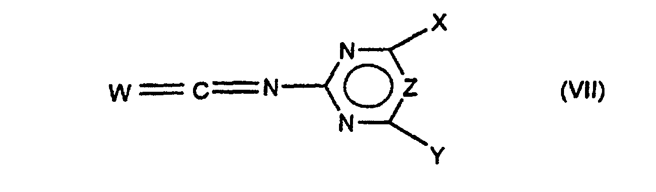 in Gegenwart einer geeigneten Base, wie z.B. Kaliumcarbonat oder Triethylamin, umsetzt,
e) im Falle daß B eine Gruppe ist, die ein Fragment der Formel -N=C-NR^{a}enthält, worin R^{a} NH₂, OH oder einen organischen Rest mit 1 bis 12 C-Atomen bedeutet, einen aminosubstituierten Sulfonylharnstoff der Formel (VIII), 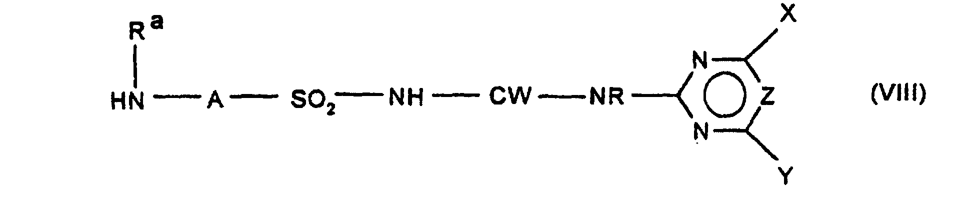 worin R^{a} wie oben definiert ist,
   mit geeigneten Reagenzien, die ein Fragment der Formel (IXa) enthalten, 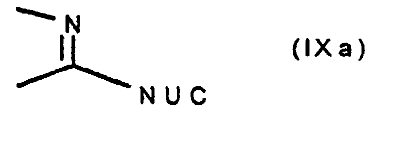 worin NUC eine nucleofuge Gruppe (Abgangsgruppe), z.B. Cl ist, umsetzt,
f) im Falle, daß B eine Gruppe ist, die ein Fragment der Formel 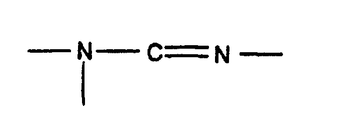 enthält,
   einen aminosubstituierten Sulfonylharnstoff der genannten Formel (VIII) (siehe e), worin R^{a} = Wasserstoff bedeutet,
   mit geeigneten Reagenzien, die ein Fragment der Formel (IXb) enthalten, 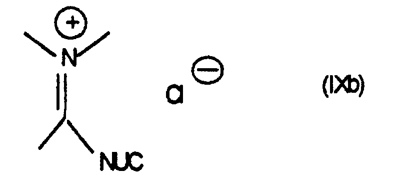
worin NUC eine nucleofuge Gruppe (Abgangsgruppe), z.B. CI, ist, umsetzt,
wobei in den Formeln (II) bis (IX) die Reste A, B, R, W, X, Y und Z wie in Formel (I) definiert sind, und wobei in den Varianten a) bis c) zunächst Verbindungen der Formel (I) erhalten werden, in denen W ein Sauerstoffatom bedeutet.

Die Sulfonamide der Formel (II), die Sulfonylisocyanate der Formel (IV) und die Sulfochloride der Formel (VI) sind neue Verbindungen und ebenfalls Gegenstand der Erfindung.

Die Umsetzung der Verbindungen der Formeln (II) und (III) erfolgt vorzugsweise basenkatalysiert in inerten Solventien, wie z.B. Dichlormethan, Acetonitril, Dioxan, Dimethylformamid (DMF), Dimethylessigsäureamid oder Tetrahydrofuran (THF), bei Temperaturen von -10°C bis zum Siedepunkt des jeweiligen Lösungsmittels. Als Basen werden dabei beispielsweise organische Aminbasen, wie 1,8-Diazabicyclo[5.4.0]undec-7-en (DBU), Triethylamin, oder Sauerstoffbasen wie Hydroxyde, wie z.B. Natrium- oder Kaliumhydroxyd, oder Alkoholate, wie z.B. Natriummethylat, Kalium-tert.-butylat oder Natriumphenolat, oder Carbonate, wie z.B. Natrium- oder Kaliumcarbonat (vgl. z.B. EP-A-44807) verwendet.

Die Sulfonamide der Formel (II) können z.B. aus den Verbindungen der Formel (X) durch Reaktion der Aminofunktion mit geeigneten Reagenzien, wie z.B. Verbindungen der Formel (IX), Orthoestern oder Amidacetalen, zu den Verbindungen der Formel B-A-SO₂NH-^{t}Bu (XI) und nachfolgender Abspaltung der tert-Butylgruppe aus Verbindungen der Formel (XI) mit Säuren erhalten werden.

Beispiele für bevorzugte Zwischenverbindungen der Formel (XI) und (II) sind in Schema 1 und Schema 2 aufgeführt, wobei die Reste A und R¹⁷ bis R²³ die weiter oben genannte Bedeutung haben; die nicht beispielhaft genannten Verbindungen können in analoger Weise, ggf. unter Zuhilfenahme von zusätzlichen üblichen Methoden, hergestellt werden.

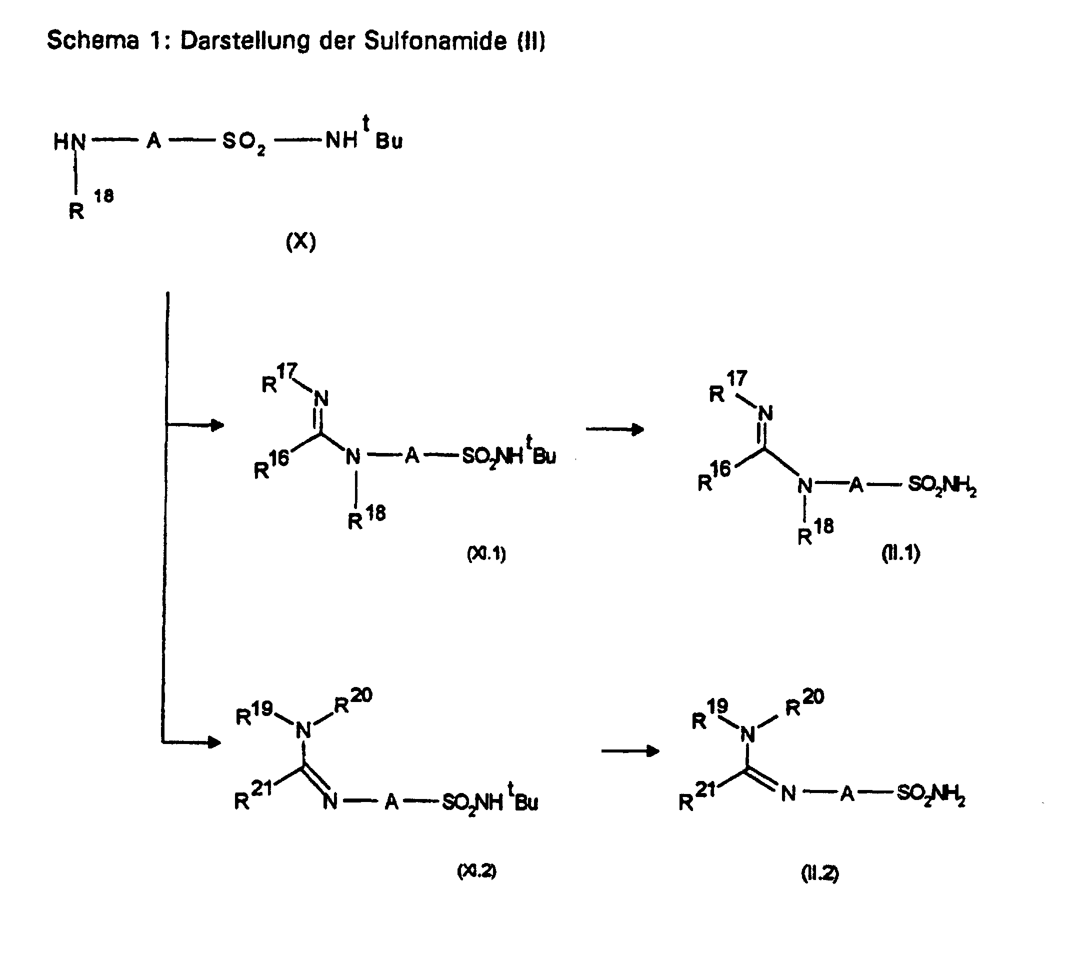

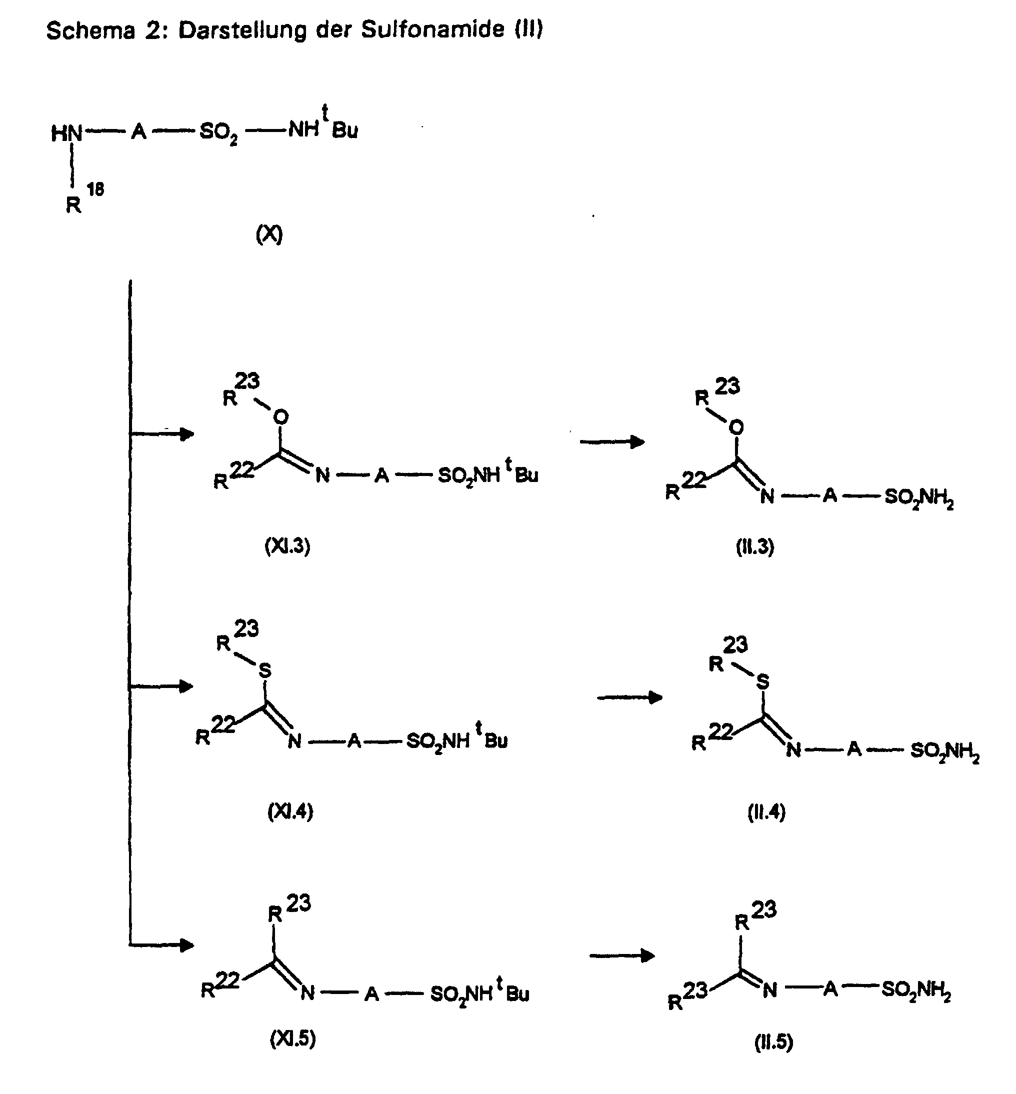

Durch eine Reihe von Standard-Reaktionen lassen sich die Verbindungen der Formel (XI) darstellen:
· Verbindungen der Formel (XI.1) lassen sich z.B. durch Umsetzung von Verbindungen des Typs der genannten Formel (IX.a) (Houben-Weyl-Falbe, "Methoden der organischen Chemie", 4. Aufl. , Bd. E 5/1, S. 628 f) mit Sulfonamiden der Formel (X) gewinnen (Houben-Weyl-Falbe, "Methoden der organischen Chemie", 4. Aufl. Bd. E5/2 S. 1305ff).
· Für den Fall, daß R²¹ Wasserstoff oder ein über ein Kohlenstoffatom gebundener Substituent bedeutet, lassen sich Verbindungen der Formel (XI.2) durch Reaktion der Sulfonamide (X) z.B. mit Salzen der Formel (IX.b) oder Carbonsäureamidacetalen herstellen.
· Für den Fall, daß R²¹ einen über ein Heteroatom gebundener Substituent bedeutet, lassen sich die Verbindungen der Formel (XI.2) (Iso(thio)harnstoffe, Guanidine) z.B. durch Umsetzung von Halogenformamidinen der Formel (XII) mit Alkoholen, Aminen oder Thiolen darstellen.

Halogenformamidine der Formel (XII) sind z.B. durch Reaktion von Harnstoffen der Formel (XIII) mit Halogenierungsmittel wie z.B. Thionylchlorid zugänglich (Houben-Weyl-Hagemann, "Methoden der organischen Chemie", 4. Aufl. Bd. 4); siehe Schema 3. 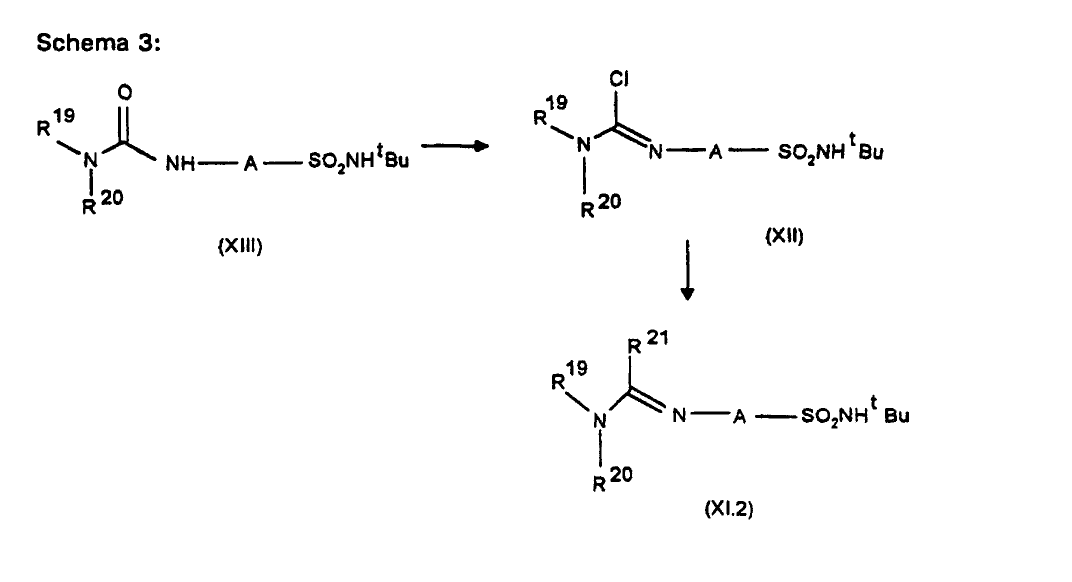
· Iminoester der Formel (XI.3) lassen sich z.B. durch Umsetzung von Orthoestern mit Anllinen der Formel (X) (R¹⁸ = H) gewinnen (Houben-Weyl-Falbe, "Methoden der organischen Chemie", 4. Aufl, Bd. 5/1, S. 812 ff).
· Iminokohlensäure-Derivat der Formel (XI.3) (R²² ist ein über das Heteroatom gebundener Substituent) lassen sich z.B. aus entsprechenden Isocyaniddichloriden (= Verbindungen der Formel (XIV), ohne Abb.) durch sukzessive Substitution der Chloratome durch Alkoxy-, Amino- und Alkylmercaptogruppen darstellen. Die Verbindungen der Formel (XIV) lassen sich beispielsweise aus den Sulfonamiden (X) (R¹⁸ = H) durch Reaktion mit Thiophosgen und anschließende Chlorierung gewinnen (Houben-Weyl-Hagemann, "Methoden der organischen Chemie", 4. Aufl., Bd. E4)
· Verbindungen der Formel (XI.4) (R²² ist Wasserstoff oder ein über Kohlenstoff gebundener Substituent) sind beispielsweise durch die Überführung von Anilinen der Formel (X) (R¹⁸ = H) in Thiocarbonsäureamide der Formel (XV) und anschließende S-Alkylierung zugänglich (Houben-Weyl-Falbe, "Methoden der organischen Chemie", 4. Aufl. Bd E5, S. 931) 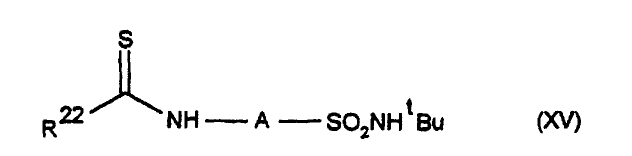
· Imine der Formel (XI.5) können z.B. durch Kondensation des Anilins (X) (R¹⁸ = H) mit Aldehyden und Ketonen bzw. mit Acetalen dargestellt werden (s. Dayagi, Y. Degani in "The Chemistry of the Carbon-Nitrogen Double Bond" (s. Patai (Ed.). s. 61ff, Wiley, London-New York-Sydney-Toronto 1970; R.W. Layer, Chem. Rev. 1963, 489).

Die Abspaltung der tert.-Butyl-Schutzgruppe aus den Verbindungen der Formel (XI) zu den Sulfonamiden der Formel (II) erfolgt durch Umsetzung mit einer starken Säure. Als Säuren kommen z.B. Mineralsäuren, wie z.B. Salz- und Schwefelsäure, oder organische Säuren, wie z.B. Trifluoressigsäure oder Ameisensäure, in Frage. Die Abspaltung der tert.-Butylgruppe erfolgt bei Temperaturen zwischen -20°C und der jeweiligen Rückflußtemperaturen des Reaktionsgemisches, vorzugsweise bei -10°C bis 60°C. Die Umsetzung kann in Substanz oder auch in einem inerten Solvens, wie z.B. Dichlormethan oder Trichlormethan, durchgeführt werden.

Die für die Umsetzung benötigten Carbamate sind literaturbekannt oder lassen sich analog bekannten Verfahren herstellen (vgl. EP-A-70804; US-A-4,480,101; EP-A-562575; EP-A-562576).

Die Umsetzung der Verbindungen der Formel (IV) mit heterocyclischen Aminen der Formel (V) führt man vorzugsweise in inerten, aprotischen Solventien, wie z.B. Dioxan, Acetonitril, Chlorbenzol oder Tetrahydrofuran, bei Temperaturen zwischen -10°C und der Siedetemperatur des Lösungsmittels durch.

Die Sulfonylisocyanate der Formel (IV) lassen sich analog literaturbekannter Verfahren (z.B. EP-A-184 385) aus den Sulfonamiden der Formel (II) herstellen. So führt beispielsweise die Umsetzung des Sulfonamides (II) mit Phosgen in inerten Solventien, wie z.B. Dioxan, Acetonitril, Chlorbenzol oder Tetrahydrofuran bei Temperaturen zwischen 0°C und der Siedetemperatur des Lösungsmittels zu den Verbindungen der Formel (IV).

Die Umsetzung der Sulfochloride (VI) mit den Aminoheterocyclen der Formel (V) und Cyanaten wie Natriumcyanat und Kaliumcyanat erfolgt z.B. in aprotischen Solventien, wie z.B. Acetonitril, gegebenenfalls in Gegenwart von Basen, z.B. 0,5 bis 2 Äquivalenten Base, oder in basischen aprotischen Solventien bei Temperaturen zwischen -10 und 100°C, vorzugsweise zwischen -10 und 60°C.

Als Base oder basische aprotische Solventien kommen, z.B. Pyridin, Picolin oder Lutidin oder eine Mischung aus diesen in Betracht (vgl. US-A-5,157,119).

Die Reaktion der Sulfonamide der Formel (II) mit einem (Thio)Isocyanat der Formel (VII) erfolgt analog literaturbekannten Verfahren (z.B. EP-A-232067, EP-A-166516) bei -10 bis 150°C, vorzugsweise 20 bis 100°C, in einem inerten Lösungsmittel wie z.B. Acetonitril, in Gegenwart einer geeigneten Base, wie z.B. Triethylamin oder Kaliumcarbonat.

Die Reaktion der aminosubstituierten Sulfonylharnstoffe der Formel (VIII) oder deren Salze mit den Reagentien der Formel (IX) zu den Sulfonylharnstoffen der Formel (I) läßt sich bei Temperaturen von -20°C bis zum Siedepunkt des jeweiligen Solvens, vorzugsweise von -10°C bis 150°C insbesondere von
- 10°C bis 100°C, durchführen. Geeignete Solventien sind organische Lösungsmittel, z.B.
- Ether wie Tetrahydrofuran, Diethylether, tert.-Butyl-methylether, Dimethoxyethan oder Dioxan,
- Ester wie z.B. Ethylacetat, Butylacetat oder Methylacetat,
- Kohlenwasserstoffe oder Halogenkohlenwasserstoff wie Toluol, Xylol, Chlorbenzol oder Chlortoluol,
- Amide und Nitrile wie z.B. Dimethylformamid (DMF), N,N-Dimethylessigsäureamid, N-Methylpyrrolidon, Acetonitril oder Propionitril,
- Basische Solventien wie z.B. Pyridin oder Lutidin oder auch Gemische der einzelnen Solventien.

Die Sulfonylharnstoffe der Formel (VIII) sind bekannt oder können analog bekannten Verfahren hergestellt werden (z.b. EP-A-1515, EP-A-23141, US-A-4,225,337, US-A-4,310,346, US-A-4,453,971, US-A-4,877,442, US-A-4,892,946, WO-89/19921).

Die Reagentien der Formel (IX), z.B. (IXa) und (IXb), sind ebenfalls literaturbekannt oder lassen sich nach literaturbekannten Methoden darstellen (Houben-Weyl-Falbe, "Methoden der organischen Chemie", 4. Aufl., Bd. E5 Houben-Weyl-Hagemann, "Methoden der organischen Chemie", 4. Aufl., Bd. E4).

Die Verbindungen der allgemeinen Formel (XII), welche Verbindungen der Formel (II), (IV), (VI) und (XI) umfaßt, sind neu und ebenfalls Gegenstand dieser Erfindung:

B-A-SO₂-R^{Z} (XIII)

Dabei bedeutet RZ = NH₂, Mono- oder Di-(C₁-C₄-alkyl)amino, Halogen oder -N=C=O; B und A sind wie in Formel (I) definiert.

Die Salze der Verbindungen der Formel (I) werden vorzugsweise in inerten Lösungsmitteln wie z.B. Wasser, Methanol, Aceton, Dichlormethan, Tetrahydrofuran, Toluol oder Heptan, bei Temperaturen von 0 bis 100°C hergestellt. Geeignete Basen zur Herstellung der erfindungsgemäßen Salze sind beispielsweise Alkalicarbonate, wie Kaliumcarbonat, Alkali- und Erdalkalihydroxide, wie NaOH, KOH und Ca(OH)₂, Ammoniak oder eine geeignete Aminbase, wie Triethylamin oder Ethanolamin. Als Säuren zur Salzbildung eignen sich z.B. HCI, HBr, H₂SO₄ oder HNO₃.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die erflndungsgemäßen Verbindungen der Formel (I) und deren Salze, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungspektrum auf Arten wie z.B. Galium, Viola, Veronica, Lamium, Stellaria, Amaranthus, Sinapis, tpomoea, Matricaria, Abutilon und Sida auf der annuellen Seite sowie Sonvolvulus, Cirsium, Rumex und Artemisia bei den perennierender Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen monound dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptlonsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I) oder deren Salze.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-% .

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie in z.B. aus Weed Research 26, 441-445 (1986), oder "The Pesticide Manual", 10th edition, The British Crop Protection Council and the Royal Soc. of Chemistry, 1994 und dort zitierter Literatur beschrieben sind. Als literaturbekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxyl-2-nitrophenyl]-2-methoxyethylidene)-amino]-oxylessigsäure und -essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazamethabenz-methyl; imazapyr; imazaquin und Salze wie das Ammoniumsalz; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monocarbamide dihydrogensulfate; monolinuron; monuron; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthelenyl]-oxy]-propansäure und -methylester; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thiazopyr (Mon-13200); thidiazimin (SN-24085); thifensutfuron-methyl; thiobencarb; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

### A. Chemische Beispiele

### Darstellung von N-tert.-Butyl-2-methoxycarbonyl-5-(2-dimethylamino-1-azaethenyl)-benzolsulfonamid

Ein Gemisch aus 3,15 g N-tert.-Butyl-4-amino-2-methoxycarbonylbenzolsulfonamid, 2,2 ml N,N-Dimethylformamiddimethylacetal und 60 ml N,N-Dimethylformamid wird bei 50-60°C bis zur vollständigen Umsetzung gerührt. Anschließend werden die flüchtigen Komponenten unter reduziertem Druck abdestilliert. Der Rückstand wird mit wenig Diisopropylether gewaschen. Man erhält so 3,52 g vom gewünschten Amidin-Derivat.
1H-NMR (D₆DMSO, 300 MHz)
δppm :
7,9 (s, 1H, CH=N)
7,6 (d, 1H)
7,5 (d, 1H)
7,2 (dd, 1H)
6,9 (s, 1H, NH)
3,8 (s, 3H, OCH₃)
3,1 (s, 3H, N-CH₃)
3.0 (s, 3H, N-CH₃)
1,1 (s, 9H, C(CH₃)₃)

### Darstellung von 5-(2-Dimethylamino-1-azaethenyl)-2-methoxycarbonylbenzolsulfonamid

Ein Gemisch aus 2,13 g N-tert.-Butyl-2-methoxy-carbonyl-5-(2-dimethylamino-1-azaethenyl)-benzolsulfonamid und 20 ml Trifluoressigsäure wird bei Raumtemperatur bis zur vollständigen Umsetzung gerührt. Anschließend werden die flüchtigen Bestandteile unter reduziertem Druck abdestilliert.
Der Rückstand wird mit Diisopropylether und anschließend mit NaHCO₃-Lösung gewaschen. Man erhält so 1,77 g der gewünschten Verbindung.
1H-NMR (D₆DMSO, 200 MHz)
δ ppm:
7,9 (s, 1H, CH=N)
7,6 (d, 1H)
7,5 (d, 1H)
7,2 (m, 3H)
3,8 (s, 3H, OCH₃)
3,1 (s, 3H, NCH₃)
3,0 (s, 3H, NCH₃)

Darstellung von 2-[[[[(4,6-Dimethoxypyrimidin-2-yl)amino]carbonyl]amino]-sulfonyl]-4-(2-dimethylamino-1-azaethenyl)-benzoesäuremethylester
(vgl. Tabelle 2, Bsp. Nr. 2-10)

Eine Suspension aus 1,50 g 5-(2-Dimethylamino-1-azaethenyl)-2-methoxycarbonyl-benzolsulfonamid, 1,61 g 4,6-Dimethoxy-2-phenoxycarbonylaminopyrimidin, 32 ml Acetonitril und 0,83 ml DBU werden zunächst bei 0°C, anschließend bei Raumtemperatur bis zur vollständigen Umsetzung gerührt. Nach beendeter Reaktion wird das Reaktionsgemisch unter reduziertem Druck eingeengt. Der Rückstand wird in Wasser aufgenommen und mit Diethylether gewaschen. Nach dem vorsichtigen Ansäuern der wäßrigen Phase mit konz. Salzsäure (pH 6) wird der ausgefallene Sulfonylharnstoff abgetrennt, mit Diisopropylether und Wasser gewaschen und getrocknet. Man erhält so 1,89 g des gewünschten Sulfonylharnstoffes mit dem Schmelzpunkt 73-75°C;
1H-NMR (D₆-DMSO, 200 MHz)
δ ppm:
12,5 (s, 1H)
10,5 (s, 1H)
8,0 (s, 1H)
7,7 (d, 1H)
7,6 (d, 1H)
7,3 (dd, 1H)
6,0 (s, 1H)
4,0 (s, 6H)
3,8 (s, 3H)
3,1 (s, 3H)
3,0 (s, 3H)

### Darstellung von 2-[[[[(4,6-Dimethoxypyrimidin-2-yl)amino]carbonyl]amino]-sulfonyl]-4-(2-dimethylamino-1-azaethenyl)-benzoesäuremethylester-Natriumsalz (Tabelle 2, Bsp.-Nr. 2-46).

Zu einem Gemisch aus 1,9 g 2-[[[[(4,6-Dimethoxypyrimidin-2-yl)amino]carbonyl]-amino]sulfonyl]-4-(2-dimethylamino-1-azaethenyl)-benzoesäuremethylester und 57 ml Dichlorethan werden 0,75 ml einer 30 %igen Natriummethylat-Lösung zugetropft. Nach beendeter Reaktion wird das Reaktionsgemisch unter reduziertem Druck scharf eingeengt. Man erhält so 1,95 g der gewünschten Verbindung mit dem Schmelzpunkt 103-104°C (unter Zers.);
1H-NMR (D₆-DMSO, 300 MHz)
δ ppm:
8,6 m, (s, 1H)
7,8 (s, 1H)
7,5 (d, 1H)
7,3 (d, 1H)
7,0 (dd, 1H)
5,7 (s, 1H)
3,8 (s, 6H)
3,7 (s, 3H)
3,1 (s, 3H)
3,0 (s, 3H)

### Darstellung von 2-([[[(4,6-Dimethoxypyrimidin-2-yl)-amino]carbonyl]amino]-sulfonyl]-4-(2-dimethylamino-1-azaethenyl)-benzoesäuredimethylamid (Tabelle 2, Bsp. Nr. 2-15)

Zu einer Mischung aus 0,80 g 4-Amino-2-[[[[(4,6-dimethoxypyrimidin-2-yl)amino]carbonyllamino]sulfonyl]-benzoesäuredimethylamid und 8 ml DMF werden 0,24 g Chlormethylen-N,N-dimethylammoniumchlorid zugesetzt. Bei Raumtemperatur wird anschließend bis zur vollständigen Umsetzung nachgerührt. Unter reduziertem Druck werden nun flüchtige Komponenten abdestilliert. Der Rückstand wird mit Wasser und Essigsäureethylester gewaschen. Nach dem Trocknen erhält man 0,64 g des gewünschten Produktes. Schmelzpunkt: 140-141°C (unter Zers.)

Darstellung von N-[(4,6-Dimethoxypyrimidin-2-yl)aminocarbonyl]-2-(2-dimethylamino-1-azaethenyl)-benzolsulfonamid (Tabelle 1, Bsp. Nr. 1-1)

Eine Mischung aus 0,80 g N-[(4,6-Dimethoxypyrimidin-2-yl)-aminocarbonyl]-2-aminobenzolsulfonamid, 0,29 g Chlormethylen-N,N-dimethylammoniumchlorid und 8 ml DMF wird bei Raumtemperatur bis zur vollständigen Umsetzung gerührt. Nach dem Abdestillieren der flüchtigen Komponenten unter reduziertem Druck und dem Waschen des Rückstandes mit Wasser und Essigsäuremethylester erhält man 0.28 g des gewünschten Produktes. Schmelzpunkt: 209-210°C (unter Zers.)

Die in den nachfolgenden Tabellen beschriebenen Verbindungen erhält man auf analoge Weise zu den vorstehenden Herstellungsbeispielen, gegebenenfalls unter Anwendung der weiter oben genannten allgemeinen Methoden.

Folgende Abkürzungen werden in den Tabellen verwendet:
Nr. = Beispielnummer
Fp. = Festpunkt (Schmelzpunkt) in °C
Me = Methyl
Et = Ethyl
Pr = ⁿPr = n-Propyl
ⁱPr = i-Propyl
^{c}Pr = Cyclopropyl
^{t}Bu = t-Butyl
ⁿBu = n-Butyl
^{c}Bu = Cyclobutyl
Ph = Phenyl
(Z) = Zersetzung

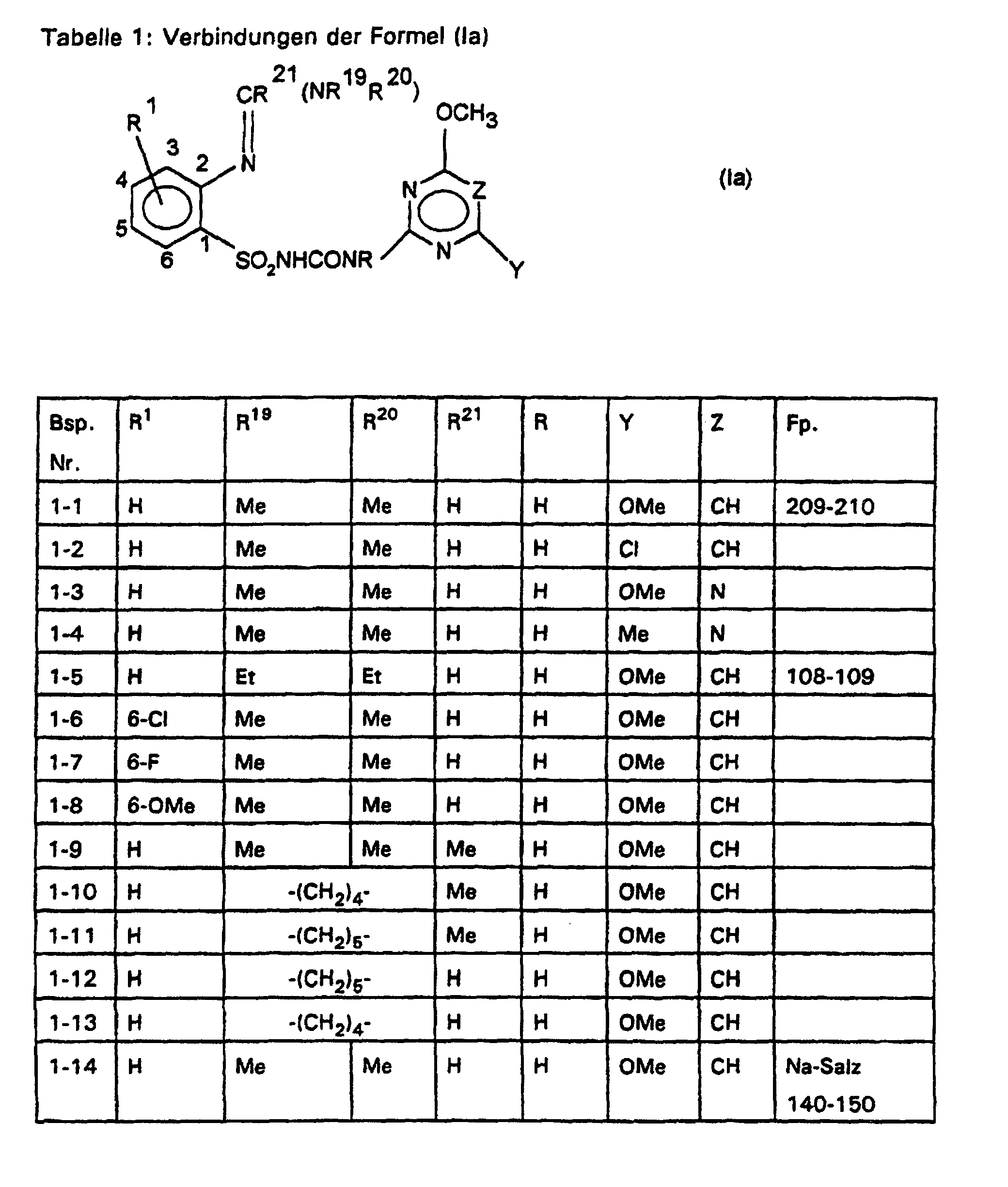

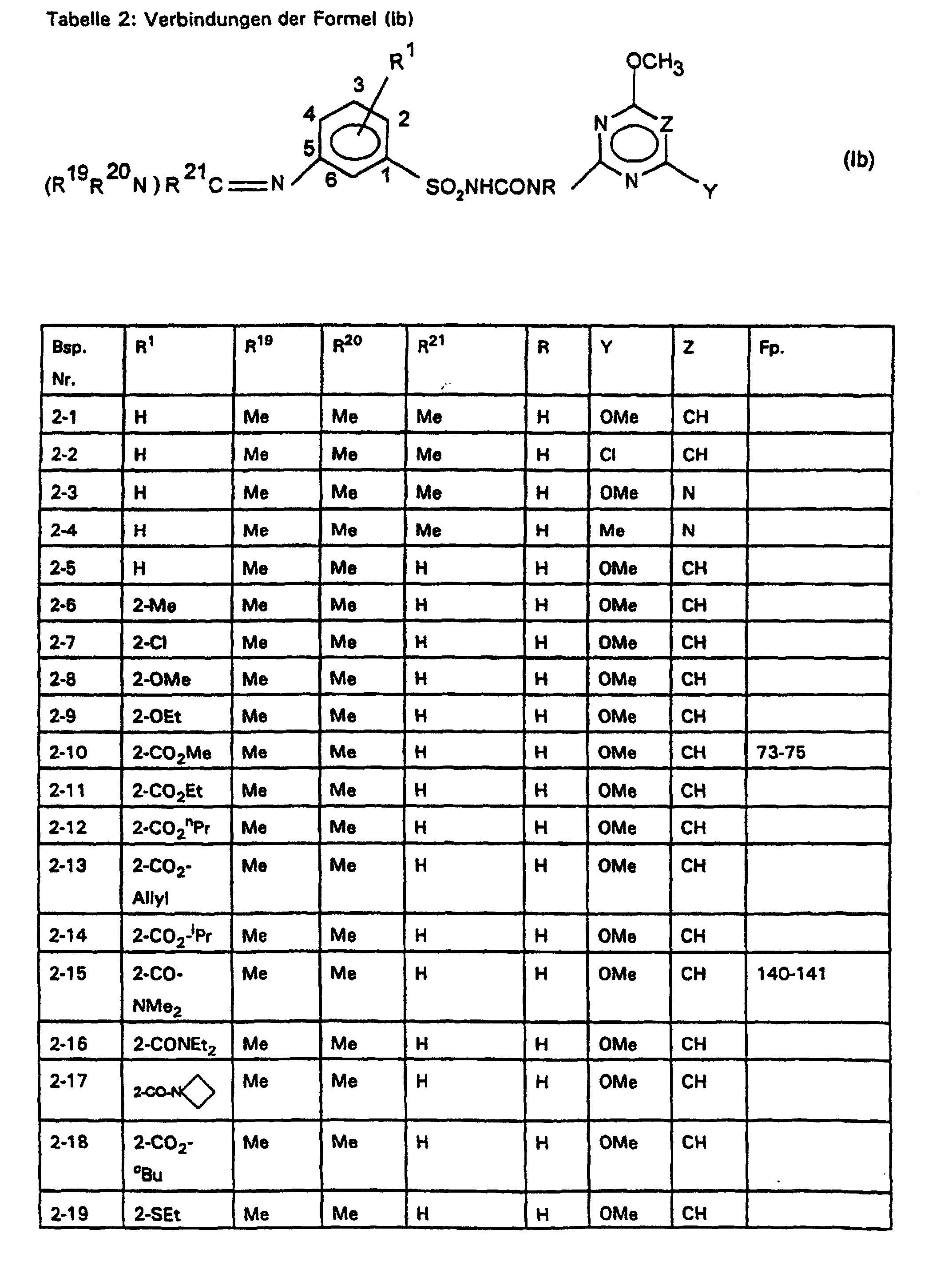

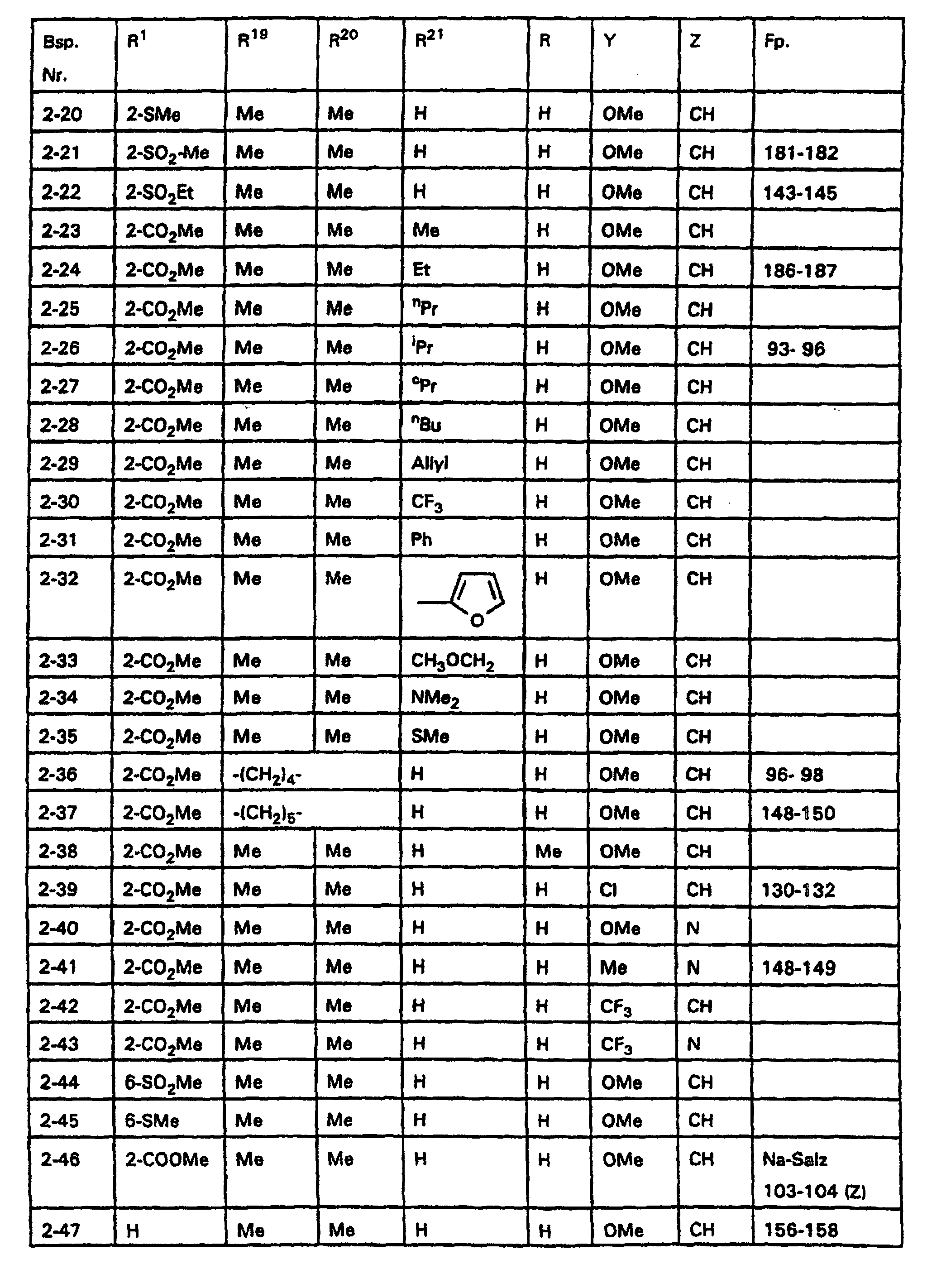

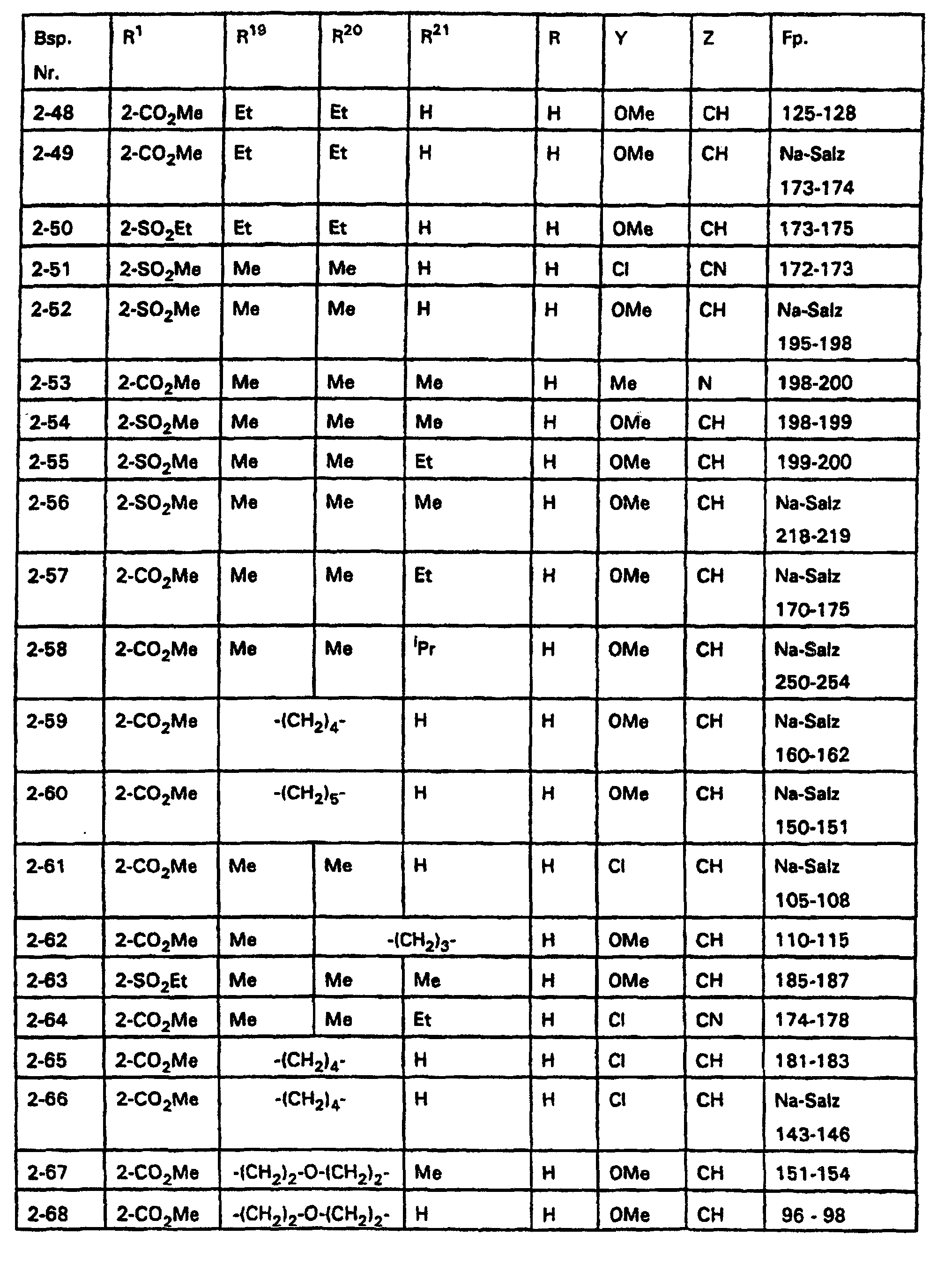

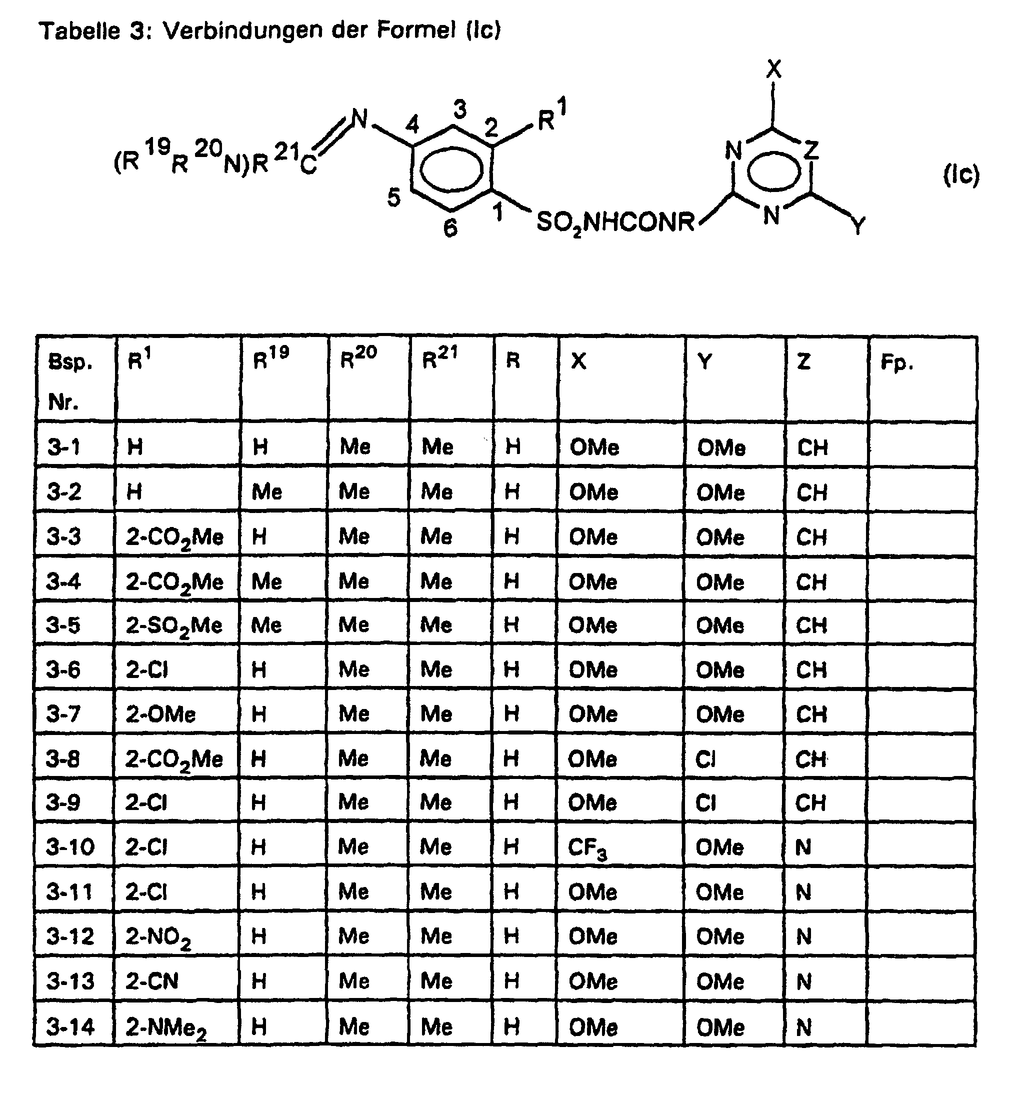

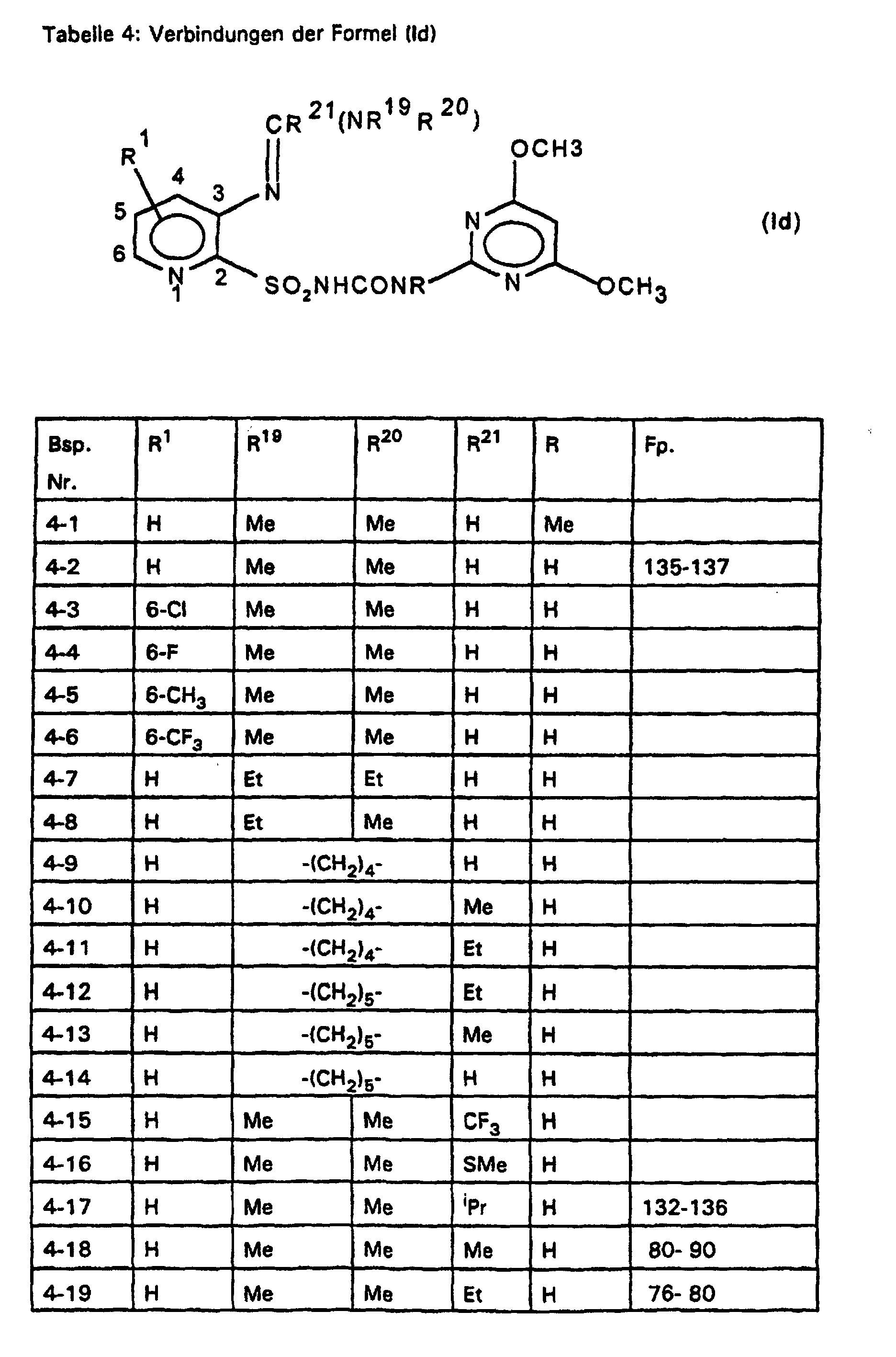

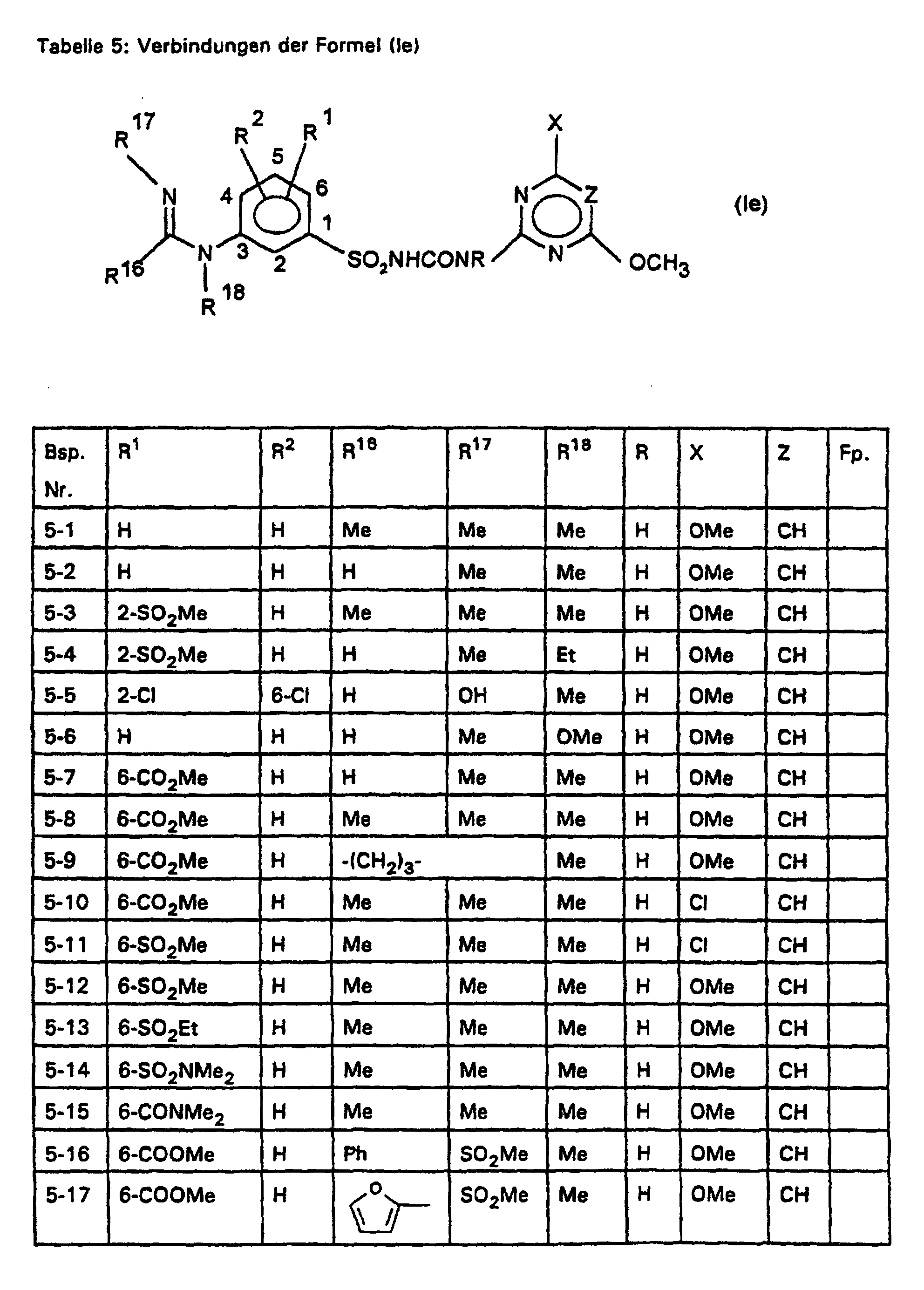

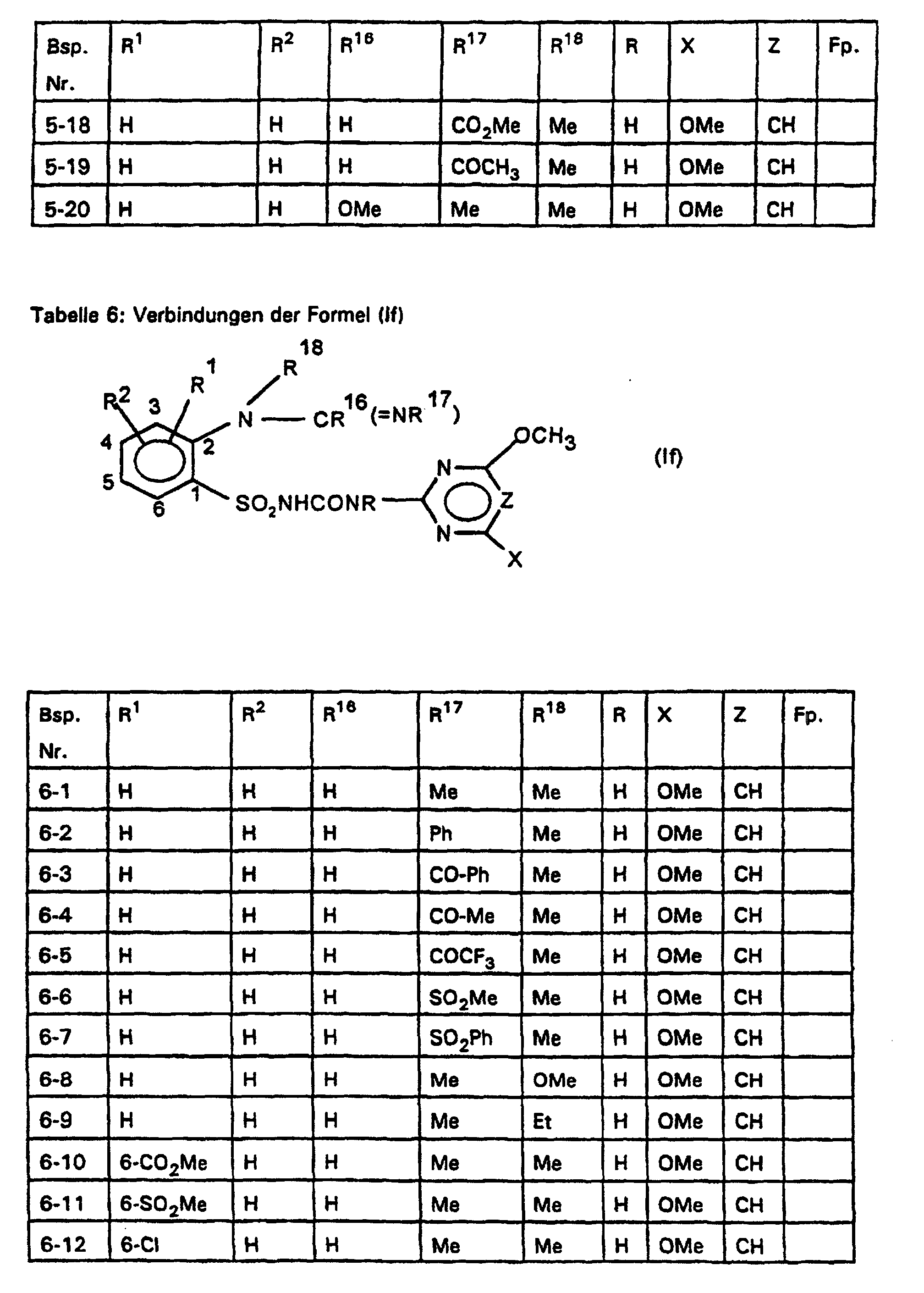

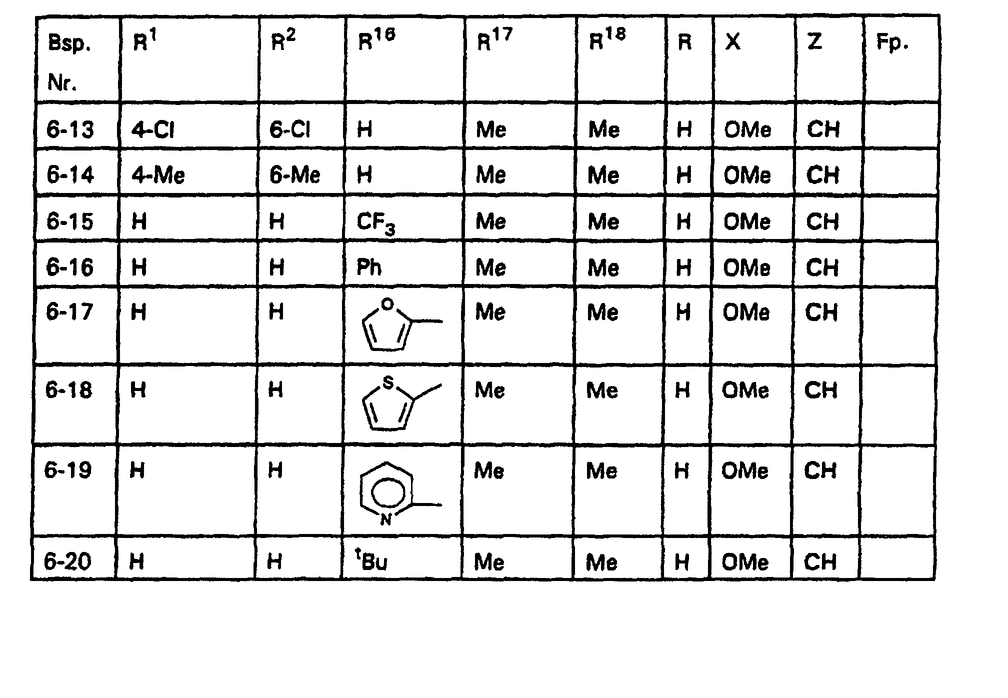

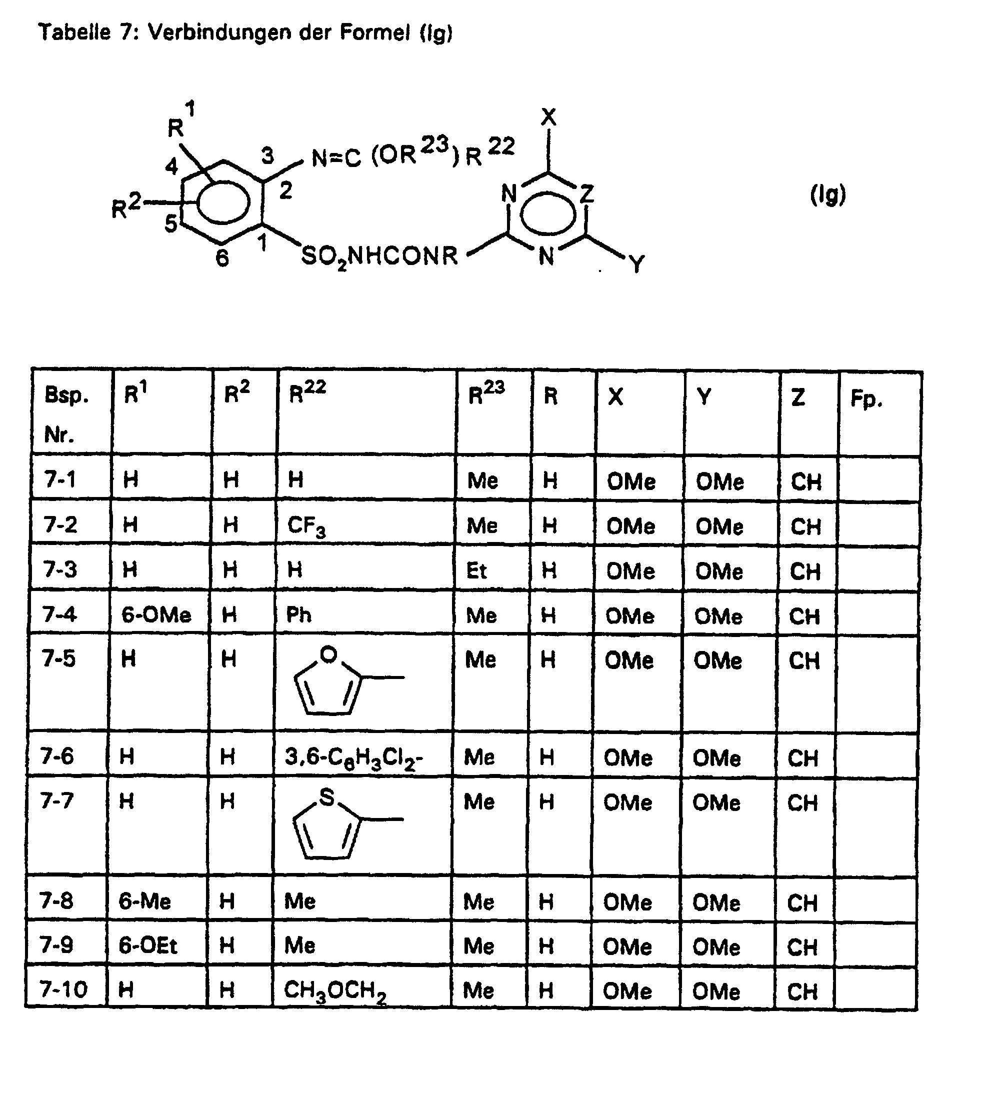

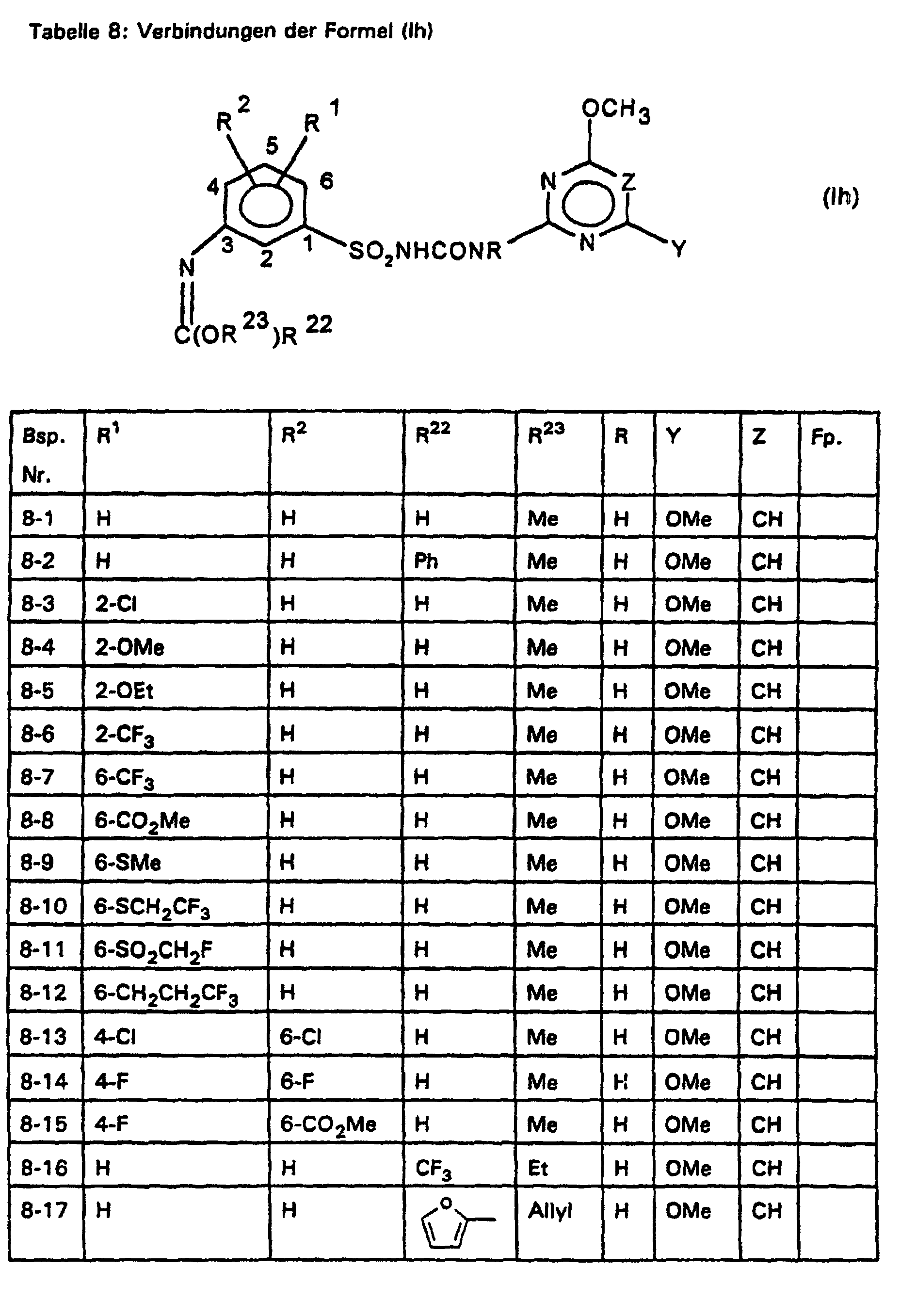

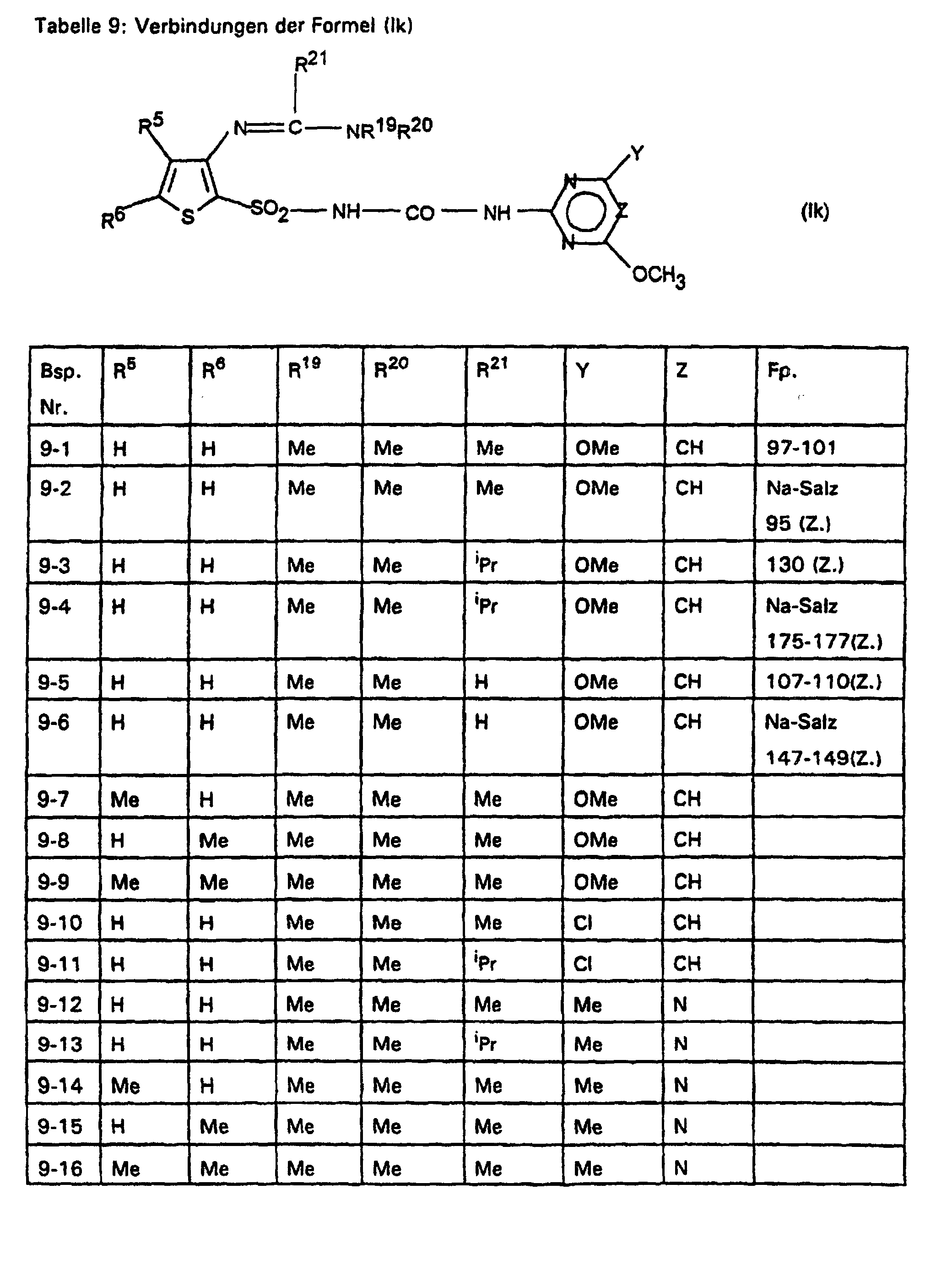

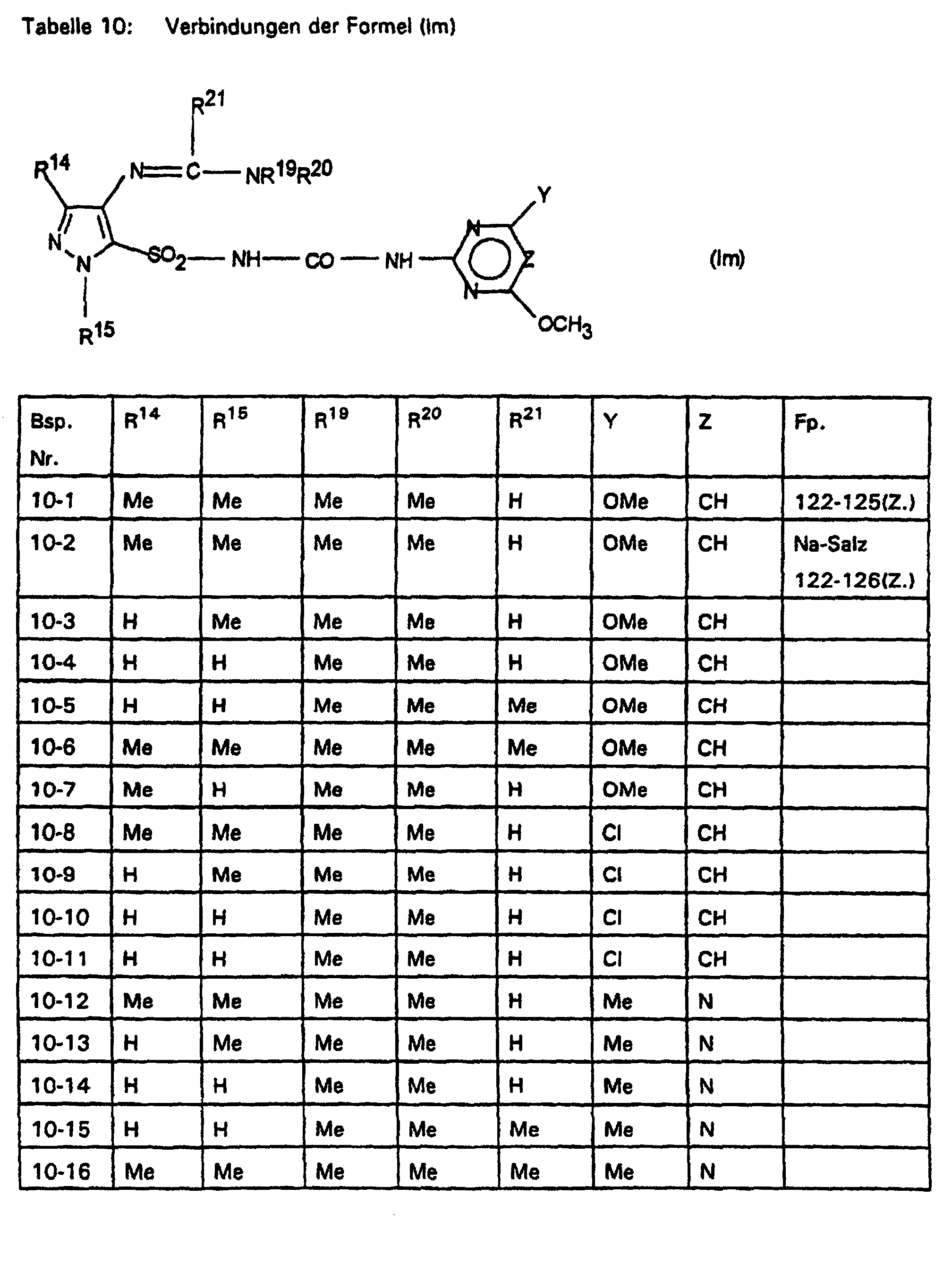

### B. Formulierungsbeispiele

a) Ein Stäubemittel wird erhalten, indem man 10 Gew.-Teile einer Verbindung der Formel (I) und 90 Gew.-Teile Talkum als Inerstoff mischt und in einer Schlagmühle zerkleinert.
b) Ein in Wasser leicht dispergierbares benetzbares Pulver wird erhalten, indem man 25 Gewichtsteile einer Verbindung der Formel (I), 64 Gewichtsteile kaolinhaltigen Quarz als Inerstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gewichtsteilen Alkylphenolpolyglykolether (®Triton X 207), 3 Gewichtsteilen Isotridecanolpolyglykolether (8 EO) und 71 Gewichsteilen paraffinischen Mineraöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gewichsteilen einer Verbindung der Formel (I), 75 Gewichsteilen Cyclohexanon als Lösungsmittel und 10 Gewichtsteilen oxethyliertes Nonylphenol als Emulgator.
e) Ein in Wasser dispergierbares Granulat wird erhalten indem man 75 Gewichsteile einer Verbindung der Formel (I), 10 Gewichsteile ligninsulfonsaures Calcium, 5 Gewichtsteile Natriumlaurylsulfat, 3 Gewichtsteile Polyvinylalkohol und 7 Gewichsteile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man 25 Gewichsteile einer Verbindung der Formel (I), 5 Gewichtsteile 2,2'-dinaphthylmethan-6,6'-disulfonsaures Natrium 2 Gewichtsteile oleoylmethyltaurinsaures Natrium, 1 Gewichtsteil Polyvinylalkohol, 17 Gewichsteile Calciumcarbonat und 50 Gewichsteile Wasser auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen bzw. Rhizomstücke von mono- und dikotylen Unkrautpflanzen werden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen der Formel (I) oder deren Salze werden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung werden die Töpfe im Gewächshaus aufgestellt und unter guten Wachstumsbedingungen für die Unkräuter gehalten. Die optische Bonitur der Pflanzen- bzw. der Auflaufschäden erfolgt nach dem Auflaufen der Versuchspflanzen nach einer Versuchszeit von 3 bis 4 Wochen im Vergleich zu unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise haben die Verbindungen der Herstellungsbeispiele Nr. 1-1, 1-5, 1-14, 2-10, 2-15, 2-21, 2-22, 2-24, 2-26, 2-36, 2-37, 2-39, 2-41, 2-47, 2-48, 2-49, 2-50, 2-51 bis 2-68, 4-2, 4-17, 4-18, 4-19, 9-1, 9-2, 9-3, 9-4, 9-5, 9-6, 10-1 und 10-2 (siehe Abschnitt A.) sehr gute herbizide Wirkung gegen Schadpflanzen wie Sinapis alba, Stellaria media, Chrysanthemum segetum und Lolium muitiflorum im Vorauflaufverfahren bei einer Aufwendmenge von 0,3 kg bis 0,005 kg Aktivsubstanz pro Hektar.

### 2. Unkrautwirkung im Nachauflauf

Samen bzw. Rhizomstücke von mono- und dikotylen Unkräutern werden in Plastiktöpfen in sandigem Lehmboden ausgelegt, mit Erde abgedeckt und im Gewächshaus unter guten Wachstumsbedingungen angezogen. Drei Wochen nach der Aussaat werden die Versuchspflanzen im Dreiblattstadium behandelt. Die als Spritzpulver bzw. als Emulsionskonzentrate formulierten erfindungsgemäßen Verbindungen der Formel (I) oder deren Salze werden in verschiedenen Dosierungen mit einer Wasseraufwandmenge von umgerechnet 600 bis 800 l/ha auf die grünen Pflanzenteile gesprüht. Nach ca. 3 bis 4 Wochen Standzeit der Versuchspflanzen im Gewächshaus unter optimalen Wachstumsbedingungen wird die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Herstellungsbeispiele Nr. 1-1, 1-5, 1-14, 2-10, 2-15, 2-21, 2-22, 2-24, 2-26, 2-36, 2-37, 2-39, 2-41, 2-47, 2-48, 2-49, 2-50, 2-51 bis 2-68, 4-2, 4-17, 4-18, 4-19, 9-1, 9-2, 9-3, 9-4, 9-5, 9-6, 10-1 und 10-2 (siehe Abschnitt A.) sehr gute herbizide Wirkung gegen Schadpflanzen wie Sinapis alba, Stellaria media, Chrysanthemum segetum und Lolium multiflorum im Nachauflaufverfahren bei einer Aufwandmenge von 0,3 kg bis 0,005 kg Aktivsubstanz pro Hektar.

### 3. Kulturpflanzenverträglichkeit

In welteren Versuchen im Gewächshaus wurden Samen einer größeren Anzahl von Kulturpflanzen und Unkräutern in sandigen Lehmboden ausgelegt und mit Erde abgedeckt.

Ein Teil der Töpfe wurde sofort wie unter 1. beschrieben behandelt, die übrigen im Gewächshaus aufgestellt, bis die Pflanzen zwei bis drei echte Blätter entwickelt hatten und dann mit den erfindungsgemäßen Substanzen der Formel (I) oder deren Salze in unterschiedlichen Dosierungen, wie unter 2. beschrieben besprüht.

Vier bis fünf Wochen nach der Applikation und Standzeit im Gewächshaus wurde mittels optischer Bonitur festgestellt, daß die erfindungsgemäßen Verbindungen zweikeimblättrigen Kulturen, wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die Verbindungen der Formel (I) oder deren Salze weisen somit eine hohe Selektivität bei Anwendung zur Bekämpfung von unerwünschtem Pflanzenwuchs in landwirtschaftlichen Kulturen auf.

## Patentansprüche

1. Verbindungen der Formel (I) oder deren Salze, 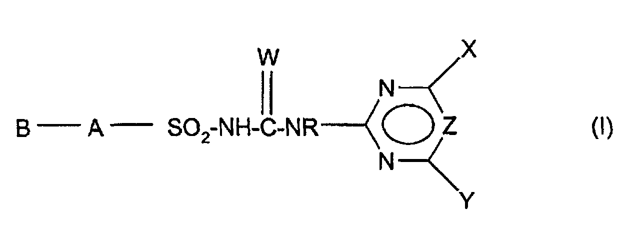 worin
A eine Brücke der Formeln A₁-A₉ 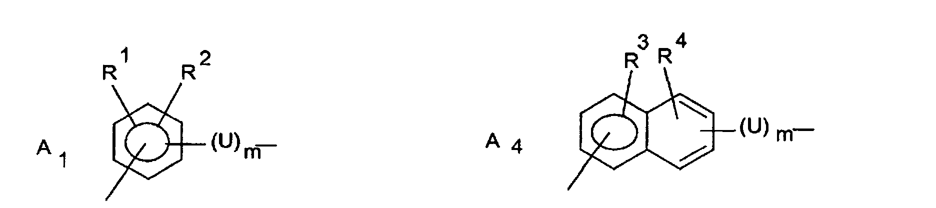 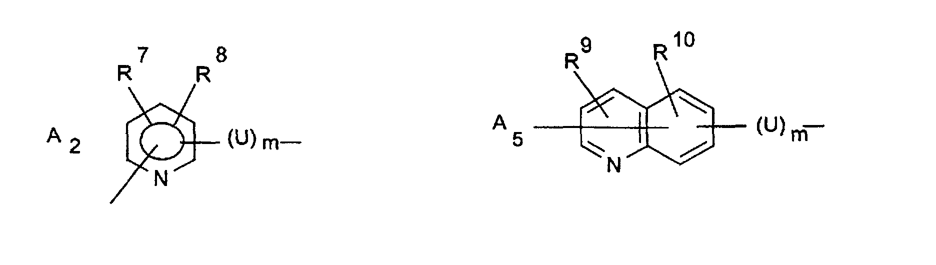 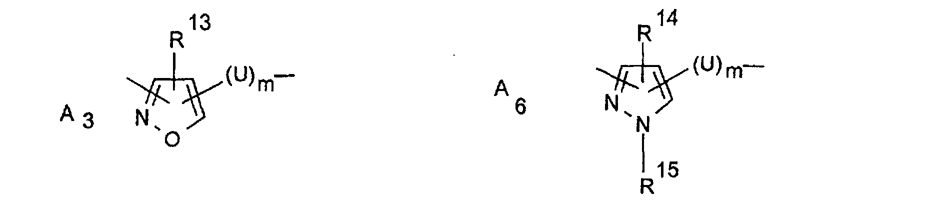 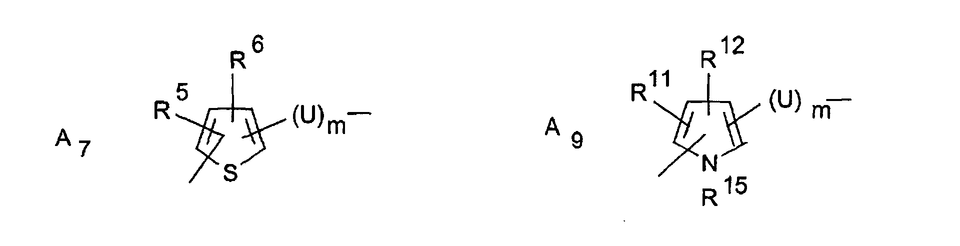 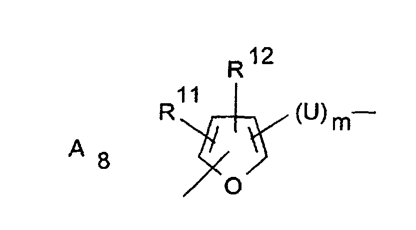
m 0 oder 1,
U CH₂, O oder NH oder (C₁-C₃-Alkyl)N,
R¹ H, CN, Halogen, Azid, NO₂, OH, C₁-C₈-Alkyl, C₂-C₈-Alkenyl, C₂-C₈-Alkinyl, C₁-C₈-Alkoxy, C₂-C₈-Alkenyloxy, C₂-C₈-Alkinyloxy, C₃-C₈-Cycloalkyl, C₃-C₈-Cycloalkyloxy, C₅-C₈-Cycloalkenyl, C₅-C₈-Cycloalkenyloxy,
wobei jeder der letztgenannten 10 Reste unsubstituiert oder durch einen oder mehrere Reste aus der Gruppe Halogen, C₁-C₄- Alkoxy, C₁-C₄-Haloalkoxy, C₁-C₄-Alkylthio, C₁-C₄-Haloalkylthio, OH, CN, NO₂, Oxo, C₁-C₄-Alkylsulfonyl, C₁-C₄-Haloalkylsulfonyl, (C₁-C₄-Alkoxy)-carbonyl, (C₁-C₄-Haloalkoxy)-carbonyl, NR³²R³³ und im Falle cyclischer Reste auch C₁-C₄-Alkyl und C₁-C₄-Haloalkyl substituiert ist, oder COR²⁴, CS-R²⁵, C(=NR²⁶)R²⁷, NR²⁸R²⁹, oder einen 3 bis 7-gliedrigen Heterocyclus mit bis zu 4 Heteroatomen aus der Gruppe N, O und S, der unsubstituiert ist oder durch einen oder mehrere Reste aus der Gruppe Halogen, C₁-C₄-Alkyl, OH, CN, NO₂, CHO, NH₂, Mono- und Di-(C₁-C₄-alkyl)-amino, C₁-C₄-Alkyl, C₁-C₄-Haloalkyl, C₁-C₄-Alkoxy, C₁-C₄-Haloalkoxy, C₁-C₄-Alkylthio, C₁-C₄-Haloalkylthio, C₁-C₄-Alkylcarbonyl, C₁-C₄-Alkoxycarbonyl und Oxo substituiert ist, oder Aryl, Heteroaryl, Aryl-C₁-C₃-alkyl oder Heteroaryl-C₁-C₃-alkyl, wobei jeder der letztgenannten 4 Reste im Aryl- bzw. Heteroarylteil unsubstituiert oder durch einen oder mehrere Reste aus der Gruppe Halogen, OH, CN, Nitro, C₁-C₄-Alkyl, C₁-C₄-Haloalkyl, C₁-C₄-Alkoxy, C₁-C₄-Haloalkoxy, C₁-C₄-Alkylthio, C₁-C₄-Haloalkylthio, C₁-C₄-Alkylsulfonyl, C₁-C₄-Haloalkylsulfonyl, C₁-C₄-Alkylcarbonyl, C₁-C₄-Haloalkylcarbonyl, (C₁-C₄-Alkoxy)carbonyl, (C₁-C₄-Haloalkoxy)-carbonyl, Mono- und Di(C₁-C₄-alkyl)amino, CHO, NH₂, CONH₂, Mono- und Di-(C₁-C₄-alkyl)-aminocarbonyl substituiert ist,
R² Wasserstoff, C₁-C₈-Alkyl, C₂-C₈-Alkenyl, C₂-C₈-Alkinyl, C₁-C₈-Alkoxy, C₂-C₈-Alkenyloxy, C₂-C₈-Alkinyloxy, C₃-C₈-Cycloalkyl, C₅-C₈-Cycloalkenyl, C₅-C₈-Cycloalkoxy, C₅-C₈-Cycloalkenyloxy,
wobei jeder der letztgenannten 10 Reste unsubstituiert oder durch einen oder mehrere Reste aus der Gruppe Halogen, C₁-C₄-Alkoxy und C₁-C₄-Haloalkoxy und im Falle cyclischer Reste auch C₁-C₄-Alkyl und C₁-C₄-Haloalkyl substituiert ist, oder Halogen, CN, NH₂, Mono- oder Di-(C₁-C₄-alkyl)-amino,
R³, R⁵, R⁷, R⁹, R¹¹, R¹³ und R¹⁴ jeweils einen Rest aus der Gruppe der für R¹ möglichen Reste,
R⁴, R⁶, R⁸, R¹⁰ und R¹² jeweils einen Rest aus der Gruppe der für R² möglichen Reste,
R¹⁵ Wasserstoff, C₁-C₈-Alkyl, C₃-C₈-Cycloalkyl, C₁-C₈-Haloalkyl, C₃-C₈-Halocycloalkyl, Aryl, das unsubstituiert oder durch einen oder mehrere Reste aus der Gruppe Halogen, C₁-C₄-Alkyl, C₁-C₄-Haloalkyl, C₁-C₄-Alkoxy und C₁-C₄-Haloalkoxy substituiert ist,
B eine Gruppe, die ein iminohaltiges 3-atomiges Fragment aus der Gruppe der Fragmente der Formeln N = C-N, N = C-S, N = C-O und N =C-C enthält und die über ein Stickstoffatom des Fragments an den Rest A gebunden ist,
R H oder einen aliphatischen Rest,
W ein Sauerstoff- oder Schwefelatom,
X,Y unabhängig voneinander Wasserstoff, Halogen, C₁-C₆-Alkyl, C₁-C₆-Alkoxy, C₁-C₆-Alkylthio, C₃-C₆-Cycloalkyl, C₃-C₆-Cycloalkoxy, C₃-C₈-Cycloalkylthio, C₂-C₆-Alkenyl, C₅-C₆-Cycloalkenyl, C₂-C₆-Alkinyl, C₃-C₆-Alkenyloxy, C₅-C₆-Cycloalkenyloxy, C₃-C₆-Alkinyloxy, wobei jeder der elf letztgenannten Reste unsubstituiert oder durch einen oder mehrere Reste aus der Gruppe Halogen, C₁-C₄-Alkoxy, C₁-C₄-Alkylthio, C₁-C₄-Haloalkoxy und im Falle cyclischer Reste auch C₁-C₄-Alkyl und C₁-C₄-Haloalkyl substituiert ist, oder Mono- oder Di-(C₁-C₄-alkyl)amino und
Z CH oder N bedeuten.

2. Verbindungen der Formel (I) oder deren Salze nach Anspruch 1, **dadurch gekennzeichnet, daß**
B eine iminohaltige Gruppe der Formel, 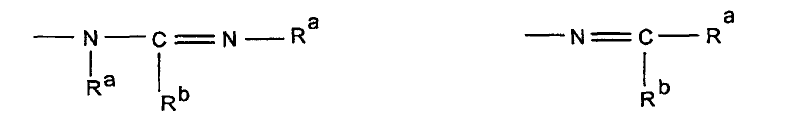 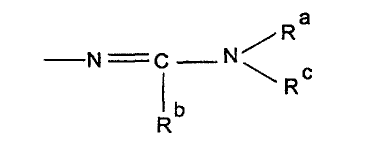 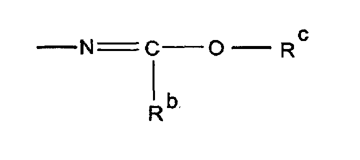
oder 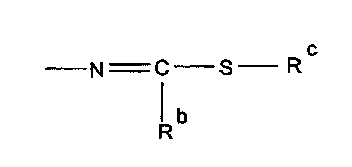 wobei die R^{a} unabhängig voneinander Wasserstoff, NH₂, OH oder einen aliphatischen, aromatischen, heterocyclischen oder anderen organischen Rest mit insgesamt 1 bis 12 C-Atomen, der auch über ein Heteroatom gebunden sein kann, bedeuten, die R^{b} unabhängig voneinander jeweils einen der für R^{a} möglichen Reste, ausgenommen OH, bedeuten, die R^{c} unabhängig voneinander H oder einen aliphatischen, aromatischen, heterocyclischen oder anderen organischen Rest mit insgesamt 1 bis 20 C-Atomen bedeuten und wobei die Reste R^{a} bis R^{c} in einer Grupppe B paarweise eine Brücke bilden können und die organische Gruppe B insgesamt 1 bis 30 C-Atome besitzt,
R H oder einen Kohlenwasserstoffrest mit 1 bis 8 C-Atomen, welcher unsubstituiert oder durch einen oder mehrere Reste aus der Gruppe Halogen, C₁-C₄-Alkoxy und C₁-C₄-Alkylthio substituiert ist,
W O oder S,
einer der Reste X und Y Halogen, C₁-C₆-Alkyl, C₁-C₆-Alkoxy, C₁-C₆-Alkylthio, wobei die letztgenannten 3 Reste unsubstituiert oder durch einen oder mehrere Reste aus der Gruppe Halogen, C₁-C₄-Alkoxy und C₁-C₄-Alkylthio substituiert sind, oder C₂-C₄-Alkenyl, C₂-C₄-Alkinyl, C₂-C₄-Alkenyloxy, C₂-C₄-Alkinyloxy, wobei jeder der letztgenannten 4 Reste unsubstituiert oder durch einen oder mehrere Halogenatome substituiert ist, oder C₃-C₆-Cycloalkyl oder Mono- oder Di-(C₁-C₂-alkyl)amino und
der andere der Reste X und Y Halogen, C₁-C₄-Alkyl, C₁-C₄-Alkoxy oder C₁-C₄-Alkylthio, wobei jeder der letztgenannten 3 Reste unsubstituiert oder durch einen oder mehrere Reste aus der Gruppe Halogen, C₁-C₄-Alkoxy und C₁-C₄-Alkylthio substituiert ist, oder Mono- oder Di-(C₁-C₂-Alkyl)amino und
Z CH oder N bedeuten.

3. Verbindungen der Formel (I) oder deren Salze nach Anspruch 1 oder 2,
**dadurch gekennzeichnet, daß**
B eine Gruppe der Formel B1 bis B5, 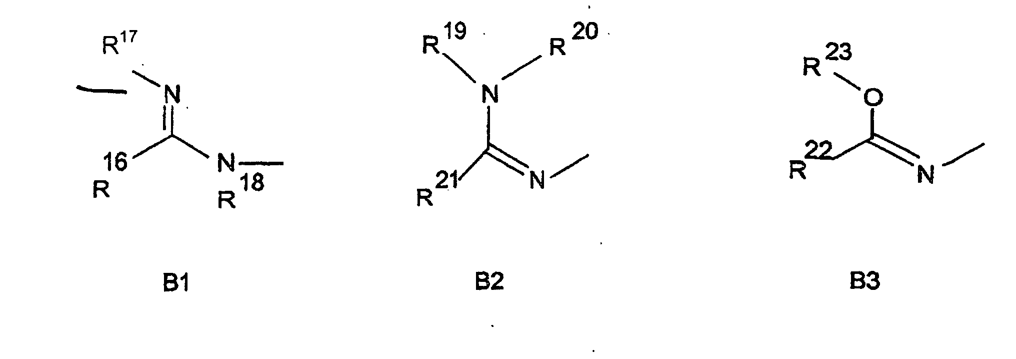 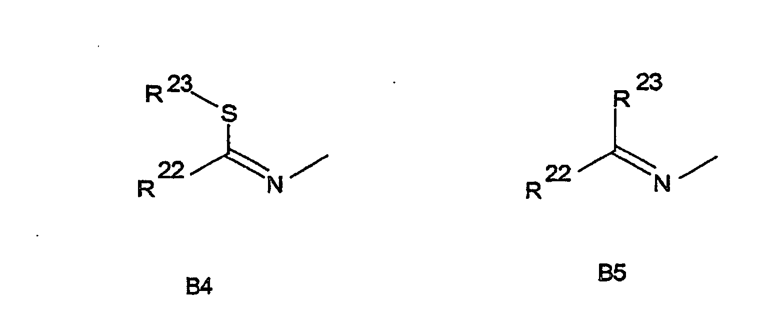
R Wasserstoff, C₁-C₅-Alkyl, C₁-C₅-Haloalkyl, C₂-C₅-Alkenyl, C₂-C₅-Haloalkenyl, C₂-C₅-Alkinyl oder C₂-C₅-Haloalkinyl,
R¹⁶ NH₂, Wasserstoff, Aryl oder Heteroaryl, insbesondere Phenyl, wobei jeder der letztgenannten beiden Reste unsubstitutiert oder substituiert ist oder C₁-C₈-Alkyl, C₂-C₈-Alkenyl, C₂-C₈-Alkinyl. C₁-C₈-Alkoxy, C₂-C₈-Alkenyloxy, C₂-C₈-Alkinyloxy, C₁-C₈-Alkylthio, C₂-C₈-Alkenylthio, C₂-C₈-Alkinylthio, C₃-C₈-Cycloalkyl, C₅-C₈-Cycloalkenyl, C₃-C₆-Cycloalkoxy, C₅-C₈-Cycloalkenyloxy, C₃-C₈-Cycloalkylthio, C₅-C₈-Cycloalkenylthio, Mono- oder Di-(C₁-C₆)alkyl-amino, wobei jeder der letztgenannten 17 Reste unsubstituiert oder substituiert ist, oder Benzyl, CN, (C₁-C₆-Alkoxy)carbonyl, (C₁-C₆-Alkyl)-carbonyl, Benzyloxycarbonyl oder Phenyl-carbonyl,
R¹⁷ OH, NH₂, Wasserstoff, C₁-C₈-Alkyl, C₂-C₈-Alkenyl, C₂-C₈-Alkinyl, C₁-C₈-Alkoxy, C₂-C₈-Alkenyloxy, C₂-C₈-Alkinyloxy, (C₁-C₈-Alkyl)-carbonyl, (C₁-C₈-Alkoxy)-carbonyl, Benzoyl, Benzyloxycarbonyl, (C₁-C₈)-Alkylsulfonyl, Arylsulfonyl, C₃-C₈-Cycloalkyl, C₅-C₈-Cycloalkenyl, C₃-C₈-Cycloalkoxy, C₅-C₈-Cycloalkenyloxy, C₃-C₈-Cycloalkylcarbonyl, C₃-C₈-Cycloalkoxycarbonyl, C₃-C₈-Cyclo-alkylsulfonyl, Mono- oder Di-(C₁-C₈-alkyl)-amino, wobei jeder der letztgenannten 21 Reste unsubstituiert oder durch einen oder mehrere Reste aus der Gruppe Halogen, C₁-C₄-Alkoxy, C₁-C₄-Haloalkoxy, CN, NO₂, OH, NH₂, Mono- und Di-(C₁-C₃-alkyl)-amino, C₁-C₄-Alkylthio und C₁-C₄-Haloalkylthio und im Falle cyclischer Reste auch C₁-C₄-Alkyl und C₁-C₄-Haloalkyl substituiert ist, oder Aryl oder Heteroaryl, wobei jeder der 2 letztgenannten Reste unsubstituiert oder durch einen oder mehrere Reste aus der Gruppe C₁-C₄-Alkyl, C₁-C₄-Haloalkyl, C₁-C₄-Alkoxy, C₁-C₄-Haloalkoxy, CN und NO₂ substituiert ist, 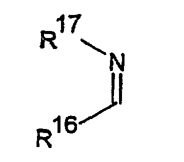 oder der Teil der Formel
aus der Gruppe der oben genannten Formel B1 (siehe Definition von B) gemeinsam einen heterocyclischen Ring mit einer Iminofunktion,
wobei der heterocyclische Ring unsubstituiert oder substituiert ist,
R¹⁸ Wasserstoff, C₁-C₈-Alkyl, C₂-C₈-Alkenyl, C₂-C₈-Alkinyl, C₁-C₈-Alkylsulfonyl, (C₁-C₈-Alkyl)-carbonyl, (C₁-C₈-Alkoxy)-carbonyl oder C₁-C₅-Alkoxy, C₃-C₈-Cycloalkyl, C₅-C₈-Cycloalkenyl, C₃-C₈-Cycloalkylsulfonyl, C₃-C₈-Cycloalkylcarbonyl, C₃-C₈-Cycloalkoxycarbonyl, C₃-C₈-Cycloalkoxy, wobei jeder der letztgenannten 13 Reste unsubstituiert oder durch einen oder mehrere Reste aus der Gruppe Halogen, C₁-C₃-Alkoxy, C₁-C₃-Haloalkoxy und im Falle cyclischer Reste auch C₁-C₄-Alkyl und C₁-C₄-Haloalkyl substituiert ist, oder CHO, NH₂, OH, Mono- oder Di-(C₁-C₄-alkyl)-amino,
R¹⁹ einen Rest aus der Gruppe der für R¹⁷ möglichen Reste und
R²⁰ Wasserstoff, C₁-C₈-Alkyl, C₃-C₈-Cycloalkyl, C₂-C₈-Alkenyl, C₃-C₈-Cycloalkenyl oder C₂-C₆-Alkinyl oder
NR¹⁹R²⁰ einen heterocyclischen Ring, wobei der heterocyclische Rest unsubstituiert oder durch einen oder mehrere Reste aus der Gruppe Halogen, C₁-C₄-Alkyl, C₁-C₄-Haloalkyl, C₁-C₄-Alkoxy, C₁-C₄-Haloalkoxy, OH, NH₂, NO₂, CN, Mono- und Di-(C₁-C₅-alkyl)-amino substituiert ist,
R²¹ einen Rest aus der Gruppe der für R¹⁶ möglichen Reste,
R²² Wasserstoff, C₁-C₈-Alkyl, C₂-C₈-Alkenyl, C₂-C₈-Alkinyl, C₁-C₈-Alkoxy, C₁-C₈-Alkylthio, C₃-C₈-Cycloalkoxy, C₃-C₈-Cycloalkylthio, C₃-C₈-Cycloalkyl, C₅-C₈-Cycloalkenyl, Mono- oder Di-(C₁-C₈-alkyl)-amino, wobei jeder der letztgenannten 11 Reste unsubstituiert oder durch einen oder mehrere Reste aus der Gruppe CN, Halogen, C₁-C₄-Alkoxy, C₁-C₄-Haloalkoxy und im Falle cyclischer Reste auch C₁-C₄-Alkyl und C₁-C₄-Haloalkyl substituiert ist, oder einen aromatischen oder heteroaromatischen Monocyclus oder Polycyclus, wobei jeder dieser Reste unsubstituiert oder substituiert ist,
R²³ C₁-C₈-Alkyl, C₂-C₈-Alkenyl, C₂-C₈-Alkinyl, C₃-C₈-Cycloalkyl, C₅-C₈-Cycloalkenyl, Heterocyclyl, Aryl oder Benzyl, wobei jeder der 8 letztgenannten Reste unsubstituiert oder durch einen oder mehrere Reste aus der Gruppe Halogen, C₁-C₄-Alkoxy, C₁-C₄-Haloalkoxy, CN und NO₂ und im Falle cyclischer Reste auch C₁-C₄-Alkyl und C₁-C₄-Haloalkyl substituiert ist,
R²⁴ Wasserstoff, OH, NH₂, Aryl oder einen Heterocyclus, wobei jeder der letztgenannten 2 Reste unsubstituiert oder substituiert ist, oder C₁-C₈-Alkyl, C₂-C₈-Alkenyl, C₂-C₈-Alkinyl, C₁-C₈-Alkoxy, C₂-C₈-Alkenyloxy, C₂-C₈-Alkinyloxy, C₁-C₈-Alkylthio, C₂-C₈-Alkenylthio, C₂-C₈-Alkinylthio, Benzyl, Aryloxy, Benzyloxy, C₃-C₈-Cycloalkyl, C₅-C₈-Cycloalkenyl, C₃-C₈-Cycloalkoxy, C₅-C₈-Cycloalkenyloxy, C₅-C₈-Cycloalkylthio oder C₅-C₈-Cycloalkenylthio, Mono- oder Di-(C₁-C₈-alkyl)amino, wobei jeder der letztgenannten 20 Reste unsubstituiert oder durch einen oder mehrere Reste aus der Gruppe Halogen, C₁-C₄-Alkoxy, C₁-C₄-Haloalkoxy, OH, NH₂, Mono- und Di(C₁-C₄-alkyl)amino, CN und NO₂ und im Falle cyclischer Reste auch C₁-C₄-Alkyl und C₁-C₄-Haloalkyl substituiert ist,
R²⁵ einen Rest aus der Gruppe der für R²⁴ möglichen Reste,
R²⁹ Wasserstoff, OH, NH₂, CN, C₁-C₈-Alkoxy, C₂-C₈-Alkenyloxy, C₂-C₈-Alkinyloxy, C₁-C₈-Alkyl, C₂-C₈-Alkenyl, C₂-C₈-Alkinyl, C₁-C₈-Alkylsulfonyl, C₂-C₈-Alkenylsulfonyl, C₂-C₈-Alkinylsulfonyl, C₃-C₈-Cycloalkoxy, C₅-C₈-Cycloalkenyloxy, C₃-C₈-Cycloalkyl, C₅-C₈-Cycloalkenyl, C₃-C₈-Cycloalkylsulfonyl, C₅-C₈-Cycloalkenyl-sulfonyl, Arylsulfonyl oder Aryl oder einen Heterocyclus oder Benzyl, (C₁-C₈-Alkoxy)-carbonyl, (C₁-C₈-Alkyl)-carbonyl, Phenoxycarbonyl, Benzylcarbonyl oder Heterocyclylcarbonyl, wobei jeder der letztgenannten 24 Reste unsubstituiert oder substitutiert ist,
R²⁷ einen Rest aus der Gruppe der für R²⁴ möglichen Reste, ausgenommen OH,
R²⁸ H, OH, CHO, C₁-C₈-Alkyl, C₂-C₈-Alkenyl, C₂-C₈-Alkinyl, C₁-C₈-Alkoxy, C₂-C₈-Alkenyloxy, C₂-C₈-Alkinyloxy, C₁-C₈Alkylsulfonyl, C₂-C₈-Alkenylsulfonyl, C₂-C₈-Alkinylsulfonyl, (C₁-C₈-Alkyl)-carbonyl, (C₂-C₈-Alkenyl)-carbonyl, (C₂-C₈-Alkinyl)-carbonyl, (C₁-C₈-Alkoxy)-carbonyl, (C₂-C₈-Alkenyloxy)-carbonyl, (C₂-C₈-Alkinyloxy)-carbonyl, Mono- oder Di-(C₁-C₈-alkyl)-aminocarbonyl, C₃-C₈-Cycloalkyl, C₅-C₈-Cycloalkenyl, C₃-C₈-Cycloalkoxy, C₅-C₈-Cycloalkenyloxy, C₃-C₉-Cycloalkylsulfonyl, C₅-C₈-Cycloalkenyl-sulfonyl, C₃-C₈-Cycloalkoxycarbonyl, C₅-C₈-Cycloalkenyloxy-carbonyl, Benzoyl, Benzyl, Benzylcarbonyl, Benzyloxycarbonyl, Aryl oder einen Heterocyclus, wobei jeder der letztgenannten 31 Reste unsubstituiert oder substituiert ist, oder SO₂NH₂ oder CONH₂,
R²⁹ einen Rest aus der Gruppe der für R²⁸ möglichen Reste, ausgenommen OH,
R³⁰ einen Rest aus der Gruppe der für R¹⁹ möglichen Reste,
R³¹ einen Reste aus der Gruppe der für R²⁰ möglichen Reste,
oder NR³⁰R³¹ gemeinsam einen heterocyclischen Ring, wie er entsprechend für NR¹⁹R²⁰ definiert ist,
R³² Wasserstoff, C₁-C₄-Alkyl, C₁-C₄-Haloalkyl, CHO, C₁-C₄-Alkylsulfonyl, C₂-C₄-Alkenyl, C₂-C₄-Alkinyl, C₂-C₄-Haloalkenyl, C₁-C₄-Haloalkyl, C₂-C₄-Haloalkinyl, C₁-C₄-Haloalkylsulfonyl, (C₁-C₄-Alkyl)-carbonyl, (C₁-C₄-Alkoxy)-carbonyl, (C₁-C₄-Haloalkyl)-carbonyl oder (C₁-C₄-Haloalkoxy)-carbonyl,
R³³ einen Rest aus der Gruppe der für R³² möglichen Reste oder
NR³²R³³ gemeinsam einen heterocyclischen Ring, wie er entsprechend für NR¹⁹R²⁰ definiert ist,
bedeuten.

4. Verbindungen der Formel (I) oder deren Salze, nach einem der Ansprüche 1 bis 3, **dadurch gekennzeichnet, daß**
R Wasserstoff, C₂-C₃-Alkyl, C₁-C₃-Alkoxy,
R¹ H, CN, NO₂, Halogen, C₁-C₆-Alkoxy, C₁-C₆-Alkyl, C₁-C₆-Haloalkoxy, C₁-C₆-Haloalkyl, (C₁-C₆-Alkoxy)-carbonyl, (C₃-C₆-Cycloalkoxy)-carbonyl oder (C₃-C₆Cycloalkyl)-methoxycarbonyl, (C₃-C₆-Cycloalkoxy)-carbonyl, (C₃-C₆-Cycloalkyl)-methoxycarbonyl, C₁-C₆-Alkylthio, C₁-C₆-Haloalkylthio, C₁-C₆-Alkylsulfonyl, C₁-C₆-Haloalkylsulfonyl, SO₂NH₂, CONH₂, Mono- oder Di-(C₁-C₄-alkyl)-aminocarbonyl oder -aminosulfonyl oder einen Rest der Formel 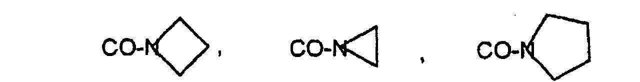 oder C₁-C₆-Alkylcarbonyl, C₁-C₆-Haloalkylcarbonyl, CHO, Cyclopropylcarbonyl, Cyclobutylcarbonyl oder NR²⁸R²⁹;
R² Wasserstoff, C₁-C₆-Alkyl, C₁-C₆-Haloalkyl, C₁-C₆-Alkoxy, C₁-C₆-Haloalkoxy, Halogen oder CN;
R³, R⁵, R⁷, R⁹, R¹¹, R¹³ und R¹⁴ jeweils einen Rest aus der Gruppe der für R¹ möglichen Reste,
R⁴, R⁶, R⁸, R¹⁰, R¹² jeweils einen Rest aus der Gruppe der für R² möglichen Reste,
R¹⁵ H, C₁-C₃-Alkyl oder Phenyl;
R¹⁶ H, NH₂, Mono- oder Di-(C₁-C₆-alkyl)amino, C₁-C₆-Alkyl, Phenyl oder C₁-C₆-Alkoxy, wobei die letztgenannten 5 Reste unsubstituiert oder durch einen oder mehrere Reste aus der Gruppe Halogen, C₁-C₃-Alkoxy, C₁-C₃-Haloalkoxyl CN, NO₂ und im Falle von Phenyl auch C₁-C₃-Alkyl und C₁-C₃-Haloalkyl, substituiert sind;
R¹⁷ Mono- oder Di-(C₁-C₆-alkyl)amino, C₁-C₆-Alkyl, C₁-C₆-Alkoxy, Phenyl, Benzyl, wobei die 6 letztgenannten Reste unsubstituiert oder durch einen oder mehrere Reste aus der Gruppe Halogen, C₁-C₃-Alkoxy, C₁-C₃-Haloalkoxy und im Falle von Phenyl auch C₁-C₃-Alkyl und C₁-C₃-Haloalkyl substituiert sind, oder OH, NH₂ oder H; oder der Teil der Formel R¹⁷-N=C-R¹⁶ aus der Gruppe der Formel B1 in der Definition für B gemeinsam einen Heterocyclus der Formel D1 oder D2, 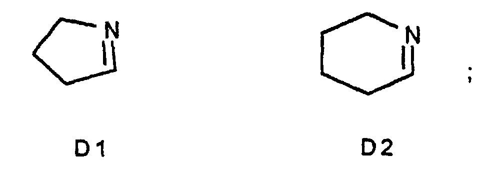
R¹⁸ H, C₁-C₆-Alkyl oder C₁-C₆-Haloalkyl,
R¹⁹ H, C₁-C₆-Alkyl, C₁-C₆-Haloalkyl, C₁-C₆-Haloalkoxy oder Phenyl,
R²⁰ H, C₁-C₆-Alkyl, C₁-C₆-Haloalkyl oder Phenyl oder
NR¹⁹R²⁰ gemeinsam einen Heterocyclus der Formel 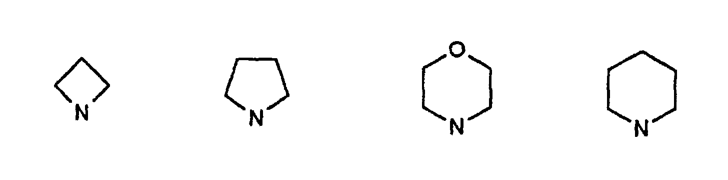 oder 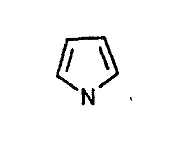
R²¹ einen Rest aus der Gruppe der für R¹⁶ möglichen Reste;
R²² H, C₁-C₆-Alkyl, C₁-C₆-Haloalkyl oder Phenyl;
R²³ C₁-C₆-Alkyl oder C₁-C₆-Haloalkyl;
R²⁸ H, C₁-C₆-Alkyl, OH, C₁-C₆-Haloalkyl oder C₁-C₆-Alkoxy,
R²⁹ H, C₁-C₆-Alkyl, C₁-C₆-Haloalkyl, CHO, C₁-C₃-Alkylsulfonyl, C₁-C₃-Alkoxycarbonyl oder C₁-C₃-Alkylcarbonyl;
X, Y unabhängig voneinander Cl, F, CF₃, CH₃, CH₂CH₃, OCH_{3,} OC₂H₅, N(CH₃)₂, NHCH₃ oder OCH₂CF₃ bedeuten.

5. Verbindungen der Formel (I) oder deren Salze nach einem der Ansprüche 1 bis 4, **dadurch gekennzeichnet, daß**
A eine Brücke der Formel 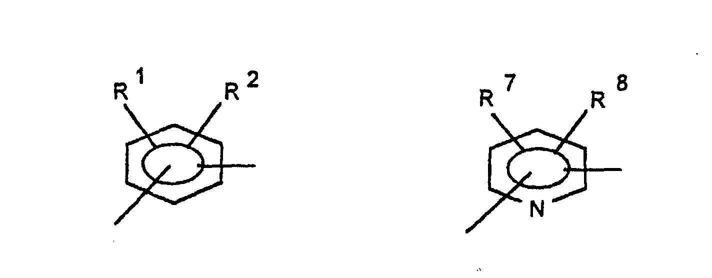 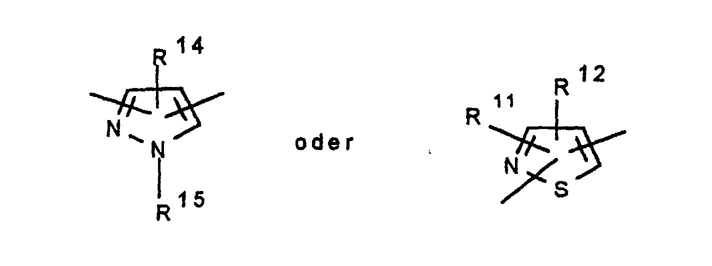 und
B eine Gruppe der Formeln B1, B2 und B3
-NR¹⁸-C(R¹⁶)=N-R¹⁷ (B1)
-N=C(R²¹)-NR¹⁹R²⁰ (B2)
-N=C(R²²)-O-R²³ (B3)
bedeuten,
wobei in den Formeln zu A und B die Reste R¹, R², R⁷, R⁸, R¹¹, R¹², R¹⁴, R¹⁵ und R¹⁶ bis R²³ wie in Formel (I) definiert sind.

6. Verfahren zur Herstellung von Verbindungen der Formel (I) oder deren Salzen, wie sie nach einem der Ansprüche 1 bis 5 definiert sind, **dadurch gekennzeichnet, daß** man
a) eine Verbindung der Formel (II)
B-A-SO₂NH₂ (II)
mit einem heterocyclischen Carbamat der Formel (III), 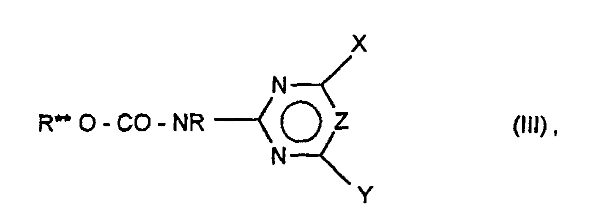 worin R** unsubstituiertes oder substituiertes Phenyl oder C₁-C₄-Alkyl bedeutet, umsetzt oder
b) ein Sulfonylisocyanat der Formel (IV)
B-A-SO₂-N=C=O (IV)
mit einem heterocyclischen Amin der Formel (V) 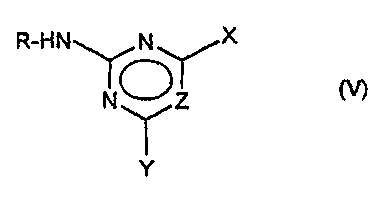 umsetzt oder
c) ein Sulfochlorid der Formel (VI)
B-A-SO₂Cl (VI)
mit einem heterocyclischen Amin der genannten Formel (V) in Gegenwart eines Cyanats umsetzt oder
d) ein Sulfochlorid der genannten Formel (II) mit einem (Thio-)-Isocyanat der Formel (VII) 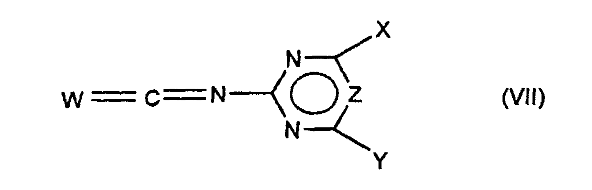 in Gegenwart einer geeigneten Base umsetzt,
e) im Falle daß B eine Gruppe ist, die ein Fragment der Formel -N=C-NR^{a}- enthält, worin R^{a} NH₂, OH oder einen organischen Rest mit 1 bis 12 C-Atomen bedeutet, einen aminosubstituierten Sulfonylharnstoff der Formel (VIII), 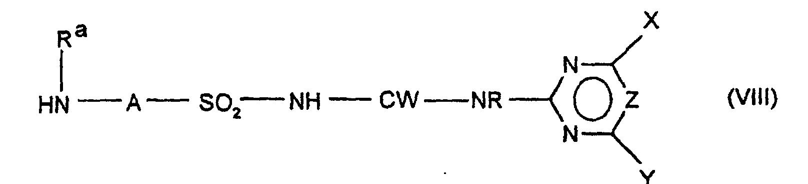 worin R^{a} wie oben definiert ist,
mit geeigneten Reagenzien, die ein Fragment der Formel (IXa) enthalten, 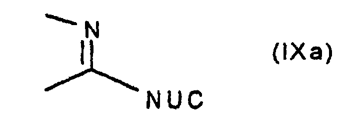 worin NUC eine nucleofuge Gruppe ist, umsetzt,
f) im Falle, daß B eine Gruppe ist, die ein Fragment der Formel 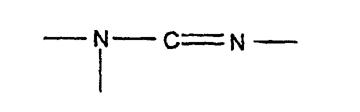 enthält,
einen aminosubstituierten Sulfonylharnstoff der genannten Formel (VIII) (siehe e), worin R^{a} = Wasserstoff bedeutet, mit geeigneten Reagenzien, die ein Fragment der Formel (IXb) enthalten, 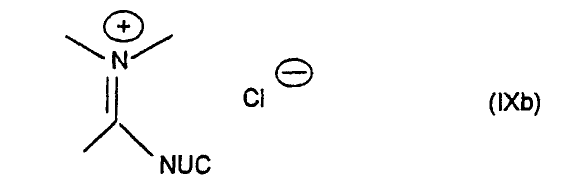 worin NUC eine nucleofuge Gruppe ist, umsetzt,
wobei in den Formeln (II) bis (IX) die Reste A, B, R, W, X, Y und Z wie in Formel (I) definiert sind, und wobei in den Varianten a) bis c) zunächst Verbindungen der Formel (I) erhalten werden, in denen W ein Sauerstoffatom bedeutet.

7. Herbizides oder pflanzenwachstumsregulierendes Mittel, **dadurch gekennzeichnet, daß** es mindestens eine Verbindung der Formel (I) oder deren Salz nach einem der Ansprüche 1 bis 5 und im Pflanzenschutz übliche Formulierungshilfsmittel enthält.

8. Verfahren zur Bekämpfung von Schadpflanzen oder zur Wachstumsregulierung von Pflanzen, **dadurch gekennzeichnet, daß** man eine wirksame Menge von mindestens einer Verbindung der Formel (I) oder deren Salz nach einem der Ansprüche 1 bis 5 auf die Schadpflanzen bzw. Pflanzen, deren Pflanzensamen oder die Fläche, auf der sie wachsen, appliziert.

9. Verwendung der Verbindungen der Formel (I) oder deren Salze nach einem der Ansprüche 1 bis 5 als Herbizide oder Pflanzenwachstumsregulatoren.

10. Verbindungen der Formel (XII),
B-A-SO₂-R^{Z} (XII)
worin
R^{Z} = NH₂, Mono- oder Di-(C₁-C₄-alkyl)amino, Halogen oder -N=CO bedeutet und B und A wie in Formel (I) nach einem der Ansprüche 1 bis 5 definiert sind,
ausgenommen Verbindungen der Formel (XII), worin
a) R^{z} = Amino,
A = Phenyl, das zwei allgemeine Reste Y' und Z' trägt, wobei Y' = H, ein Halogenatom odsr eine Alkylgruppe ist, Z' = H, ein Halogenatem oder eine Alkyl-, Alkoxy-, Trifluormethyl-, Sulfamyl- oder Nitrogruppe ist, und
B = eine Gruppe der Formel -N=CR^{b}-NH₂ bedeutet, worin R^{b} = einen gesattigten oder nicht konjugiert ungesättigten substitulerten oder unsubstituierten allphatiachen oder cycloaliphatischen Kohienwasserstoffrest, der auch durch ein oder mehrere Heterostome unterbrochen sein kann, oder einen substituierten oder unsubstituierten aromatischen Rest, wobei der jeweilige Rest auch über ein Sauerstoffatom oder eine NH-Gruppe gebunden sein kann,
b) R^{z} = Dimethylamino oder Diethylamino,
A = Phenyl, das in Orthostellung zur Gruppe SO₂-R^{z} gegebenenfalls durch Chlor, Brom, Methyl, Methoxy oder Trifluormethyl substituiert sein kann, und
B = eine Gruppe der Formel -N=CH-NR'-Ar, worin Ar für Phenyl oder 4-Dimethylaminosulfonylphenyl oder 4-Diethylaminosulfonylphenyl, wobei jeder der letzten belden Reste im Phenylteil gegebenentalls durch 3-Chlor, 3-Brom, 3-Methyi, 3-Methoxy oder 3-Trifluormethyl substituiert sein kann, steht und R Wasserstoff oder eine N-(C₁-C₄)Alkylamino- oder N,N-Di-(C₁-C₄)alkylaminocarbonyl ist,
c) R^{z} = SO₂Cl,
A = Phenyl und
B = eine Gruppe der Formel -NH-C(=NH)-NH₂, und
d) R^{z} = SO₂NH₂,
A = Phenyl, das gegebenenfalls noch substituiert sein kann,
B = ein Rest der Formel -NR'-C(=NH)-R", worin R' Wasserstoff oder (C₁-C₁₈)Alkyl ist und R" [(C₁-C₄)Alkoxy-carbonyl]-methyl ist, bedeutet.

## Claims

1. A compound of the formula (I) or a salt thereof, 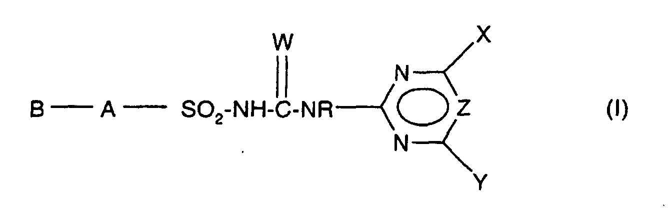 in which
A is a bridge of the formulae A₁-A₉ 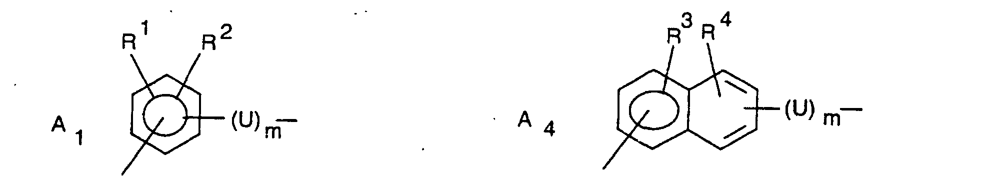 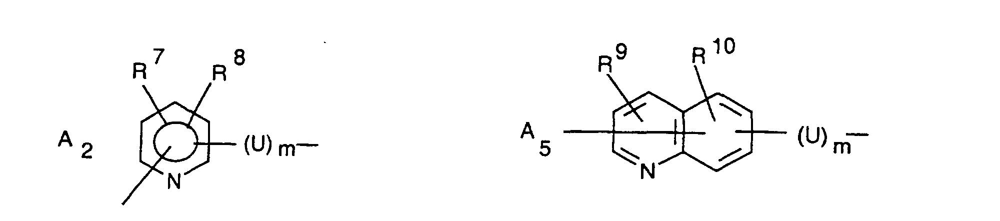 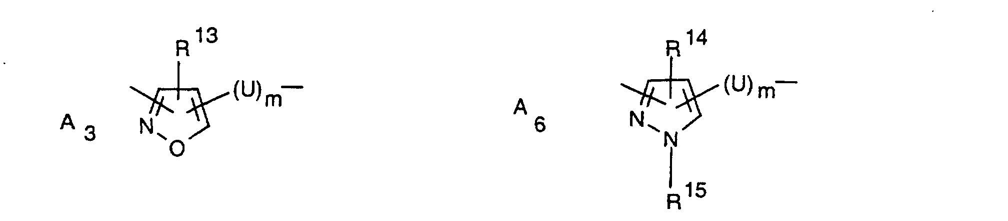 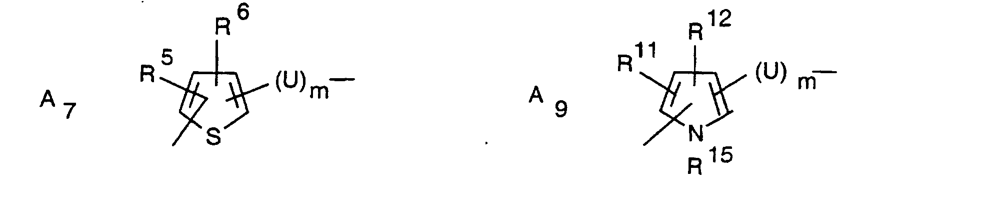 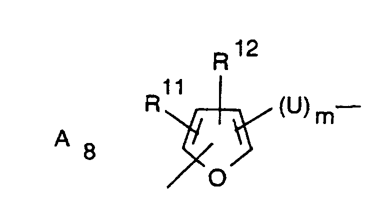
m is 0 or 1,
U is CH₂, O or NH or (C₁-C₃-alkyl)N,
R¹ is H, CN, halogen, azide, NO₂, OH, C₁-C₈-alkyl, C₂-C₈-alkenyl, C₂-C₈-alkynyl, C₁-C₈-alkoxy, C₂-C₈-alkenyloxy, C₂-C₈-alkynyloxy, C₃-C₈-cycloalkyl, C₃-C₈-cycloalkyloxy, C₅-C₈-cycloalkenyl, C₅-C₈-cycloalkenyloxy,
each of the last 10 radicals being unsubstituted or substituted by one or more radicals from the group consisting of halogen, C₁-C₄-alkoxy, C₁-C₄-haloalkoxy, C₁-C₄-alkylthio, C₁-C₄-haloalkylthio, OH, CN, NO₂, oxo, C₁-C₄-alkylsulfonyl, C₁-C₄-haloalkylsulfonyl, (C₁-C₄-alkoxy)carbonyl, (C₁-C₄-haloalkoxy)-carbonyl, NR³²R³³ and, in the case of cyclic radicals, also C₁-C₄-alkyl and C₁-C₄-haloalkyl, or is COR²⁴, CS-R²⁵, C(=NR²⁶)R²⁷, NR²⁸R²⁹ or a 3-to 7-membered heterocycle having up to 4 hetero atoms from the group consisting of N, O and S which is unsubstituted or substituted by one or more radicals from the group consisting of halogen, C₁-C₄-alkyl, OH, CN, NO₂, CHO, NH₂, mono- and di-(C₁-C₄-alkyl)amino, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₁-C₄-alkylcarbonyl, C₁-C₄-alkoxycarbonyl and oxo, or is aryl, hetaryl, aryl-C₁-C₃-alkyl or hetaryl-C₁-C₃-alkyl, the aryl or hetaryl moiety of each of the last 4 radicals being unsubstituted or substituted by one or more radicals from the group consisting of halogen,OH, CN, nitro, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₁-C₄-alkylsulfonyl, C₁-C₄-haloalkylsulfonyl, C₁-C₄-alkylcarbonyl, C₁-C₄-haloalkylcarbonyl, (C₁-C₄-alkoxy)carbonyl, (C₁-C₄-haloalkoxy)carbonyl, mono- and di(C₁-C₄-alkyl)amino, CHO, NH₂, CONH₂, mono- and di-(C₁-C₄-alkyl)-aminocarbonyl,
R² is hydrogen, C₁-C₈-alkyl, C₂-C₈-alkenyl, C₂-C₈-alkynyl, C₁-C₈-alkoxy, C₂-C₈-alkenyloxy, C₂-C₈-alkynyloxy, C₃-C₈-cycloalkyl, C₅-C₈-cycloalkenyl, C₅-C₈-cycloalkoxy, C₅-C₈-cycloalkenyloxy, each of the last 10 radicals being unsubstituted or substituted by one or more radicals from the group consisting of halogen, C₁-C₄-alkoxy and C₁-C₄-haloalkoxy and, in the case of cyclic radicals, also C₁-C₄-alkyl and C₁-C₄-haloalkyl, or is halogen, CN, NH₂, mono- or di-(C₁-C₄-alkyl)amino,
R³, R⁵, R⁷, R⁹, R¹¹, R¹³ and R¹⁴ are each a radical from the group of the radicals possible for R¹,
R⁴, R⁶, R⁸, R¹⁰ and R¹² are each a radical from the group of the radicals possible for R²,
R¹⁵ is hydrogen, C₁-C₈-alkyl, C₃-C₈-cycloalkyl, C₁-C₈-haloalkyl, C₃-C₈-halocycloalkyl, or aryl which is unsubstituted or substituted by one or more radicals from the group consisting of halogen, C₁-C₄-alkyl, C₁-C₄-haloalkyl, C₁-C₄-alkoxy and C₁-C₄-haloalkoxy,
B is a group which contains an imino-containing 3 atom fragment from the group consisting of the fragments of the formulae N=C-N, N=C-S, N=C-O and N=C-C and which is attached to the radical A via a nitrogen atom of the fragment,
R is H or an aliphatic radical,
W is an oxygen or sulfur atom,
X and Y are each independently of the other hydrogen, halogen, C₁-C₆-alkyl, C₁-C₆-alkoxy, C₁-C₆-alkylthio, C₃-C₆-cycloalkyl, C₃-C₆-cycloalkoxy, C₃-C₆-cycloalkylthio, C₂-C₆-alkenyl, C₅-C₆-cycloalkenyl, C₂-C₆-alkynyl, C₃-C₆-alkenyloxy, C₅-C₆-cycloalkenyloxy or C₃-C₆-alkynyloxy, each of the last eleven radicals being unsubstituted or substituted by one or more radicals from the group consisting of halogen, C₁-C₄-alkoxy, C₁-C₄-alkylthio, C₁-C₄-haloalkoxy and, in the case of cyclic radicals, also C₁-C₄-alkyl and C₁-C₄-haloalkyl, or are mono- or di-(C₁-C₄-alkyl)amino and
Z is CH or N.

2. A compound of the formula (I) or a salt thereof as claimed in claim 1, wherein
B is an imino-containing group of the formula 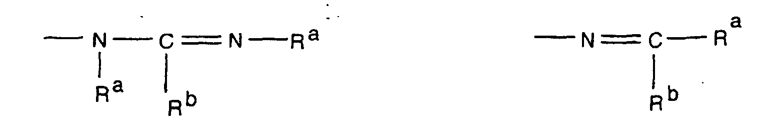 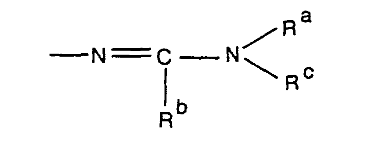 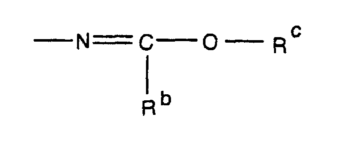 or 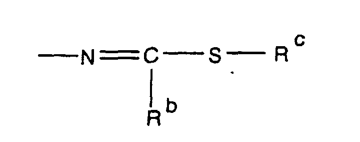 where the radicals R^{a} are each independently of one another hydrogen, MH₂, OH or an aliphatic, aromatic, heterocyclic or another organic radical having a total of 1 to 12 carbon atoms which may also be attached via a hetero atom, the radicals R^{b} are each independently of one another one of the radicals possible for R^{a}, excluding OH, the radicals R^{c} are each independently of one another H or an aliphatic, aromatic, heterocyclic or another organic radical having a total of 1 to 20 carbon atoms, and where the radicals R^{a} to R^{c} may be paired to form a bridge in a group B, the organic group B having a total of 1 to 30 carbon atoms,
R is H or a hydrocarbon radical having 1 to 8 carbon atoms which is unsubstituted or substituted by one or more radicals from the group consisting of halogen, C₁-C₄-alkoxy and C₁-C₄-alkylthio,
W is O or S,
one of the radicals X and Y is
halogen, C₁-C₆-alkyl, C₁-C₆-alkoxy, C₁-C₆-alkylthio, the last 3 radicals being unsubstituted or substituted by one or more radicals from the group consisting of halogen, C₁-C₄-alkoxy and C₁-C₄-alkylthio, or is C₂-C₄-alkenyl, C₂-C₄-alkynyl, C₂-C₄-alkenyloxy or C₂-C₄-alkynyloxy, each of the last 4 radicals being unsubstituted or substituted by one or more halogen atoms, or is C₃-C₆-cycloalkyl or mono- or di-(C₁-C₂-alkyl)amino and
the other of the radicals X and Y is halogen,
C₁-C₄-alkyl, C₁-C₄-alkoxy or C₁-C₄-alkylthio, each of the last 3 radicals being unsubstituted or substituted by one or more radicals from the group consisting of halogen, C₁-C₄-alkoxy and C₁-C₄-alkylthio, or is mono- or di-(C₁-C₂-alkyl)amino and
Z is CH or N.

3. A compound of the formula (I) or a salt thereof as claimed in claim 1 or 2, wherein
B is a group of the formula B1 to B5, 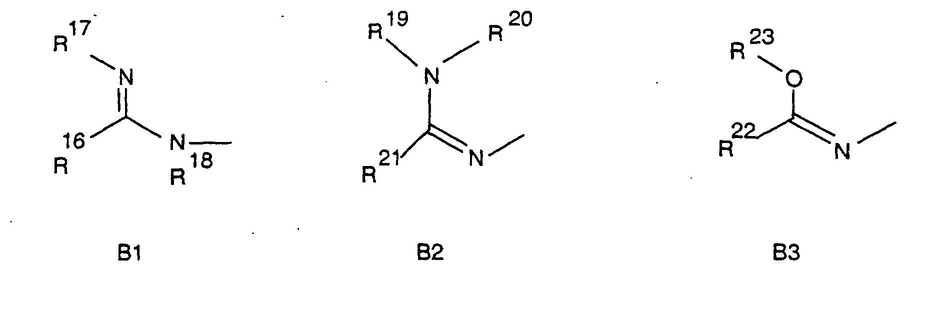 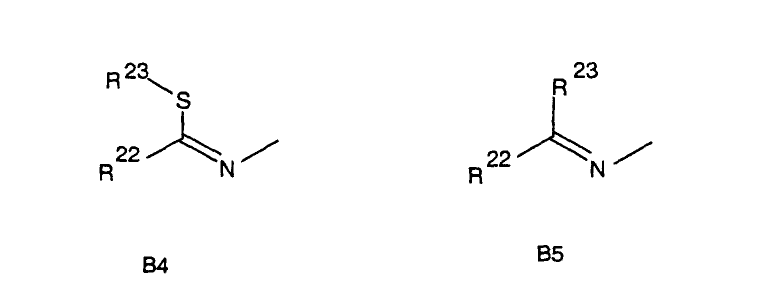
R is hydrogen, C₁-C₅-alkyl, C₁-C₅-haloalkyl, C₂-C₅-alkenyl, C₂-C₅-haloalkenyl, C₂-C₅-alkynyl or C₂-C₅-haloalkynyl,
R¹⁶ is NH₂, hydrogen, aryl or hetaryl, in particular phenyl, each of the last two radicals being unsubstituted or substituted, or C₁-C₈-alkyl, C₂-C₈-alkenyl, C₂-C₈-alkynyl, C₁-C₈-alkoxy, C₂-C₈-alkenyloxy, C₂-C₈-alkynyloxy, C₁-C₈-alkylthio, C₂-C₈-alkenylthio, C₂-C₈-alkynylthio, C₃-C₈-cycloalkyl, C₅-C₈-cycloalkenyl, C₃-C₆-cycloalkoxy, C₅-C₈-cycloalkenyloxy, C₃-C₈-cycloalkylthio, C₅-C₈-cycloalkenylthio, mono- or di-(C₁-C₆)alkylamino, each of the last 17 radicals being unsubstituted or substituted, or benzyl, CN, (C₁-C₆-alkoxy)carbonyl, (C₁-C₆-alkyl)carbonyl, benzyloxycarbonyl or phenylcarbonyl,
R¹⁷ is OH, NH₂, hydrogen, C₁-C₈-alkyl, C₂-C₈-alkenyl, C₂-C₈-alkynyl, C₁-C₈-alkoxy, C₂-C₈-alkenyloxy, C₂-C₈-alkynyloxy, (C₁-C₈-alkyl)carbonyl, (C₁-C₈-alkoxy)carbonyl, benzoyl, benzyloxycarbonyl, (C₁-C₈)-alkylsulfonyl, arylsulfonyl, C₃-C₈-cycloalkyl, C₅-C₈-cycloalkenyl, C₃-C₈-cycloalkoxy, C₅-C₈-cycloalkenyloxy, C₃-C₈-cycloalkylcarbonyl, C₃-C₈-cycloalkoxycarbonyl, C₃-C₈-cycloalkylsulfonyl, mono- or di-(C₁-C₈-alkyl)amino, each of the last 21 radicals being unsubstituted or substituted by one or more radicals from the group consisting of halogen, C₁-C₄-alkoxy, C₁-C₄-haloalkoxy, CN, NO₂, OH, NH₂, mono- and di-(C₁-C₃-alkyl)amino, C₁-C₄-alkylthio and C₁-C₄-haloalkylthio and, in the case of cyclic radicals, also C₁-C₄-alkyl and C₁-C₄-haloalkyl, or is aryl or hetaryl, each of the last 2 radicals being unsubstituted or substituted by one or more radicals from the group consisting of C₁-C₄-alkyl, C₁-C₄-haloalkyl, C₁-C₄-alkoxy, C₁-C₄-haloalkoxy, CN and NO₂,
or the part of the formula 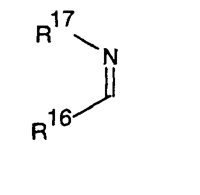 from the group of the abovementioned formula B1 (see definition of B) as a whole forms a heterocyclic ring having an imino function, the heterocyclic ring being unsubstituted or substituted,
R¹⁸ is hydrogen, C₁-C₈-alkyl, C₂-C₈-alkenyl, C₂-C₈-alkynyl, C₁-C₈-alkylsulfonyl, (C₁-C₈-alkyl)carbonyl, (C₁-C₈-alkoxy)carbonyl, C₁-C₅-alkoxy, C₃-C₈-cycloalkyl, C₅-C₈-cycloalkenyl, C₃-C₈-cycloalkylsulfonyl, C₃-C₈-cycloalkylcarbonyl, C₃-C₈-cycloalkoxycarbonyl or C₃-C₈-cycloalkoxy, each of the last 13 radicals being unsubstituted or substituted by one or more radicals from the group consisting of halogen, C₁-C₃-alkoxy, C₁-C₃-haloalkoxy and, in the case of cyclic radicals, also C₁-C₄-alkyl and C₁-C₄-haloalkyl, or is CHO, NN₂, OH, mono- or di-(C₁-C₄-alkyl)amino,
R¹⁹ is a radical from the group of the radicals possible for R¹⁷ and
R²⁰ is hydrogen, C₁-C₈-alkyl, C₃-C₈-cycloalkyl, C₂-C₈-alkenyl, C₃- C₈-cycloalkenyl or C₂-C₆-alkynyl or
NR¹⁹R²⁰ is a heterocyclic ring in which the heterocyclic radical is unsubstituted or substituted by one or more radicals from the group consisting of halogen, C₁-C₄-alkyl, C₁-C₄-haloalkyl, C₁-C₄-alkoxy, C₁-C₄-haloalkoxy, OH, NH₂, NO₂, CN, mono- and di-(C₁-C₅-alkyl)amino,
R²¹ is a radical from the group of the radicals possible for R¹⁶,
R²² is hydrogen, C₁-C₈-alkyl, C₂-C₈-alkenyl, C₂-C₈-alkynyl, C₁-C₈-alkoxyl C₁-C₈-alkylthio, C₃-C₈-cycloalkoxy, C₃-C₈-cycloalkylthio, C₃-C₈-cycloalkyl, C₅-C₈-cycloalkenyl, mono- or di-(C₁-C₈-alkyl)amino, each of the last 11 radicals being unsubstituted or substituted by one or more radicals from the group consisting of CN, halogen, C₁-C₄-alkoxy, C₁-C₄-haloalkoxy and, in the case of cyclic radicals, also C₁-C₄-alkyl and C₁-C₄-haloalkyl, or is an aromatic or heteroaromatic monocycle or polycycle, each of these radicals being unsubstituted or substituted,
R²³ is C₁-C₈-alkyl, C₂-C₈-alkenyl, C₂-C₈-alkynyl, C₃-C₈-cycloalkyl, C₅-C₈-cycloalkenyl, heterocyclyl, aryl or benzyl, each of the last 8 radicals being unsubstituted or substituted by one or more radicals from the group consisting of halogen, C₁-C₄-alkoxy, C₁-C₄-haloalkoxy, CN and NO₂ and, in the case of cyclic radicals, also C₁-C₄-alkyl and C₁-C₄-haloalkyl,
R²⁴ is hydrogen, OH, NH₂, aryl or a heterocycle, each of the last two radicals being unsubstituted or substituted, or is C₁-C₈-alkyl, C₂-C₈-alkenyl, C₂-C₈-alkynyl, C₁-C₈-alkoxy, C₂-C₈-alkenyloxy, C₂-C₈-alkynyloxy, C₁-C₈-alkylthio, C₂-C₈-alkenylthio, C₂-C₈-alkynylthio, benzyl, aryloxy, benzyloxy, C₃-C₈-cycloalkyl, C₅-C₈-cycloalkenyl, C₃-C₈-cycloalkoxy, C₅-C₈-cycloalkenyloxy, C₅-C₈-cycloalkylthio or C₅-C₈-cycloalkenylthio, mono- or di-(C₁-C₈-alkyl)amino, each of the last 20 radicals being unsubstituted or substituted by one or more radicals from the group consisting of halogen, C₁-C₄-alkoxy, C₁-C₄-haloalkoxy, OH, NH₂, mono- and di(C₁-C₄-alkyl)amino, CN and NO₂ and, in the case of cyclic radicals, also C₁-C₄-alkyl and C₁-C₄-haloalkyl,
R²⁵ is a radical from the group of the radicals possible for R²⁴,
R²⁶ is hydrogen, OH, MH₂, CN, C₁-C₈-alkoxy, C₂-C₈-alkenyloxy, C₂-C₈-alkynyloxy, C₁-C₈-alkyl, C₂-C₈-alkenyl, C₂-C₈-alkynyl, C₁-C₈-alkylsulfonyl, C₂-C₈-alkenylsulfonyl, C₂-C₈-alkynylsulfonyl,
C₃-C₈-cycloalkoxy, C₅-C₈-cycloalkenyloxy, C₃-C₈-cycloalkyl, C₅-C₈-cycloalkenyl, C₃-C₈-cycloalkylsulfonyl, C₅-C₈-cycloalkenylsulfonyl, arylsulfonyl or aryl or a heterocycle or benzyl, (C₁-C₈-alkoxy)carbonyl, (C₁-C₈-alkyl)carbonyl, phenoxycarbonyl, benzylcarbonyl or heterocyclylcarbonyl, each of the last 24 radicals being unsubstituted or substituted,
R²⁷ is a radical from the group of the radicals possible for R²⁴, excluding OH,
R²⁸ is H, OH, CHO, C₁-C₈-alkyl, C₂-C₈-alkenyl, C₂-C₈-alkynyl, C₁-C₈-alkoxy, C₂-C₈-alkenyloxy, C₂-C₈-alkynyloxy, C₁-C₈-alkylsulfonyl, C₂-C₈-alkenylsulfonyl, C₂-C₈-alkynylsulfonyl, (C₁-C₈-alkyl)carbonyl, (C₂-C₈-alkenyl)carbonyl, (C₂-C₈-alkynyl)-carbonyl, (C₁-C₈-alkoxy)carbonyl, (C₂-C₈-alkenyloxy)carbonyl, (C₂-C₈-alkynyloxy)carbonyl, mono- or di-(C₁-C₈-alkyl)-aminocarbonyl, C₃-C₈-cycloalkyl, C₅-C₈-cycloalkenyl, C₃-C₈-cycloalkoxy, C₅-C₈-cycloalkenyloxy, C₃-C₉-cycloalkylsulfonyl, C₅-C₈-cycloalkenylsulfonyl, C₃-C₈-cycloalkoxycarbonyl, C₅-C₈-cycloalkenyloxycarbonyl, benzoyl, benzyl, benzylcarbonyl, benzyloxycarbonyl, aryl or a heterocycle, each of the last 31 radicals being unsubstituted or substituted, or is SO₂NH₂ or CONH₂,
R²⁹ is a radical from the group of the radicals possible for R²⁸, excluding OH,
R³⁰ is a radical from the group of the radicals possible for R¹⁹,
R³¹ is a radical from the group of the radicals possible for R²⁰,
or NR³⁰R³¹ as a whole forms a heterocyclic ring as defined for
NR¹⁹R²⁰, R³² is hydrogen, C₁-C₄-alkyl, C₁-C₄-haloalkyl, CHO, C₁-C₄-alkylsulfonyl, C₂-C₄-alkenyl, C₂-C₄-alkynyl, C₂-C₄-haloalkenyl, C₁-C₄-haloalkyl, C₂-C₄-haloalkynyl, C₁-C₄-haloalkylsulfonyl, (C₁-C₄-alkyl)carbonyl, (C₁-C₄-alkoxy)carbonyl, (C₁-C₄-haloalkyl)carbonyl or (C₁-C₄-haloalkoxy)carbonyl,
R³³ is a radical from the group of the radicals possible for R³² or
NR³²R³³ as a whole forms a heterocyclic ring as defined for NR¹⁹R²⁰.

4. A compound of the formula (I) or a salt thereof as claimed in any of claims 1 to 3, wherein
R is hydrogen, C₂-C₃-alkyl, C₁-C₃-alkoxy,
R¹ is H, CN, NO₂, halogen, C₁-C₆-alkoxy, C₁-C₆-alkyl, C₁-C₆-haloalkoxy, C₁-C₆-haloalkyl, (C₁-C₆-alkoxy)carbonyl, (C₃-C₆-cycloalkoxy)carbonyl or (C₃-C₆-cycloalkyl)methoxycarbonyl, (C₃-C₆-cycloalkoxy)carbonyl, (C₃-C₆-cycloalkyl)-methoxycarbonyl, C₁-C₆-alkylthio, C₁-C₆-haloalkylthio, C₁-C₆-alkylsulfonyl, C₁-C₆-haloalkylsulfonyl, SO₂NH₂, CONH₂, mono- or di-(C₁-C₄-alkyl)aminocarbonyl or -aminosulfonyl or a radical of the formula 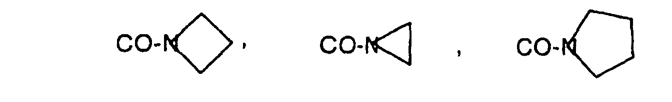 or
C₁-C₆-alkylcarbonyl, C₁-C₆-haloalkylcarbonyl, CHO, cyclopropylcarbonyl, cyclobutylcarbonyl or NR²⁸R²⁹;
R² is hydrogen, C₁-C₆-alkyl, C₁-C₆-haloalkyl, C₁-C₆-alkoxy, C₁-C₆-haloalkoxy, halogen or CN;
R³, R⁵, R⁷, R⁹, R¹¹, R¹³ and R¹⁴ are each a radical from the group of radicals possible for R¹,
R⁴, R⁶, R⁸, R¹⁰, R¹² are each a radical from the group of radicals possible for R²,
R¹⁵ is H, C₁-C₃-alkyl or phenyl;
R¹⁶ is H, NH₂, mono- or di-(C₁-C₆-alkyl)amino, C₁-C₆-alkyl, phenyl or C₁-C₆-alkoxy, each of the last 5 radicals being unsubstituted or substituted by one or more radicals from the group consisting of halogen, C₁-C₃-alkoxy, C₁-C₃-haloalkoxy, CN, NO₂ and, in the case of phenyl, also C₁-C₃-alkyl and C₁-C₃-haloalkyl;
R¹⁷ is mono- or di-(C₁-C₆-alkyl)amino, C₁-C₆-alkyl, C₁-C₆-alkoxy, phenyl, benzyl, each of the last 6 radicals being unsubstituted or substituted by one or more radicals from the group consisting of halogen, C₁-C₃-alkoxy, C₁-C₃-haloalkoxy and, in the case of phenyl, also C₁-C₃-alkyl and C₁-C₃-haloalkyl, or is OH, NH₂ or H; or the part of the formula R¹⁷-N=C-R¹⁶ from the group of the formula B1 in the definition for B as a whole forms a heterocycle of the formula D1 or D2 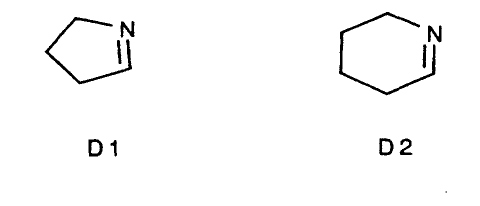
R¹⁸ is H, C₁-C₆-alkyl or C₁-C₆-haloalkyl,
R¹⁹ is H, C₁-C₆-alkyl, C₁-C₆-haloalkyl, C₁-C₆-haloalkoxy or phenyl,
R²⁰ is H, C₁-C₆-alkyl, C₁-C₆-haloalkyl or phenyl or
NR¹⁹R²⁰ as a whole forms a heterocycle of the formula 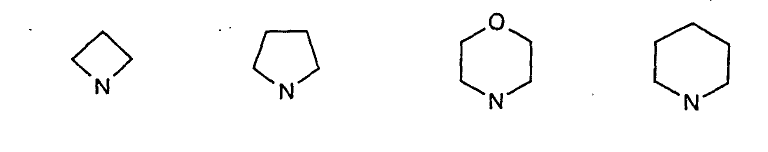
or 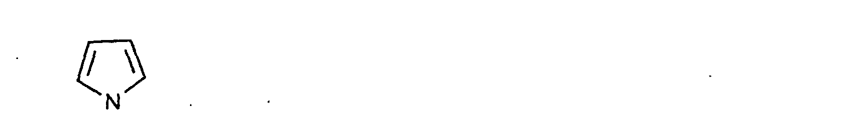
R²¹ is a radical from the group of radicals possible for R¹⁶;
R²² is H, C₁-C₆-alkyl, C₁-C₆-haloalkyl or phenyl;
R²³ is C₁-C₆-alkyl or C₁-C₆-haloalkyl;
R²⁸ is H, C₁-C₆-alkyl, OH, C₁-C₆-haloalkyl, or C₁-C₆-alkoxy,
R²⁹ is H, C₁-C₆-alkyl, C₁-C₆-haloalkyl, CHO, C₁-C₃-alkylsulfonyl, C₁-C₃-alkoxycarbonyl or C₁-C₃-alkylcarbonyl;
X and Y are each independently of the other Cl, F, CF₃, CH₃, CH₂CH₃, OCH₃, OC₂H₅, N(CH₃)₂, NHCH₃ or OCH₂CF₃.

5. A compound of the formula (I) or a salt thereof as claimed in any of claims 1 to 4, wherein
A is a bridge of the formula 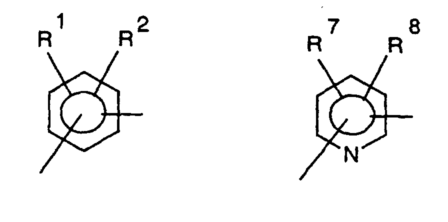 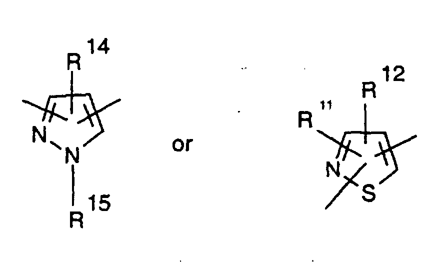 and
B is a group of the formulae B1, B2 and B3
-NR¹⁸-C(R¹⁶)=N-R¹⁷ (B1)
-N=C(R²¹)-NR¹⁹R²⁰ (B2)
-N=C(R²²)-O-R²³ (B3)
where the radicals R¹, R², R⁷, R⁸, R¹¹, R¹², R¹⁴, R¹⁵ and R¹⁶ to R²³ in the formulae for A and B are as defined in the formula (I).

6. A process for preparing compounds of the formula (I) or salts thereof as defined in any of claims 1 to 5, which comprises
a) reacting a compound of the formula (II)
B-A-SO₂NH₂ (II)
with a heterocyclic carbamate of the formula (III) 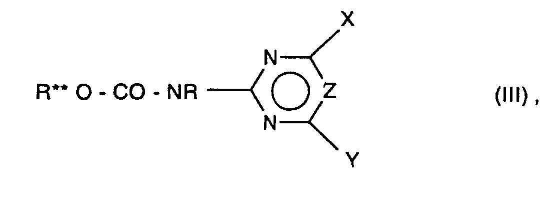 where R** is unsubstituted or substituted phenyl or C₁-C₄-alkyl, or
b) reacting a sulfonyl isocyanate of the formula (IV)
B-A-SO₂-N=C=O (IV)
with a heterocyclic amine of the formula (V) 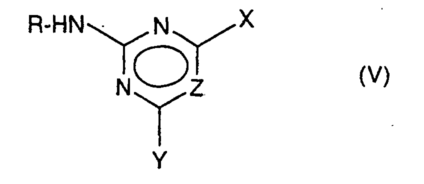 or
c) reacting a sulfonyl chloride of the formula (VI)
B-A-SO₂Cl (VI)
with a heterocyclic amine of the formula (V) already mentioned in the presence of a cyanate, or
d) reacting a sulfonyl chloride of the formula (II) already mentioned with a (thio)isocyanate of the formula (VII) 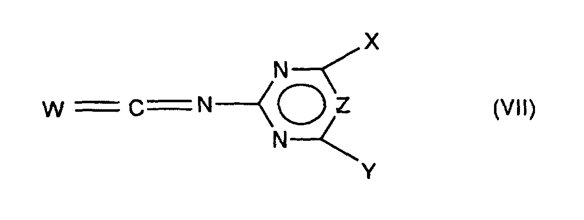 in the presence of a suitable base,
e) if B is a group containing a fragment of the formula -N=C-NR^{a}- where R^{a} is NH₂, OH or an organic radical having 1 to 12 carbon atoms, reacting an amino-substituted sulfonylurea of the formula (VIII), 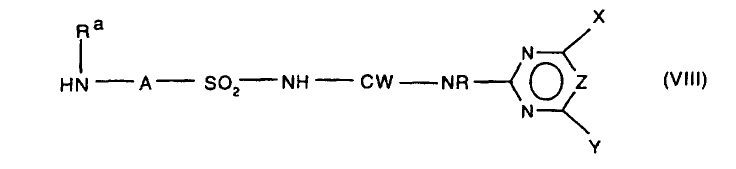 where R^{a} is as defined above,
with suitable reagents which contain a fragment of the formula (IXa) 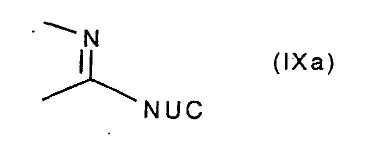 where NUC is a nucleofugal group,
f) if B is a group containing a fragment of the formula 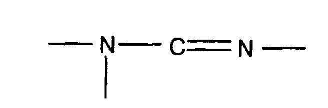 reacting an amino-substituted sulfonylurea of the formula (VIII) already mentioned (cf. e) where R^{a} = hydrogen with suitable reagents containing a fragment of the formula (IXb) 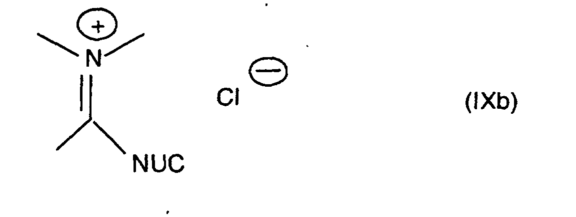 where NUC is a nucleofugal group,
where the radicals A, B, R, W, X, Y and Z in the formulae (II) to (IX) are as defined for the formula (I) and where the compounds initially obtained in variants a) to c) are compounds of the formula (I) where W is an oxygen atom.

7. A herbicidal or plant growth regulating composition, which comprises at least one compound of the formula (I) or a salt thereof as claimed in any of claims 1 to 5 and formulation auxiliaries customary in crop protection.

8. A method for controlling harmful plants or for regulating the growth of plants, which comprises applying an active amount of at least one compound of the formula (I) of a salt thereof as claimed in any of claims 1 to 5 to the plants, seeds thereof or the area where they grow.

9. Use of the compounds of the formula (I) or salts thereof as claimed in any of claims 1 to 5 as herbicides or plant growth regulators.

10. A compound of the formula (XII),
B-A-SO₂-R^{z} (XIII)
in which
R^{z} = NH₂, mono- or di-(C₁-C₄-alkyl)amino, halogen or =N=CO and B and A are as defined in the formula (I) according to any of claims 1 to 5,
except for compounds of the formula (XII) in which
a) R^{z} = amino,
A = phenyl which carries two radicals Y' and Z',
where
Y' = H, a halogen atom or an alkyl group,
Z' = H, a halogen atom or an alkyl, alkoxy, trifluoromethyl, sulfamyl or nitro group, and
B = a group of the formula -N=CR^{b}-NH₂ in which R^{b} = a saturated or unconjugated unsaturated substituted or unsubstituted aliphatic or cycloaliphatic hydrocarbon radical which may also be interrupted by one or more hetero-atoms, or a substituted or unsubstituted aromatic radical, it being possible for the radical in question to be attached via an oxygen atom or an NH group,
b) R^{z} = dimethylamino or diethylamino,
A = phenyl which may optionally be substituted by chlorine, bromine, methyl, methoxy or trifluoromethyl in the ortho-position to the group SO₂-R^{z}, and
B = a group of the formula -N=CH-NR'-Ar in which Ar is phenyl or 4-dimethylaminosulfonylphenyl or 4-diethylaminosulfonylphenyl, where each of the two lastmentioned radicals may optionally be substituted by 3-chlorine, 3-bromine, 3-methyl, 3-methoxy or 3-trifluoromethyl in the phenyl moiety, and R is hydrogen or an N-(C₁-C₄)alkylamino- or N,N-di-(C₁-C₄)alkylamino-carbonyl,
c) R^{z} = SO₂Cl,
A = phenyl and
B = a group of the formula -NH-C(=NH)-NH₂, and
d) R^{z} = SO₂NH₂,
A = phenyl which may additionally optionally be substituted,
B = a radical of the formula -NR'-C(=NH)-R" in which R' is hydrogen or (C₁-C₁₈)alkyl and R" is [(C₁-C₄)alkoxy-carbonyl]methyl.

## Revendications

1. Composés de formule (I) ou leurs sels 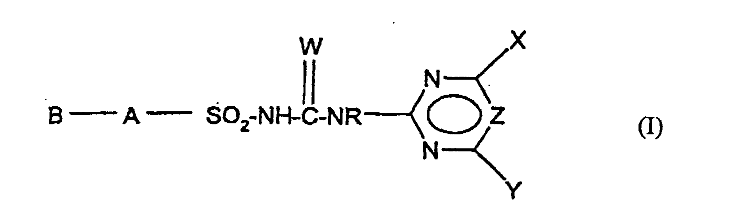 dans laquelle
A représente un pont ayant l'une des formules A₁ à A₉: 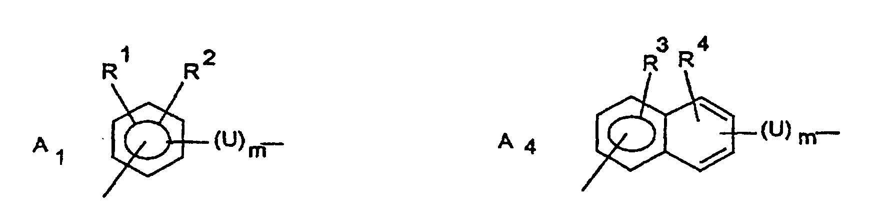 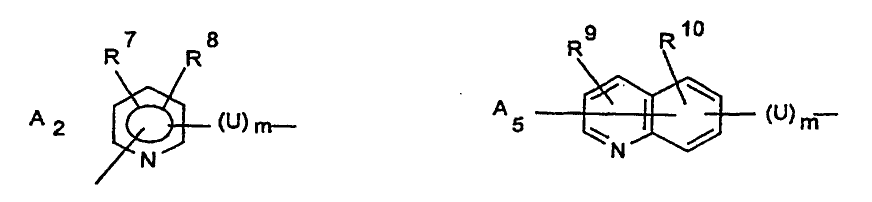 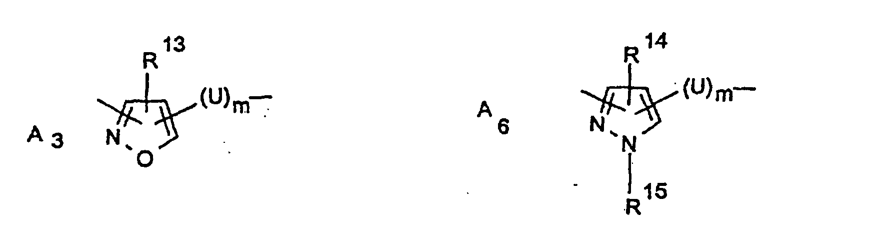 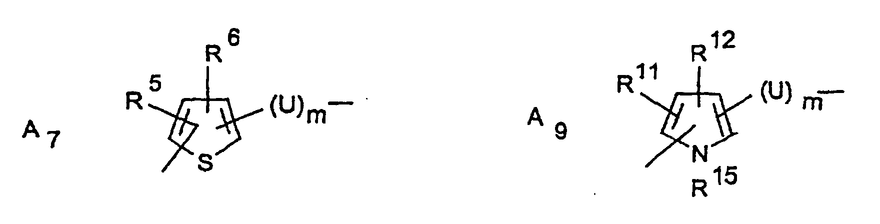 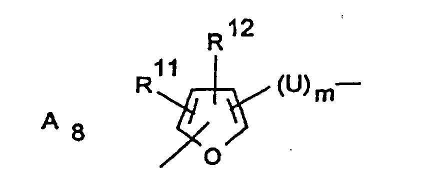
m est 0 ou 1,
U représente un groupe CH₂, O ou NH ou (alkyl en C₁-C₃)N,
R¹ représente un atome d'hydrogène ou un reste CN, halogéno, azido, NO₂, OH, alkyle en C₁-C₈, alcényle en C₂-C₈, alcynyle en C₂-C₈, alcoxy en C₁-C₈, alcényloxy en C₂-C₈, alcynyloxy en C₂-C₈, cycloalkyle en C₃-C₈, cycloalkyloxy en C₃-C₈, cycloalcényle en C₅-C₈, cycloalcényloxy en C₅-C₈, chacun des 10 derniers restes cités étant non substitué ou substitué par un ou plusieurs restes du groupe constitué par les restes halogéno, alcoxy en C₁-C₄, halogénoalcoxy en C₁-C₄, alkylthio en C₁-C₄, halogénoalkylthio en C₁-C₄, OH, CN, NO₂, oxo, alkylsulfonyle en C₁-C₄, halogénoalkylsulfonyle en C₁-C₄, (alcoxy en C₁-C₄)carbonyle, (halogénoalcoxy en C₁-C₄)carbonyle et NR³²R³³, ainsi que, dans le cas des restes cycliques, par des restes alkyle en C₁-C₄ et halogénoalkyle en C₁-C₄, ou un reste COR²⁴, CS-R²⁵, C(=NR²⁶)R²⁷, NR²⁸R²⁹, ou un hétérocycle de 3 à 7 chaînons ayant jusqu'à 4 hétéroatomes du groupe constitué par N, O et S, non substitué ou substitué par un ou plusieurs restes du groupe constitué par les restes halogéno, alkyle en C₁-C₄, OH, CN, NO₂, CHO, NH₂, mono- et di(alkyl en C₁-C₄)amino, alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄, (alkyl en C₁-C₄)carbonyle, (alcoxy en C₁-C₄)carbonyle et oxo, ou un reste aryle, hétéroaryle, aryl-(alkyle en C₁-C₃) ou hétéroaryl-(alkyle en C₁-C₃), chacun des 4 derniers restes cités étant non substitué ou substitué dans la partie aryle ou hétéroaryle par un ou plusieurs restes du groupe constitué par les restes halogéno, OH, CN, nitro, alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄, alkylsulfonyle en C₁-C₄, halogénoalkylsulfonyle en C₁-C₄, (alkyl en C₁-C₄)carbonyle, (halogénoalkyl en C₁-C₄)carbonyle, (alcoxy en C₁-C₄)carbonyle, (halogénoalcoxy en C₁-C₄)carbonyle, mono- et di(alkyl en C₁-C₄)amino, CHO, NH₂, CONH₂, mono- et di(alkyl en C₁-C₄)aminocarbonyle,
R² représente un atome d'hydrogène ou un reste alkyle en C₁-C₈, alcényle en C₂-C₈, alcynyle en C₂-C₈, alcoxy en C₁-C₈, alcényloxy en C₂-C₈, alcynyloxy en C₂-C₈, cycloalkyle en C₃-C₈, cycloalcényle en C₅-C₈, cycloalcoxy en C₅-C₈, cycloalcényloxy en C₅-C₈, chacun des 10 derniers restes cités étant non substitué ou substitué par un ou plusieurs restes du groupe constitué par les restes halogéno, alcoxy en C₁-C₄ et halogénoalcoxy en C₁-C₄, ainsi que, dans le cas des restes cycliques, par des restes alkyle en C₁-C₄ et halogénoalkyle en C₁-C₄, ou un reste halogéno, CN, NH₂, mono- ou di(alkyl en C₁-C₄)amino,
R³, R⁵, R⁷, R⁹, R¹¹, R¹³ et R¹⁴ représentent chacun un reste choisi dans le groupe constitué par les restes possibles pour R',
R⁴, R⁶, R⁸, R¹⁰ et R¹² représentent chacun un reste choisi dans le groupe constitué par les restes possibles pour R²,
R¹⁵ représente un atome d'hydrogène ou un reste alkyle en C₁-C₈, cycloalkyle en C₃-C₈, halogénoalkyle en C₁-C₈, halogénocycloalkyle en C₃-C₈ ou aryle pouvant être non substitué ou substitué par un ou plusieurs restes du groupe constitué par les restes halogéno, alkyle en C₁-C₄, halogénoalkyle en C₁-C₄, alcoxy en C₁-C₄ et halogénoalcoxy en C₁-C₄,
B représente un groupe qui contient un fragment de 3 atomes contenant un groupe imino, choisi dans le groupe constitué par les fragments de formule N=C-N, N=C-S, N=C-O et N=C-C, et qui est lié au reste A par l'intermédiaire d'un atome d'azote du fragment,
R représente un atome d'hydrogène ou un reste aliphatique,
W représente un atome d'oxygène ou de soufre,
X, Y représentent, indépendamment l'un de l'autre, un atome d'hydrogène ou un reste halogéno, alkyle en C₁-C₆, alcoxy en C₁-C₆, alkylthio en C₁-C₆, cycloalkyle en C₃-C₆, cycloalcoxy en C₃-C₆, cycloalkylthio en C₃-C₆, alcényle en C₂-C₆, cycloalcényle en C₅-C₆, alcynyle en C₂-C₆, alcényloxy en C₃-C₆, cycloalcényloxy en C₅-C₆, alcynyloxy en C₃-C₆, chacun des 11 derniers restes cités étant non substitué ou substitué par un ou plusieurs restes du groupe constitué par les restes halogéno, alcoxy en C₁-C₄, alkylthio en C₁-C₄, halogénoalcoxy en C₁-C₄, ainsi que, dans le cas des restes cycliques, par des restes alkyle en C₁-C₄ et halogénoalkyle en C₁-C₄, ou un reste mono- ou di(alkyl en C₁-C₄)amino, et
Z représente CH ou N.

2. Composés de formule (I) ou leurs sels selon la revendication 1, **caractérisés en ce que**
B représente un groupe contenant un groupe imino de formule 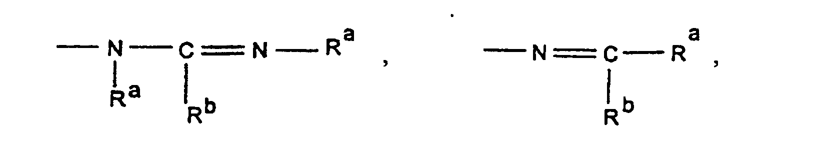 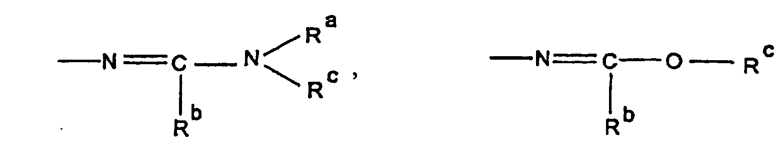 ou 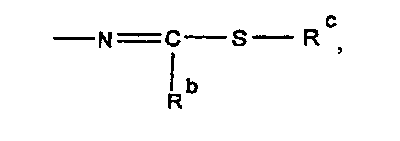 où les R^{a} représentent, indépendamment les uns des autres, un atome d'hydrogène, un reste NH₂ ou OH ou un reste organique aliphatique, aromatique, hétérocyclique ou autre, ayant en tout 1 à 12 atomes de carbone, qui peut aussi être lié par l'intermédiaire d'un hétéroatome, les R^{b} représentent chacun, indépendamment les uns des autres, l'un des restes possibles pour R^{a} à l'exception de OH, les R^{c} représentent, indépendamment les uns des autres, un atome d'hydrogène ou un reste organique aliphatique, aromatique, hétérocyclique ou autre, ayant en tout 1 à 20 atomes de carbone, les restes R^{a} à R^{c} d'un groupe B pouvant s'apparier pour former un pont et le groupe organique B ayant en tout 1 à 30 atomes de carbone,
R représente un atome d'hydrogène ou un reste hydrocarboné de 1 à 8 atomes de carbone non substitué ou substitué par un ou plusieurs restes du groupe constitué par les restes halogéno, alcoxy en C₁-C₄ et alkylthio en C₁-C₄,
W représente O ou S,
l'un des restes X et Y représente un reste halogéno, alkyle en C₁-C₆, alcoxy en C₁-C₆, alkylthio en C₁-C₆, les 3 derniers restes cités étant non substitués ou substitués par un ou plusieurs restes du groupe constitué par les restes halogéno, alcoxy en C₁-C₄ et alkylthio en C₁-C₄, ou un reste alcényle en C₂-C₄, alcynyle en C₂-C₄, alcényloxy en C₂-C₄, alcynyloxy en C₂-C₄, chacun des 4 derniers restes cités étant non substitué ou substitué par un ou plusieurs atomes d'halogène, ou un reste cycloalkyle en C₃-C₆ ou mono- ou di(alkyl en C₁-C₂)amino, et
l'autre reste X ou Y représente un reste halogéno, alkyle en C₁-C₄, alcoxy en C₁-C₄
ou alkylthio en C₁-C₄, chacun des 3 derniers restes cités étant non substitué ou substitué par un ou plusieurs restes du groupe constitué par les restes halogéne, alcoxy en C₁-C₄ et alkylthio en C₁-C₄, ou un reste mono- ou di(alkyl en C₁-C₂)amino, et
Z représente CH ou N.

3. Composés de formule (I) ou leurs sels selon la revendication 1 ou 2, **caractérisés en ce que**
B représente un groupe de formule B1 à B5 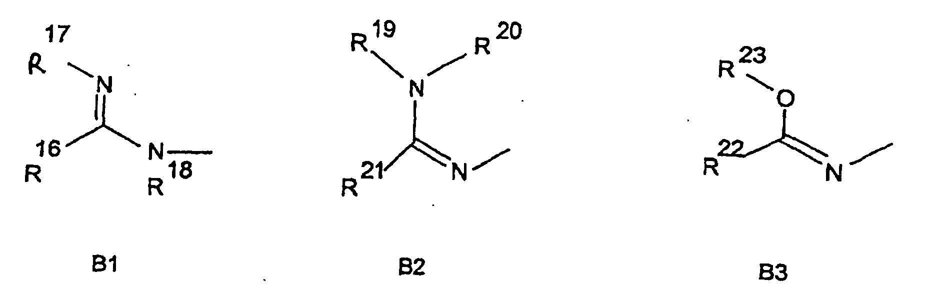 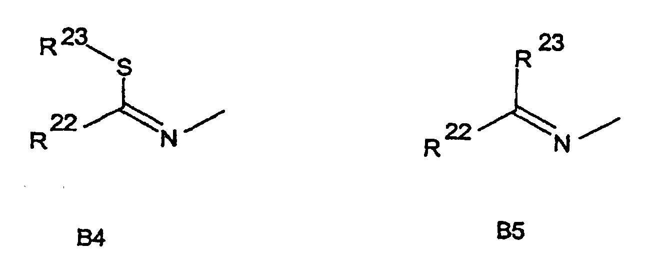
R représente un atome d'hydrogène ou un reste alkyle en C₁-C₅, halogénoalkyle en C₁-C₅, alcényle en C₂-C₅, halogénoalcényle en C₂-C₅, alcynyle en C₂-C₅ ou halogénoalcynyle en C₂-C₅,
R¹⁶ représente un reste NH₂, un atome d'hydrogène, un reste aryle ou hétéroaryle, en particulier un reste phényle, chacun des deux derniers restes cités étant non substitué ou substitué, ou un reste alkyle en C₁-C₈, alcényle en C₂-C₈, alcynyle en C₂-C₈, alcoxy en C₁-C₈, alcényloxy en C₂-C₈, alcynyloxy en C₂-C₈, alkylthio en C₁-C₈, alcénylthio en C₂-C₈, alcynylthio en C₂-C₈, cycloalkyle en C₃-C₈, cycloalcényle en C₅-C₈, cycloalcoxy en C₃-C₆, cycloalcényloxy en C₅-C₈, cycloalkylthio en C₃-C₈, cycloalcénylthio en C₅-C₈, mono- ou di(alkyl en C₁-C₆)amino, chacun des 17 derniers restes cités étant non substitué ou substitué, ou un reste benzyle, CN, (alcoxy en C₁-C₆)carbonyle, (alkyl en C₁-C₆)carbonyle, benzyloxycarbonyle ou phénylcarbonyle,
R¹⁷ représente un reste OH, NH₂, un atome d'hydrogène, un reste alkyle en C₁-C₈, alcényle en C₂-C₈, alcynyle en C₂-C₈, alcoxy en C₁-C₈, alcényloxy en C₂-C₈, alcynyloxy en C₂-C₈, (alkyl en C₁-C₈)carbonyle, (alcoxy en C₁-C₈)carbonyle, benzoyle, benzyloxycarbonyle, (alkyl en C₁-C₈)sulfonyle, arylsulfonyle, cycloalkyle en C₃-C₈, cycloalcényle en C₅-C₈, cycloalcoxy en C₃-C₈, cycloalcényloxy en C₅-C₈, (cycloalkyl en C₃-C₈)carbonyle, (cycloalcoxy en C₃-C₈)carbonyle, cycloalkylsulfonyle en C₃-C₈, mono- ou di(alkyl en C₁-C₈)amino, chacun des 21 derniers restes cités étant non substitué ou substitué par un ou plusieurs restes du groupe constitué par les restes halogéno, alcoxy en C₁-C₄, halogénoalcoxy en C₁-C₄, CN, NO₂, OH, NH₂, mono- et di(alkyl en C₁-C₃)amino, alkylthio en C₁-C₄ et halogénoalkylthio en C₁-C₄, ainsi que, dans le cas de restes cycliques, par des restes alkyle en C₁-C₄ et halogénoalkyle en C₁-C₄, ou un reste aryle ou hétéroaryle, chacun des 2 derniers restes cités étant non substitué ou substitué par un ou plusieurs restes du groupe constitué par les restes alkyle en C₁-C₄, halogénoalkyle en C₁-C₄, alcoxy en C₁-C₄, halogénoalcoxy en C₁-C₄, CN et NO₂, ou le fragment de formule 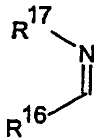 du groupe de formule
B1 précité (voir la définition de B) représente globalement un noyau hétérocyclique à fonction imino, le noyau hétérocyclique étant non substitué
ou substitué,
R¹⁸ représente un atome d'hydrogène, un reste alkyle en C₁-C₈, alcényle en C₂-C₈, alcynyle en C₂-C₈, alkylsulfonyle en C₁-C₈, (alkyl en C₁-C₈)carbonyle, (alcoxy en C₁-C₈)carbonyle, alcoxy en C₁-C₅, cycloalkyle en C₃-C₈, cycloalcényle en C₅-C₈, cycloalkylsulfonyle en C₃-C₈, (cycloalkyl en C₃-C₈)carbonyle, (cycloalcoxy en C₃-C₈)carbonyle, cycloalcoxy en C₃-C₈, les 13 derniers restes cités étant non substitués ou substitués par un ou plusieurs restes du groupe constitué par les restes halogéno, alcoxy en C₁-C₃, halogénoalcoxy en C₁-C₃ ainsi que, dans le cas des restes cycliques, par des restes alkyle en C₁-C₄ et halogénoalkyle en C₁-C₄, ou un reste CHO, NH₂, OH, mono- ou di(alkyl en C₁-C₄)amino,
R¹⁹ représente un reste du groupe constitué par les restes possibles pour R¹⁷ et
R²⁰ représente un atome d'hydrogène ou un reste alkyle en C₁-C₈, cycloalkyle en C₃-C₈, alcényle en C₂-C₈, cycloalcényle en C₃-C₈ ou alcynyle en C₂-C₆, ou
NR¹⁹R²⁰ représente un noyau hétérocyclique, le reste hétérocyclique étant non substitué ou substitué par un ou plusieurs restes du groupe constitué par les restes halogéno, alkyle en C₁-C₄, halogénoalkyle en C₁-C₄, alcoxy en C₁-C₄, halogénoalcoxy en C₁-C₄, OH, NH₂, NO₂, CN, mono- et di(alkyl en C₁-C₅)amino,
R²¹ représente un reste du groupe constitué par les restes possibles pour R¹⁶,
R²² représente un atome d'hydrogène, un reste alkyle en C₁-C₈, alcényle en C₂-C₈, alcynyle en C₂-C₈, alcoxy en C₁-C₈, alkylthio en C₁-C₈, cycloalcoxy en C₃-C₈, cycloalkylthio en C₃-C₈, cycloalkyle en C₃-C₈, cycloalcényle en C₅-C₈, mono- ou di(alkyl en C₁-C₈)amino, chacun des 11 derniers restes cités étant non substitué ou substitué par un ou plusieurs restes du groupe constitué par les restes CN, halogéno, alcoxy en C₁-C₄ et halogénoalcoxy en C₁-C₄, ainsi que, dans le cas des restes cycliques, par des restes alkyle en C₁- C₄ et halogénoalkyle en C₁-C₄, ou un système monocyclique ou polycyclique aromatique ou hétéroaromatique, chacun de ces restes étant non substitué ou substitué,
R²³ représente un reste alkyle en C₁-C₈, alcényle en C₂-C₈, alcynyle en C₂-C₈, cycloalkyle en C₃-C₈, cycloalcényle en C₅-C₈, hétérocyclyle, aryle ou benzyle, chacun des 8 derniers restes cités étant non substitué ou substitué par un ou plusieurs restes du groupe constitué par les restes halogéno, alcoxy en C₁-C₄, halogénoalcoxy en C₁-C₄, CN et NO₂, ainsi que, dans le cas des restes cycliques, par des restes alkyle en C₁-C₄ et halogénoalkyle en C₁-C₄,
R²⁴ représente un atome d'hydrogène, un reste OH, NH₂ ou aryle ou un reste hétérocyclique, chacun des deux derniers restes cités étant non substitué ou substitué, ou un reste alkyle en C₁-C₈, alcényle en C₂-C₈, alcynyle en C₂-C₈, alcoxy en C₁-C₈, alcényloxy en C₂-C₈, alcynyloxy en C₂-C₈, alkylthio en C₁-C₈, alcénylthio en C₂-C₈, alcynylthio en C₂-C₈, benzyle, aryloxy, benzyloxy, cycloalkyle en C₃-C₈, cycloalcényle en C₅-C₈, cycloalcoxy en C₃-C₈, cycloalcényloxy en C₅-C₈, cyclalkylthio en C₅-C₈ ou cycloalcénylthio en C₅-C₈, mono- ou di(alkyl en C₁-C₈)amino, chacun des 20 derniers restes cités étant non substitué ou substitué par un ou plusieurs restes du groupe constitué par les restes halogéno, alcoxy en C₁-C₄, halogénoalcoxy en C₁-C₄, OH, NH₂, mono- et di(alkyl en C₁-C₄)amino, CN et NO₂, ainsi que, dans le cas de restes cycliques, par des restes alkyle en C₁-C₄ et halogénoalkyle en C₁-C₄,
R²⁵ représente un reste du groupe constitué par les restes possibles pour R²⁴,
R²⁶ représente un atome d'hydrogène ou un reste OH, NH₂, CN, alcoxy en C₁-C₈, alcényloxy en C₂-C₈, alcynyloxy en C₂-C₈, alkyle en C₁-C₈, alcényle en C₂-C₈, alcynyle en C₂-C₈, alkylsulfonyle en C₁-C₈, alcénylsulfonyle en C₂-C₈, alcynylsulfonyle en C₂-C₈, cycloalcoxy en C₃-C₈, cycloalcényloxy en C₅-C₈, cycloalkyle en C₃-C₈, cycloalcényle en C₅-C₈, cycloalkylsulfonyle en C₃-C₈, cycloalcénylsulfonyle en C₅-C₈, arylsulfonyle ou aryle ou un reste hétérocyclique ou benzyle, (alcoxy en C₁-C₈)carbonyle, (alkyl en C₁-C₈)carbonyle, phénoxycarbonyle, benzylcarbonyle ou hétérocyclylcarbonyle, chacun des 24 derniers restes cités étant non substitué ou substitué,
R²⁷ représente un reste du groupe constitué par les restes possibles pour R²⁴, à l'exception de OH,
R²⁸ représente un atome d'hydrogène ou un reste OH, CHO, alkyle en C₁-C₈, alcényle en C₂-C₈, alcynyle en C₂-C₈, alcoxy en C₁-C₈, alcényloxy en C₂-C₈, alcynyloxy en C₂-C₈, alkylsulfonyle en C₁-C₈, alcénylsulfonyle en C₂-C₈, alcynylsulfonyle en C₂-C₈, (alkyl en C₁-C₈)carbonyle, (alcényl en C₂-C₈)carbonyle, (alcynyl en C₂-C₈)carbonyle, (alcoxy en C₁-C₈)carbonyle, (alcényloxy en C₂-C₈)carbonyle, (alcynyloxy en C₂-C₈)carbonyle, mono- ou di(alkyl en C₁-C₈)aminocarbonyle, cycloalkyle en C₃-C₈, cycloalcényle en C₅-C₈, cycloalcoxy en C₃-C₈, cycloalcényloxy en C₅-C₈, cycloalkylsulfonyle en C₃-C₉, cycloalcénylsulfonyle en C₅-C₈, (cycloalcoxy en C₃-C₈)carbonyle, (cycloalcényloxy en C₅-C₈)carbonyle, benzoyle, benzyle, benzylcarbonyle, benzyloxycarbonyle ou aryle ou un reste hétérocyclique, chacun des 31 derniers restes cités étant non substitué ou substitué, ou un reste SO₂NH₂ ou CONH₂,
R²⁹ représente un reste du groupe constitué par les restes possibles pour R²⁸ à l'exception de OH,
R³⁰ représente un reste du groupe constitué par les restes possibles pour R¹⁹,
R³¹ représente un reste du groupe constitué par les restes possibles pour R²⁰, ou
NR³⁰R³¹ représente globalement un reste hétérocyclique tel que défini de façon correspondante pour NR¹⁹R²⁰,
R³² représente un atome d'hydrogène ou un reste alkyle en C₁-C₄, halogénoalkyle en C₁-C₄, CHO, alkylsulfonyle en C₁-C₄, alcényle en C₂-C₄, alcynyle en C₂-C₄, halogénoalcényle en C₂-C₄, halogénoalkyle en C₁-C₄, halogénoalcynyle en C₂-C₄, halogénoalkylsulfonyle en C₁-C₄, (alkyl en C₁-C₄)carbonyle, (alcoxy en C₁-C₄)carbonyle, (halogénoalkyl en C₁-C₄)-carbonyle ou (halogénoalcoxy en C₁-C₄)carbonyle,
R³³ représente un reste du groupe constitué par les restes possibles pour R³², ou
NR³²R³³ représente globalement un reste hétérocyclique tel que défini de façon correspondante pour NR¹⁹R²⁰.

4. Composés de formule (I) ou leurs sels selon l'une des revendications 1 à 3, **caractérisés en ce que**
R représente un atome d'hydrogène ou un reste alkyle en C₂-C₃ ou alcoxy en C₁-C₃,
R¹ représente un atome d'hydrogène ou un reste CN, NO₂, halogéno, alcoxy en C₁-C₆, alkyle en C₁-C₆, halogénoalcoxy en C₁-C₆, halogénoalkyle en C₁-C₆, (alcoxy en C₁-C₆)carbonyle, (cycloalcoxy en C₃-C₆)carbonyle, (cycloalkyl en C₃-C₆)méthoxycarbonyle, alkylthio en C₁-C₆, halogénoalkylthio en C₁-C₆, alkylsulfonyle en C₁-C₆, halogénoalkylsulfonyle en C₁-C₆, SO₂NH₂, CONH₂ mono- ou di(alkyl en C₁-C₄)aminocarbonyle ou -aminosulfonyle
ou un reste de formule 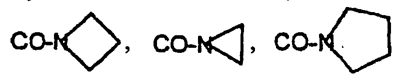 ou un reste
(alkyl en C₁-C₆)carbonyle, (halogénoalkyl en C₁-C₆)carbonyle, CHO, cyclopropylcarbonyle, cyclobutylcarbonyle ou NR²⁸R²⁹;
R² représente un atome d'hydrogène ou un reste alkyle en C₁-C₆, halogénoalkyle en C₁-C₆, alcoxy en C₁-C₆, halogénoalcoxy en C₁-C₆, halogéno ou CN;
R³, R⁵, R⁷, R⁹, R¹¹, R¹³ et R¹⁴ représentent chacun un reste du groupe constitué par les restes possibles pour R¹,
R⁴, R⁶, R⁸, R¹⁰ et R¹² représentent chacun un reste du groupe constitué par les restes possibles pour R²,
R¹⁵ représente un atome d'hydrogène ou un reste alkyle en C₁-C₃ ou phényle;
R¹⁶ représente un atome d'hydrogène ou un reste NH₂, mono- ou di(alkyl en C₁-C₆)amino, alkyle en C₁-C₆, phényle ou alcoxy en C₁-C₆, les 5 derniers restes cités étant non substitués ou substitués par un ou plusieurs restes du groupe constitué par les restes halogéno, alcoxy en C₁-C₃, halogénoalcoxy en C₁-C₃, CN et NO₂, ainsi que, dans le cas du reste phényle, par des restes alkyle en C₁-C₃ et halogénoalkyle en C₁-C₃,
R¹⁷ représente un reste mono- ou di(alkyl en C₁-C₆)amino, alkyle en C₁-C₆, alcoxy en C₁-C₆, phényle, benzyle, les 6 derniers restes cités étant non substitués ou substitués par un ou plusieurs restes du groupe constitué par les restes halogéno, alcoxy en C₁-C₃ et halogénoalcoxy en C₁-C₃, ainsi que, dans le cas du reste phényle, par des restes alkyle en C₁-C₃ et halogénoalkyle en C₁-C₃, ou un reste OH ou NH₂ ou un atome d'hydrogène; ou le fragment de formule R¹⁷-N=C-R¹⁶ du groupe de formule B1 dans la définition de B forme globalement un hétérocycle de formule D1 ou D2 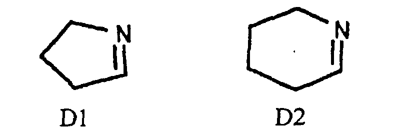
R¹⁸ représente un atome d'hydrogène ou un reste alkyle en C₁-C₆ ou halogénoalkyle en C₁-C₆,
R¹⁹ représente un atome d'hydrogène ou un reste alkyle en C₁-C₆, halogénoalkyle en C₁-C₆, halogénoalcoxy en C₁-C₆ ou phényle,
R²⁰ représente un atome d'hydrogène ou un reste alkyle en C₁-C₆, halogénoalkyle en C₁-C₆ ou phényle, ou
NR¹⁹R²⁰ représente globalement un hétérocycle de formule 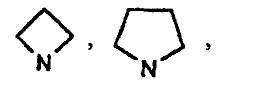 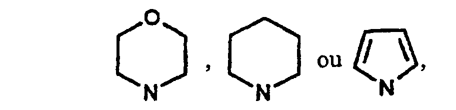
R²¹ représente un reste du groupe constitué par les restes possibles pour R¹⁶;
R²² représente un atome d'hydrogène ou un reste alkyle en C₁-C₆, halogénoalkyle en C₁-C₆ ou phényle;
R²³ représente un reste alkyle en C₁-C₆ ou halogénoalkyle en C₁-C₆;
R²⁸ représente un atome d'hydrogène ou un reste alkyle en C₁-C₆, OH, halogénoalkyle en C₁-C₆ ou alcoxy en C₁-C₆;
R²⁹ représente un atome d'hydrogène ou un reste alkyle en C₁-C₆, halogénoalkyle en C₁-C₆, CHO, alkylsulfonyle en C₁-C₃, (alcoxy en C₁-C₃)carbonyle ou (alkyl en C₁-C₃)carbonyle;
X, Y représentent indépendamment l'un de l'autre un reste Cl, F, CF₃, CH₃, CH₂CH₃, OCH₃, OC₂H₅, N(CH₃)₂, NHCH₃ ou OCH₂CF₃.

5. Composés de formule (I) ou leurs sels selon l'une des revendications 1 à 4, **caractérisés en ce que**
A représente un pont de formule 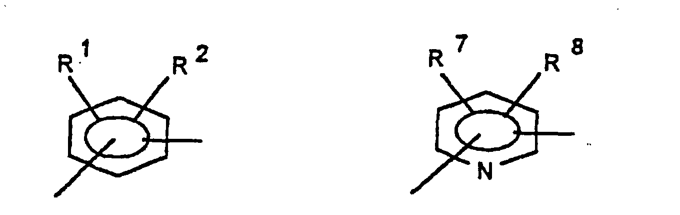 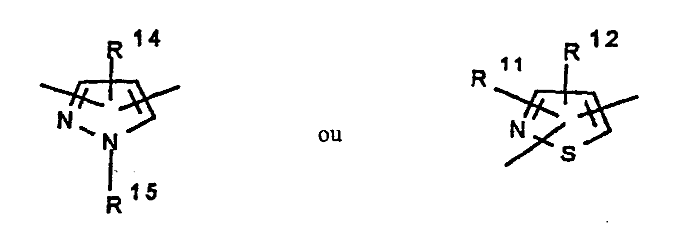 et
B représente un groupe de formule B1, B2 ou B3
-NR¹⁸-C(R¹⁶)=N-R¹⁷ (B1)
-N=C(R²¹)-NR¹⁹R²⁰ (B2)
-N=C(R²²)-O-R²³ (B3)
les restes R¹, R², R⁷, R⁸, R¹¹, R¹², R¹⁴, R¹⁵ et R¹⁶ à R²³ dans les formules données pour A et B ayant la définition donnée pour la formule (I).

6. Procédé de préparation de composés de formule (I) ou de leurs sels, tels que définis selon l'une des revendications 1 à 5, **caractérisé en ce que**
a) on fait réagir un composé de formule (II)
B-A-SO₂NH₂ (II)
avec un carbamate hétérocyclique de formule (III) 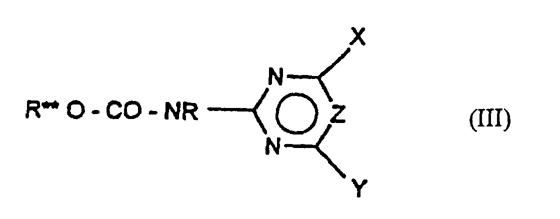 dans laquelle R** représente un reste phényle ou alkyle en C₁-C₄ substitué ou non substitué, ou
b) on fait réagir un isocyanate de sulfonyle de formule (IV)
B-A-SO₂-N=C=O (IV)
avec une amine hétérocyclique de formule (V) 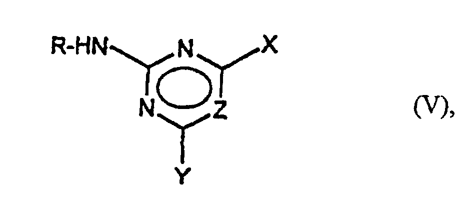 ou
c) on fait réagir un sulfochlorure de formule (VI)
B-A-SO₂Cl (VI)
avec une amine hétérocyclique de la formule (V) précitée en présence d'un cyanate, ou
d) on fait réagir un sulfochlorure de la formule (II) précitée avec un thio)isocyanate de formule (VII) 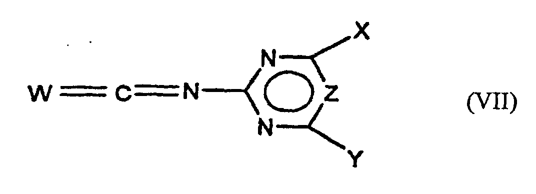 en présence d'une base appropriée,
e) dans le cas où B est un groupe contenant un fragment de formule -N=C-NR^{a}- dans laquelle R^{a} représente un groupe NH₂ ou OH ou un reste organique de 1 à 12 atomes de carbone, on fait réagir une sulfonylurée amino-substituée de formule (VIII) 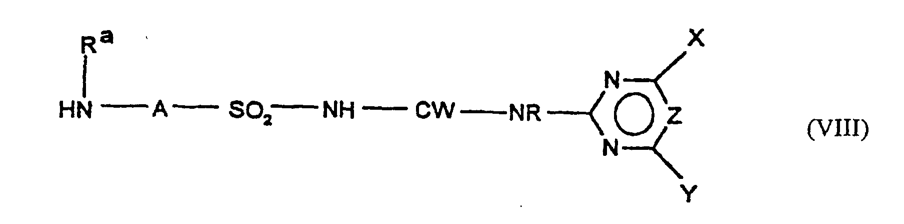 dans laquelle R^{a} a la définition donnée ci-dessus,
avec des réactifs appropriés contenant un fragment de formule (IXa) 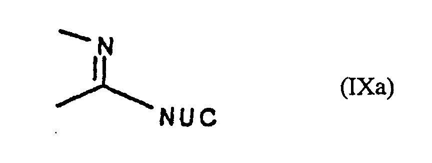 dans laquelle NUC est un groupe nucléofuge,
f) dans le cas où B est un groupe contenant un fragment de formule 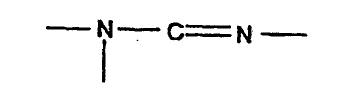 on fait réagir une sulfonylurée amino-substituée de la formule (VIII) précitée (voir e), dans laquelle R^{a} est un atome d'hydrogène, avec des réactifs appropriés contenant un fragment de formule (IXb) 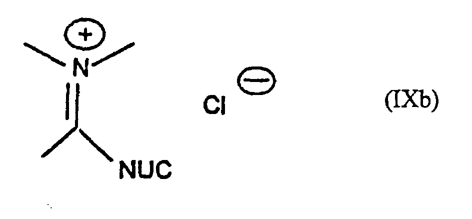 dans laquelle NUC est un groupe nucléofuge,
où les restes A, B, R, W, X, Y et Z des formules (II) à (IX) ont la définition donnée pour la formule (I), et où, dans les variantes a) à c), on obtient d'abord des composés de formule (I) dans lesquels W représente un atome d'oxygène.

7. Composition herbicide ou régulatrice de croissance des plantes, **caractérisée en ce qu'**elle contient au moins un composé de formule (I) ou un de ses sels selon l'une des revendications 1 à 5 et des auxiliaires de formulation classiques dans le domaine phytosanitaire.

8. Procédé de lutte contre des plantes nuisibles ou de régulation de croissance de plantes, **caractérisé en ce que** l'on applique sur les plantes nuisibles, ou sur les plantes, sur leurs graines ou sur la surface sur laquelle elles poussent, une quantité active d'au moins un composé de formule (I) ou d'un de ses sels selon l'une des revendications 1 à 5.

9. Utilisation des composés de formule (I) ou de leurs sels selon l'une des revendications 1 à 5 comme herbicides ou comme régulateurs de croissance de plantes.

10. Composés de formule (XII)
B-A-SO₂-R^{z} (XII)
dans laquelle
R^{z} représente un reste NH₂, mono- ou di(alkyl en C₁-C₄)amino, halogéno ou -N=CO, et B et A ont la définition donnée pour la formule (I) dans l'une des revendications 1 à 5,
à l'exception des composés de formule (XII) dans lesquels
a) R^{z} est un groupe amino,
A est un reste phényle portant deux restes généraux Y' et Z', Y' étant un atome d'hydrogène ou d'halogène ou un groupe alkyle et Z' étant un atome d'hydrogène ou d'halogène ou un groupe alkyle, alcoxy, trifluorométhyle, sulfamyle ou nitro, et
B est un groupe de formule -N=CR^{b}-NH₂ dans laquelle R^{b} est un reste hydrocarboné aliphatique ou cycloaliphatique saturé ou insaturé non conjugué, substitué ou non substitué, qui peut aussi être interrompu par un
ou plusieurs hétéroatomes, ou un reste aromatique substitué ou non substitué, chacun des restes pouvant aussi être lié par l'intermédiaire d'un atome d'oxygène ou d'un groupe NH,
b) R^{z} est un groupe diméthylamino ou diéthylamino,
A est un reste phényle pouvant être éventuellement substitué en position ortho par rapport au groupe SO₂-R^{z} par un reste chloro, bromo, méthyle, méthoxy ou trifluorométhyle, et
B est un groupe de formule -N=CH-NR'-Ar dans laquelle Ar représente un reste phényle ou 4-diméthylaminosulfonylphényle ou 4-diéthylamino-sulfonylphényle, chacun des deux derniers restes pouvant éventuellement être substitué dans la partie phényle par des restes 3-chloro, 3-bromo, 3-méthyle, 3-méthoxy ou 3-trifluorométhyle, et R' représente un atome d'hydrogène ou un reste N-(alkyl en C₁-C₄)aminocarbonyle ou N,N-di(alkyl en C₁-C₄)aminocarbonyle,
c) R^{z} est un groupe SO₂Cl,
A est un reste phényle et
B est un groupe de formule -NH-C(=NH)-NH₂, et
d) R^{z} est un groupe SO₂NH₂,
A est un reste phényle pouvant éventuellement être encore substitué, et
B est un reste de formule -NR'-C(=NH)-R", dans laquelle R' représente un atome d'hydrogène ou un reste alkyle en C₁-C₁₈ et R" représente un reste [(alcoxy en C₁-C₄)carbonyl]méthyle.
